(19)

Europäisches
Patentamt
European
Patent Office
Office européen
des brevets

(11) **EP 4 289 866 A2**

(12) **EUROPEAN PATENT APPLICATION**
published in accordance with Art. 153(4) EPC

(43) Date of publication:
**13.12.2023 Bulletin 2023/50**

(21) Application number: **22750023.8**

(22) Date of filing: **03.02.2022**

(51) International Patent Classification (IPC):
**C07K 16/28** (2006.01)   **A01K 67/027** (2006.01)
**C12N 7/00** (2006.01)   **A61P 35/00** (2006.01)
**A61P 25/28** (2006.01)   **G01N 33/68** (2006.01)
**A61P 37/00** (2006.01)   **A61K 39/00** (2006.01)

(52) Cooperative Patent Classification (CPC):
**C07K 16/28**

(86) International application number:
**PCT/KR2022/001714**

(87) International publication number:
**WO 2022/169274 (11.08.2022 Gazette 2022/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.02.2021   KR 20210015937
31.05.2021   KR 20210070443
19.10.2021   KR 20210139442**

(71) Applicant: **Genuv Inc.
Seoul 04520 (KR)**

(72) Inventors:
• **PARK, Heung Rok**
 **Seoul 04520 (KR)**
• **BAE, Dong Goo**
 **Seoul 04520 (KR)**
• **HAN, Sung Ho**
 **Seoul 04520 (KR)**

• **PARK, Chae Gyu**
 **Seoul 04520 (KR)**
• **YOON, Myeong Jin**
 **Seoul 04520 (KR)**
• **KIM, Hae Mi**
 **Seoul 04520 (KR)**
• **CHO, Eun Ji**
 **Seoul 04520 (KR)**
• **KIM, Kyoung Jin**
 **Seoul 04520 (KR)**
• **KIM, Ja Young**
 **Seoul 04520 (KR)**

(74) Representative: **J A Kemp LLP
80 Turnmill Street
London EC1M 5QU (GB)**

Remarks:
The complete document including Reference
Table(s) and the Sequence Listing(s) can be
downloaded from the EPO website

(54) **ANTI-PD-1 ANTIBODY AND USE THEREOF**

(57)   The present disclosure is directed to a protein binding agent, or an antibody or an antigen-binding fragment thereof, which binds to a programmed death-1 (PD-1) protein. The present disclosure further provides a polynucleotide sequence encoding the protein binding agent, the antibody or the antigen-binding fragment thereof, a vector comprising the polynucleotide sequence, or a host cell comprising the vector. Also, the present disclosure provides a pharmaceutical composition or a kit, which comprises the protein binding agent, the antibody or the antigen-binding fragment thereof.

EP 4 289 866 A2

Figure 6

## Human PD-1

## Mouse PD-1

## Description

### Technical Field

The present disclosure relates to antibodies that bind to programmed cell death-1 (PD-1) protein, antigen-binding fragments thereof, and uses thereof.

### Background Art

The description of this section merely provides information on the background art and does not constitute prior art.

Programmed cell death-1 (Programmed death-1; PD-1; also known as CD279) is a cell surface protein frequently found in immune cells such as T-cells, B-cells, monocytes, natural killer (NK) cells, and dendritic cells. It down-regulates the immune system to modulate the immune response and promotes self-tolerance by suppressing T-cell inflammatory activity. While this can prevent autoimmune diseases, it can interfere with the ability of the immune system to kill cancer cells (Syn et al., 2017 Lancet Oncol 18(12): e731-e741).

In recent years, the development of immuno-oncology agents that target the immune system to restore and promote immunity has been actively conducted. One of the immune checkpoint proteins, the PD-1/PD-L1 pathway, has been clinically confirmed to be a target for cancer immunotherapy (Patsoukis et al., 2020 Sci. Adv. 6: eabd2712). Therefore, PD-1 inhibitors are being developed to block PD-1 and activate the immune system to attack tumors and treat certain types of cancer.

In addition, upregulation of PD-1 signaling is also relevant to viral infection and expansion in humans. Infectious hepatitis viruses HBV and HCV induce overexpression of PD-1 ligand in hepatocytes and activate PD-1 signaling in effector T cells, leading to T-cell depletion and tolerance to viral infection (Golden-Mason et al., 2008 J Immunol 180:3637-3641). Likewise, HIV infection frequently evades the human immune system by similar mechanisms. It has been reported that therapeutic modulation of PD-1 signaling by antagonist molecules may result in recovery of immune cells from tolerance and elimination of cancer and chronic viral infection through reactivation of immune cells (Okazaki et al., 2007 Int Immunol 19:813-824).

Meanwhile, anti-PD-1 agonist antibodies are being developed for the treatment of autoimmune disorders such as rheumatoid arthritis and for reducing rejection of transplanted cells/tissues (Grebinoski and Vignali, 2020 Curr Opin Immunol 67:1- 9).

In addition, it has been proposed that IFNγ-dependent systemic immune response is beneficial for the treatment of Alzheimer's disease and other central nervous system pathologies that share common neuroinflammatory components, and International Publication No. WO2015/136541 discloses the use of anti-PD-1 antibodies to treat Alzheimer's disease. International Publication No. WO2017/220990 discloses that blocking of the PD-1/PD-L1 inhibitory immune checkpoint pathway increases the secretion of IFNγ by IFNγ-producing cells, and the increased IFNγ activity may enable the brain's choroid plexus to allow selective leukocyte trafficking and infiltration of T-cells and monocytes into the damaged CNS, homing of these immune cells to sites of neurodegenerative pathology and neuroinflammation, and may modulate the environment to become less toxic and more permissive for clearance of toxic agents, rescue of neurons, regeneration and repair.

Although the development of anti-PD-1 antibody therapeutics is active, there is still an urgent need for the development of a variety of anti-PD-1 antibodies with more diverse indications and characteristics. In addition, in order to efficiently develop anti-PD-1 antibody therapeutics or combinations containing the same, it is important that the antibody binds not only to human PD-1 but also to mouse PD-1 so that the efficacy, pharmacokinetic properties, and toxicity of the antibody can be evaluated in mice in the preclinical stage. However, most of the commercially available antibodies bind only to human PD-1. Therefore, the development of a novel anti-PD-1 antibody with cross-reactivity is necessary.

### Disclosure of Invention

### Technical Problem

One object of the present disclosure is to provide a novel PD-1 binding agent that binds to PD-1.

One object of the present disclosure is to provide a novel anti-PD-1 antibody and antigen-binding fragment thereof that bind to PD-1.

Another object of the present disclosure is to provide a novel cross-reactive protein binding agent, antibody, and antigen-binding fragment thereof that bind to both human PD-1 and mouse PD-1.

Another object of the present disclosure is to provide a method for producing a novel PD-1 binding agent, anti-PD-1 antibody, and antigen-binding fragment thereof.

Another object of the present disclosure is to provide a use of a novel PD-1 binding agent, anti-PD-1 antibody, and antigen-binding fragment thereof.

However, the problems to be solved by the present disclosure are not limited to the problems mentioned above, and other problems not mentioned will be clearly understood by those skilled in the art from the following description.

**Solution to Problem**

In order to solve the above problems, the inventors went through numerous experiments and developed a novel PD-1 binding agent, an anti-PD-1 antibody, and an antigen-binding fragment thereof.

In one aspect, the present disclosure provides an anti-PD-1 antibody and antigen-binding fragment thereof that bind to PD-1 wherein the antibody and antigen-binding fragment comprise a heavy chain variable region and/or a light chain variable region,

wherein the heavy chain variable region comprises heavy chain complementarity determining region 1 (HCDR1) comprising the amino acid sequence of SEQ ID NO: 1, HCDR2 comprising the amino acid sequence of SEQ ID NO: 3, 63, 64, 65, 66, or 67, and HCDR3 comprising an amino acid sequence selected from the group consisting of SEQ ID NOs: 5 and 68 to 82, or an HCDR variant(s) with a conservative amino acid substitution(s) or no more than three amino acid mutations compared to those sequences;

the light chain variable region comprises light chain complementarity determining region 1 (LCDR1) comprising the amino acid sequence of SEQ ID NO: 7, 60, 83, or 84, LCDR2 comprising the amino acid sequence of SEQ ID NO: 9, and LCDR3 comprising the amino acid sequence of SEQ ID NO: 11, or an LCDR variant(s) with a conservative amino acid substitution(s) or no more than three amino acid mutations compared to those sequences.

In one aspect, the present disclosure provides an anti-PD-1 antibody and antigen-binding fragment thereof that bind to PD-1 wherein the antibody comprises a heavy chain variable region and/or a light chain variable region, wherein the heavy chain variable region comprises a sequence as indicated by SEQ ID NO: 13, 54, 56, or 58, or a variant thereof with 1 to 10 or less amino acid mutations compared to those sequences; and the light chain variable region comprises a sequence as indicated by SEQ ID NO: 15, 55, 57, or 59, or a variant with 1 to 10 or less amino acid mutations compared to those sequences.

In one aspect, the present disclosure provides an anti-PD-1 antibody or antigen-binding fragment thereof that specifically binds to an epitope of PD-1 comprising P130, L128, and 1126 of human PD-1 (SEQ ID NO: 62). The anti-PD-1 antibody or antigen-binding fragment thereof may specifically bind to an additional epitope comprising at least one selected from the group consisting of N66, Y68, K78, A129, and A132 of SEQ ID NO: 62.

In one aspect, the present disclosure provides an isolated immunoglobulin heavy chain variable region polypeptide comprising heavy chain complementarity determining region 1 (HCDR1) comprising the amino acid sequence of SEQ ID NO: 1, HCDR2 comprising the amino acid sequence of SEQ ID NO: 3, 63, 64, 65, 66 or 67, and HCDR3 comprising an amino acid sequence selected from the group consisting of SEQ ID NO: 5 and 68 to 82, or an HCDR variant(s) with a conservative amino acid substitution(s) or no more than three amino acid mutations compared to those sequences.

In one aspect, the present disclosure provides an isolated immunoglobulin light chain variable region polypeptide comprising light chain complementarity determining region 1 (LCDR1) comprising the amino acid sequence of SEQ ID NO: 7, 60, 83, or 84, LCDR2 comprising the amino acid sequence of SEQ ID NO: 9, and LCDR3 comprising the amino acid sequence of SEQ ID NO: 11, or an LCDR variant(s) with a conservative amino acid substitution(s) or no more than three amino acid mutations compared to those sequences.

In one aspect, the present disclosure provides an isolated immunoglobulin heavy chain variable region polypeptide that binds to PD-1 comprising a sequence as indicated by SEQ ID NO: 13, 54, 56, or 58, or a variant with 1 to 10 or less amino acid mutations compared to those sequences.

In one aspect, the present disclosure provides an isolated immunoglobulin light chain variable region polypeptide that binds to PD-1 comprising a sequence as indicated by SEQ ID NO: 15, 55, 57, or 59, or a variant with 1 to 10 or less amino acid mutations compared to those sequences.

In one aspect, the present disclosure provides an isolated immunoglobulin heavy chain variable region polypeptide that binds to PD-1, having an amino acid sequence with at least 75%, 80%, 85%, 86%, 87%, 88%, 89%, 90%, 91%, 92%, 93%, 94%, 95%, 96%, 97%, 98%, or 99% sequence identity to SEQ ID NO: 13, 54, 56, or 58.

In one aspect, the present disclosure provides an isolated immunoglobulin light chain variable region polypeptide that binds to PD-1, having an amino acid sequence with at least 75%, 80%, 85%, 86%, 87%, 88%, 89%, 90%, 91%, 92%, 93%, 94%, 95%, 96%, 97%, 98%, or 99% sequence identity to SEQ ID NO: 15, 55, 57, or 59.

In one aspect, the present disclosure provides a heavy chain variable region polypeptide that binds to PD-1, having an amino acid sequence comprising addition, deletion, or conservative substitution, or any combination thereof, of 1 to 10 amino acids compared to the amino acid sequence of SEQ ID NO: 13, 54, 56, or 58.

In one aspect, the present disclosure provides a light chain variable region polypeptide that binds to PD-1, having an amino acid sequence comprising addition, deletion, or conservative substitution, or any combination thereof, of 1 to 10 amino acids compared to the amino acid sequence of SEQ ID NO: 15, 55, 57, or 59.

In one aspect, the present disclosure provides an isolated immunoglobulin heavy chain polypeptide comprising heavy chain complementarity determining region 1 (HCDR1) comprising the amino acid sequence of SEQ ID NO: 1, HCDR2 comprising the amino acid sequence of SEQ ID NO: 3, 63, 64, 65, 66, or 67, and HCDR3 comprising an amino acid sequence selected from the group consisting of SEQ ID NO: 5 and 68 to 82, or an HCDR variant(s) with a conservative amino acid substitution(s) or no more than three amino acid mutations compared to those sequences, and further comprising framework regions and constant regions.

In one aspect, the present disclosure provides an isolated immunoglobulin light chain polypeptide that binds to PD-1, comprising light chain complementarity determining region 1 (LCDR1) comprising the amino acid sequence of SEQ ID NO: 7, 60, 83, or 84, LCDR2 comprising the amino acid sequence of SEQ ID NO: 9, and LCDR3 comprising the amino acid sequence of SEQ ID NO: 11, or an LCDR variant(s) with a conservative amino acid substitution(s) or no more than three amino acid mutations compared to those sequences, and further comprising framework regions and constant regions.

In one aspect, the present disclosure provides an isolated immunoglobulin heavy chain polypeptide comprising a sequence as indicated by SEQ ID NO: 13, 54, 56, or 58, or a variant with 1 to 10 or less amino acid mutations compared to those sequences.

In one aspect, the present disclosure provides an isolated immunoglobulin light chain polypeptide comprising a sequence as indicated by SEQ ID NO: 15, 55, 57, or 59, or a variant with 1 to 10 or less amino acid mutations compared to those sequences.

In one aspect, the present disclosure provides an isolated immunoglobulin heavy chain polypeptide having an amino acid sequence with at least 75%, 80%, 85%, 86%, 87%, 88%, 89%, 90%, 91%, 92%, 93%, 94%, 95%, 96%, 97%, 98%, or 99% sequence identity to SEQ ID NO: 13, 54, 56, or 58.

In one aspect, the present disclosure provides an isolated immunoglobulin light chain polypeptide having an amino acid sequence with at least 75%, 80%, 85%, 86%, 87%, 88%, 89%, 90%, 91%, 92%, 93%, 94%, 95%, 96%, 97%, 98%, or 99% sequence identity to SEQ ID NO: 15, 55, 57, or 59.

In one aspect, the present disclosure provides an immunoglobulin heavy chain polypeptide that binds to PD-1, having an amino acid sequence comprising addition, deletion, or conservative substitution, or any combination thereof, of 1 to 10 amino acids compared to the amino acid sequence of SEQ ID NO: 13, 54, 56, or 58.

In one aspect, the present disclosure provides an immunoglobulin light chain polypeptide that binds to PD-1, having an amino acid sequence comprising addition, deletion, or conservative substitution, or any combination thereof, of 1 to 10 amino acids compared to the amino acid sequence of SEQ ID NO: 15, 55, 57, or 59.

In one aspect, the present disclosure provides a PD-1 binding agent comprising the anti-PD-1 antibody or antigen-binding fragment thereof, antibody conjugate, immunoglobulin heavy chain polypeptide and/or immunoglobulin light chain polypeptide, or immunoglobulin heavy chain variable region polypeptide and/or immunoglobulin light chain variable region polypeptide as described above.

In one aspect, the present disclosure provides a PD-1 binding agent, which is an antibody or antigen-binding fragment thereof selected from a camelized single domain antibody, a diabody, F(ab')$_2$, Fab', Fab, Fv, scFv, scFv dimer, BsFv, dsFv, (dsFv)$_2$, dsFv-dsFv', Fv fragment, ds diabody, nanobody, minibody, domain antibody, bivalent domain antibody, dAb, and single chain binding polypeptide.

In one aspect, the present disclosure provides an isolated or purified polynucleotide molecule comprising a polynucleotide sequence encoding any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide as described above.

In another aspect, the present disclosure provides an isolated or purified polynucleotide molecule comprising the polynucleotide sequence of SEQ ID NO: 14, which encodes a polypeptide of the immunoglobulin heavy chain variable region according to the present disclosure.

In one aspect, the present disclosure provides an isolated or purified polynucleotide molecule comprising the polynucleotide sequence of SEQ ID NO: 16, which encodes a polypeptide of the immunoglobulin light chain variable region according to the present disclosure.

In one aspect, the present disclosure provides a vector comprising a polynucleotide molecule comprising a polynucleotide sequence encoding any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure.

In one aspect, the present disclosure provides an isolated host cell comprising a vector comprising a polynucleotide molecule comprising a polynucleotide sequence encoding any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure.

In one aspect, the present disclosure provides a transgenic animal engineered to express any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure.

In one aspect, the present disclosure provides a method of expressing any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure, comprising culturing a host cell comprising a vector comprising an isolated polynucleotide encoding said PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide under conditions in which said isolated polynucleotide is expressed.

In one aspect, the present disclosure provides a method of screening a PD-1-like substance, comprising reacting any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure with a test substance and measuring the binding activity.

In one aspect, the present disclosure provides a PD-1-like substance screened by a method of screening a PD-1-like substance, comprising reacting any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure with a test substance and measuring the binding activity.

In one aspect, the present disclosure provides a method for producing an anti-PD-1 antibody, comprising immunizing PD-1 knockout mice with a PD-1 antigen, isolating B lymphocytes from the spleen removed from the mice, and selecting a hybridoma that produces an antibody that reacts with a PD-1 antigen from the hybridoma cells obtained by the fusion of myeloma cells with the B lymphocytes.

In one aspect, the present disclosure provides a hybridoma that produces an antibody that reacts with a PD-1 antigen selected by the antibody-producing method according to the present disclosure.

In one aspect, the present disclosure provides a multispecific antigen binding molecule, immunoconjugate, chimeric antigen receptor, engineered T cell receptor, or oncolytic virus comprising any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure.

In one aspect, the present disclosure provides a pharmaceutical composition comprising at least one selected from the group consisting of any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure, and a pharmaceutically acceptable excipient or carrier.

Said pharmaceutical composition may be a pharmaceutical composition for preventing, ameliorating, or treating tumor, cancer, metastatic tumor, metastatic cancer, autoimmune disease, neurological disease, neurodegenerative disease, or infectious disease.

In one aspect, the present disclosure provides a pharmaceutical composition further comprising a second therapeutic agent in the above pharmaceutical composition.

In one aspect, the present disclosure provides a kit for therapeutic, diagnostic, or detection use comprising at least one selected from the group consisting of any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure.

In one aspect, the present disclosure provides a method for preventing or treating tumor, cancer, metastatic tumor, metastatic cancer, autoimmune disease, neurological disease, neurodegenerative disease, or infectious disease

comprising a step of administering to an individual at least one selected from the group consisting of any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure.

In one aspect, the present disclosure provides a method for modulating an immune response in an individual, comprising administering to the individual at least one selected from the group consisting of any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure.

In one aspect, the present disclosure provides use of a PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, or a multispecific antigen binding molecule, immunoconjugate, chimeric antigen receptor, engineered T cell receptor, or oncolytic virus comprising the above agent, antibody, fragment, or polypeptide according to the present disclosure in the manufacture of a medicament for the prevention, amelioration, or treatment of tumor, cancer, metastatic tumor, metastatic cancer, autoimmune disease, neurological disease, neurodegenerative disease, or infectious disease.

In one aspect, the present disclosure also provides a method for inhibiting the growth of tumor cells in an individual, comprising administering to the individual at least one selected from the group consisting of a PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, or a multispecific antigen binding molecule, immunoconjugate, chimeric antigen receptor, engineered T cell receptor, or oncolytic virus comprising the above agent, antibody, fragment, or polypeptide according to the present disclosure in a therapeutically effective amount to inhibit the growth of tumor cells.

Other aspects will be apparent from the detailed description herein and common general technical knowledge in the art.

**Advantageous Effects**

The PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, or a multispecific antigen binding molecule, immunoconjugate, chimeric antigen receptor, engineered T cell receptor, or oncolytic virus comprising the above agent, antibody, fragment, or polypeptide according to the present disclosure can be used to modulate immune responses by binding to human PD-1. They are useful, for example, for targeting T cells expressing PD-1 and for modulating PD-1 activity. For example, they may be used for the prevention or treatment of tumor, cancer, metastatic tumor, metastatic cancer, autoimmune disease, neurological disease, neurodegenerative disease, or infectious disease.

In addition, the PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure can bind to mouse PD-1 as well as human PD-1, which allows testing in mice to measure the efficacy, pharmacokinetic properties, and toxicity of the antibody at the preclinical stage, playing an important role in the efficient development of antibody preparations or combinations containing the same.

The effect of the present disclosure is not limited to such literal description but includes the effects that a person skilled in the art can infer from this disclosure.

**Brief Description of Drawings**

FIG. 1 provides graphs for the test results using ELISA to show the binding of the hybridoma 1G1 antibody according to the present disclosure to the cell surface human PD-1 or mouse PD-1.

FIG. 2 provides graphs for the test results using flow cytometry to show the binding of the hybridoma 1G1 antibody according to the present disclosure to the cell surface human PD-1 or mouse PD-1.

FIG. 3 provides graphs for the test results using ELISA to measure the blocking of the binding between human PD-L1 or mouse PD-L1 and the cell surface human PD-1 or mouse PD-1 by the hybridoma 1G1 antibody of the present disclosure.

FIG. 4 provides graphs for the test results using flow cytometry to measure the blocking of the binding between human PD-L1 or mouse PD-L1 and the cell surface human PD-1 or mouse PD-1 by the hybridoma 1G1 antibody of the present disclosure.

FIG. 5 is the results of an SDS-PAGE to identify the purified hybridoma 1G1 antibody and the chimeric 1G1 antibody (1G1 chimeric).

FIG. 6 provides graphs for the test results using ELISA to show the binding of the purified hybridoma 1G1 antibody to the cell surface human PD-1 or mouse PD-1.

FIG. 7 is a result of testing by ELISA to confirm whether the monoclonal cell (hybridoma) 1G1 antibody of the present disclosure selectively binds to immune checkpoint proteins on human T cell surface.

FIG. 8 shows the heavy chain amino acid sequence of each of the three humanized 1G1 antibodies (humanized antibodies 1G1-h61, 1G1-h68, and 1G1-h70). The sequence is shown in the order of a leader sequence-**VH/VL (bold and underlined)**-hIgG4CH/hIgkappaCL.

FIG. 9 shows the light chain amino acid sequence of each of the three humanized 1G1 antibodies (humanized antibodies 1G1-h61, 1G1-h68, and 1G1-h70). The sequence is shown in the order of a leader sequence-**VH/VL (bold and underlined)**-hIgG4CH/hIgkappaCL.

FIG. 10a shows the heavy chain nucleic acid sequence of the humanized antibody 1G1-h61. The sequence is shown in the order of a leader sequence-**VH/VL (bold and underlined)-**hIgG4CH/hIgkappaCL-stop *codon (italic)*.

FIG. 10b shows the heavy chain nucleic acid sequence of the humanized antibody 1G1-h68. The sequence is shown in the order of a leader sequence-**VH/VL (bold and underlined)-**hIgG4CH/hIgkappaCL-stop *codon (italic)*.

FIG. 10c shows the heavy chain nucleic acid sequence of the humanized antibody 1G1-h70. The sequence is shown in the order of a leader sequence-**VH/VL (bold and underlined)-**hIgG4CH/hIgkappaCL-stop *codon (italic)*.

FIG. 11 shows the light chain nucleic acid sequences of each of the three humanized 1G1 antibodies (humanized antibodies 1G1-h61, 1G1-h68, and 1G1-h70). The sequence is shown in the order of a leader sequence-**VH/VL (bold and underlined)**-hIgG4CH/hIgkappaCL-*stop codon (italic)*.

FIG. 12a shows the binding kinetics of the humanized 1G1 antibodies according to the present disclosure to human PD-1.

FIG. 12b shows the affinity of the humanized 1G1 antibodies according to the present disclosure for human PD-1 described by ka (Kon), kd (Koff), and KD values.

FIG. 13 is the result of ELISA test to confirm whether the 1G1 antibodies according to the present disclosure (chimeric 1G1 antibody and humanized 1G1 antibodies) selectively bind (cross-reactivity) to the extracellular domain of the PD-1 antigens from human, mouse, rabbit, cynomolgus monkey and rat.

FIG. 14 provides a schedule of an animal experiment to measure the anticancer effect of a 1G1 antibody.

FIG. 15 is a graph showing changes in tumor size over time after administration of a 1G1 antibody in a mouse melanoma model.

FIG. 16 is a graph showing the survival over time after administration of a 1G1 antibody in a mouse melanoma model.

FIG. 17 shows the relative tumor size change and tumor growth inhibition rate over time after administration of a 1G1 antibody in the MC38 colorectal cancer syngeneic mouse model.

FIG. 18 depicts the binding regions of a humanized 1G1 antibody, Keytruda and Opdivo to human PD-1.

FIG. 19a shows the binding kinetics of a humanized 1G1 antibody according to the present disclosure to human PD-1 at pH 6.0.

FIG. 19b shows the affinity of a humanized 1G1 antibody according to the present disclosure for human PD-1 at pH 6.0 described by ka (Kon), kd (Koff), and KD values.

**Mode for the Invention**

It is to be understood that as the specific methods and experimental conditions described herein may vary, the present disclosure is not limited to such methods and conditions. Since the scope of the invention will be limited only by the appended claims, the terminology used herein is only for the purpose of describing particular aspects and is not intended to limit the claims.

Unless defined otherwise, all technical and scientific terms used herein have the same meaning as commonly understood by one of ordinary skill in the art to which this disclosure belongs. Any methods and materials similar or equivalent to those described herein can be used in the practice or testing of the present disclosure. All documents mentioned herein are incorporated herein by reference in their entirety.

The terms "programmed cell death-1," "PD-1," "PD-1 protein" are used herein interchangeably and include variants, isoforms, species homologs of human PD-1, and analogs having at least one common epitope with PD-1. PD-1 is a T-cell co-inhibitor, also known as CD279.

The term "binding molecule" or "binding agent" used herein includes antibodies, antigen-binding fragments thereof, and conjugates thereof with other molecules.

The term "antibody," as referred to herein, includes whole antibodies and any antigen-binding fragment (i.e., "antigen-binding portion") or single chains thereof. An "antibody" refers to a protein comprising at least two heavy chains and two light chains inter-connected by disulfide bonds or an antigen-binding portion thereof. Each heavy chain is comprised of a heavy chain variable region and a heavy chain constant region. The heavy chain constant region is comprised of three domains, CH1, CH2, and CH3. Each light chain is comprised of a light chain variable region and a light chain constant region. The light chain constant region is comprised of one domain, CL. The heavy chain variable region (VH) and light chain variable region (VL) can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen.

"Antibody," as used herein, refers to an immunoglobulin or fragment or derivative thereof and encompasses any polypeptide comprising an antigen-binding portion, whether produced *in vitro* or *in vivo*. The term includes, but is not limited to, polyclonal, monoclonal, monospecific, polyspecific, non-specific, humanized, single-chain, chimeric, synthetic, recombinant, hybrid, mutated, and grafted antibodies. The term "antibody" also encompasses an antibody fragment, for example, an Fab, Fab', F(ab')$_2$, Fv, scFv, BsFv, dsFv, (dsFv)$_2$, dsFv-dsFv', Fd, dAb, and other antibody fragments that maintain an antigen-binding ability, i.e., the ability to specifically bind to PD-1. Typically, such a fragment may include antigen-binding fragments.

"Antigen-binding fragment," "antigen-binding domain," and "binding fragment," as used herein, refer to a portion of an antibody molecule comprising the amino acids that cause the specific binding between the antibody and antigen. For example, if an antigen is a large molecule, the antigen-binding fragment may bind to only a portion of the antigen. The portion of an antigen molecule that causes the specific interaction with the antigen-binding fragment is referred to as "epitope" or "antigenic determinant."

An antigen-binding fragment may comprise antibody light chain variable region (VL) and heavy chain variable region (VH), but not necessarily both. For example, the so-called Fd antibody fragment consists only of the VH domain but still retains some antigen-binding function of the intact antibody.

The term "epitope" defines an antigenic determinant, which is specifically bound/identified by a binding fragment as defined above. The binding fragment may specifically bind to/interact with conformational or continuous epitopes which are unique for the target structure, e.g., the human PD-1 and rodent PD-1. A conformational or discontinuous epitope is characterized for polypeptide antigens by the presence of two or more discrete amino acid residues which are separated in the primary sequence but come together on the surface of the molecule when the polypeptide folds into the native protein/antigen. The two or more discrete amino acid residues contributing to the epitope are present on separate sections of one or more polypeptide chain(s). These residues come together on the surface of the molecule when the polypeptide chain(s) fold(s) into a three-dimensional structure to constitute the epitope. In contrast, a continuous or linear epitope consists of two or more contiguous amino acid residues which are present in a single linear segment of a polypeptide chain.

The term "binds to an epitope of PD-1" indicates that an antibody has specific binding for a particular epitope of PD-1, which may be defined by a linear amino acid sequence or by a tertiary, i.e., three-dimensional, conformation on part of the PD-1 polypeptide. Specific binding means that the antibody affinity for the portion of PD-1 is substantially greater than their affinity for other related polypeptides.

The term "greater affinity" means that there is a measurable increase in the affinity for the portion of PD-1 as compared with the affinity for other related polypeptides. Preferably, the affinity is at least 1.5-fold, 2-fold, 5-fold, 10-fold, 100-fold, $10^2$-fold, $10^3$-fold, $10^4$-fold, $10^5$-fold, or $10^6$-fold greater for the particular portion of PD-1 than for other proteins. Binding affinity can be determined by enzyme-linked immunosorbent assay (ELISA), fluorescence-activated cell sorting (FACS) analysis, or surface plasma resonance (SPR).

The term "cross-reactivity" in the present disclosure refers to the binding of an antigen-binding fragment described herein to the same target molecule in humans and rodents (mouse or rat). Therefore, "cross-reactivity" should be understood as interspecies reactivity to the same molecule X expressed in different species, not to a molecule other than X. For example, the cross-species specificity of a monoclonal antibody recognizing both human PD-1 and rodent (mouse or rat) PD-1 can be determined, for example, by FACS analysis.

In the present disclosure, "individual" or "subject" means a subject in need of treatment for a disease, more specifically, a mammal such as a human or non-human primate, rat, mouse, dog, cat, horse, and cow.

In the present disclosure, "treatment" refers to any action in which symptoms for a disease are improved or beneficially changed by administration of the pharmaceutical composition according to the present disclosure. Treatment also includes prevention. Those in need of treatment include those who already have a particular medical disorder, as well as those who will eventually acquire the disorder.

"Amelioration" in the present disclosure means any action that at least reduces a parameter associated with the condition being treated, e.g., the severity of a symptom.

Hereinafter, details for carrying out the present disclosure will be described in detail with reference to the specific examples and accompanying drawings. Matters that are not different from the prior art and are not necessary to understand the technical concept of the present disclosure are excluded from the description.

**Exemplary anti-PD-1 antibodies and PD-1 binding agents, etc.**

The present disclosure, in one aspect, discloses an anti-PD-1 antibody or antigen-binding fragment thereof that binds to PD-1 wherein the antibody or antigen-binding fragment comprises a heavy chain variable region and/or a light chain variable region,

> wherein the heavy chain variable region comprises a heavy chain complementarity determining region 1 (HCDR1) comprising the amino acid sequence of SEQ ID NO: 1, HCDR2 comprising the amino acid sequence of SEQ ID NO: 3, 63, 64, 65, 66, or 67, and HCDR3 comprising an amino acid sequence selected from the group consisting of SEQ ID NOs: 5 and 68 to 82, or an HCDR variant(s) with a conservative amino acid substitution(s) or no more than three amino acid mutations compared to those sequences;
> the light chain variable region comprises light chain complementarity determining region 1 (LCDR1) comprising the amino acid sequence of SEQ ID NO: 7, 60, 83, or 84, LCDR2 comprising the amino acid sequence of SEQ ID NO: 9, and LCDR3 comprising the amino acid sequence of SEQ ID NO: 11, or an LCDR variant(s) with a conservative amino acid substitution(s) or no more than three amino acid mutations compared to those sequences.

In some embodiments, "no more than three amino acid mutation(s)" means 3, 2, 1, or 0 amino acid mutation(s).

In some embodiments, the anti-PD-1 antibody or antigen-binding fragment thereof of the present disclosure may have an equivalent level of binding affinity for human PD-1 and mouse PD-1.

In some embodiments, the anti-PD-1 antibody or antigen-binding fragment thereof binds to PD-1 with a KD value of $10^{-7}$ M or less; in some embodiments, it binds to PD-1 with a KD value of $10^{-8}$, $10^{-9}$, $10^{-10}$, or $10^{-11}$ M or less.

In some embodiments, the anti-PD-1 antibody or an antigen-binding fragment thereof binds to PD-1 even in a low pH environment with a KD of $10^{-9}$ M or less, preferably a KD of $10^{-10}$ M or less, more preferably a KD of $10^{-11}$ M or less. In some embodiments, the anti-PD-1 antibody or an antigen-binding fragment thereof binds to PD-1 with a KD of $9 \times 10^{-10}$ M or less at pH 6.0.

The present disclosure provides, in one aspect, an anti-PD-1 antibody and antigen-binding fragment thereof that binds to PD-1 wherein the antibody comprises a heavy chain variable region and/or a light chain variable region, wherein the heavy chain variable region comprises a sequence as indicated by SEQ ID NO: 13, 54, 56, or 58, or a variant thereof with 1 to 10 or less amino acid mutations compared to those sequences; the light chain variable region comprises a sequence as indicated by SEQ ID NO: 15, 55, 57, or 59, or a variant with 1 to 10 or less amino acid mutations compared to those sequences.

In one aspect, the present disclosure provides an isolated immunoglobulin heavy chain variable region polypeptide comprising heavy chain complementarity determining region 1 (HCDR1) comprising the amino acid sequence of SEQ ID NO: 1, HCDR2 comprising the amino acid sequence of SEQ ID NO: 3, 63, 64, 65, 66, or 67, and HCDR3 comprising an amino acid sequence selected from the group consisting of SEQ ID NO: 5 and 68 to 82, or an HCDR variant(s) with a conservative amino acid substitution(s) or no more than three amino acid mutations compared to those sequences.

In one aspect, the present disclosure provides an isolated immunoglobulin light chain variable region polypeptide comprising light chain complementarity determining region 1 (LCDR1) comprising the amino acid sequence of SEQ ID NO: 7, 60, 83, or 84, LCDR2 comprising the amino acid sequence of SEQ ID NO: 9, and LCDR3 comprising the amino acid sequence of SEQ ID NO: 11, or an LCDR variant(s) with a conservative amino acid substitution(s) or no more than three amino acid mutations compared to those sequences.

In one aspect, the present disclosure provides an isolated immunoglobulin heavy chain variable region polypeptide that binds to PD-1 comprising a sequence as indicated by SEQ ID NO: 13, 54, 56, or 58, or a variant with 1 to 10 or less amino acid mutations compared to those sequences.

In one aspect, the present disclosure provides an isolated immunoglobulin light chain variable region polypeptide that binds to PD-1 comprising a sequence as indicated by SEQ ID NO: 15, 55, 57, or 59, or a variant with 1 to 10 or less amino acid mutations compared to those sequences.

"Isolated" in the present disclosure refers to having been separated from its natural milieu.

In one aspect, the present disclosure provides an isolated immunoglobulin heavy chain variable region polypeptide that binds to PD-1, having an amino acid sequence with at least 75%, 80%, 85%, 86%, 87%, 88%, 89%, 90%, 91%, 92%, 93%, 94%, 95%, 96%, 97%, 98%, or 99% sequence identity to SEQ ID NO: 13, 54, 56, or 58.

In one aspect, the present disclosure provides an isolated immunoglobulin light chain variable region polypeptide that binds to PD-1, having an amino acid sequence with at least 75%, 80%, 85%, 86%, 87%, 88%, 89%, 90%, 91%, 92%, 93%, 94%, 95%, 96%, 97%, 98%, or 99% sequence identity to SEQ ID NO: 15, 55, 57, or 59.

Sequence similarity for a polypeptide is typically measured using sequence analysis software. Protein analysis software matches similar sequences using a measure of similarity assigned to various substitutions, deletions, and other modifications, including conservative amino acid substitutions. For example, GCG software contains programs such as GAP and BESTFIT, which can be used with default parameters to determine sequence similarity or sequence identity between closely related polypeptides, such as homologous polypeptides from different species of organisms or between a wild-type protein and a mutein thereof. See, for example, GCG version 6.1. Polypeptide sequences can also be compared using FASTA with default or recommended parameters; a program in GCG Version 6.1. FASTA (e.g., FASTA2 and FASTA3) provides alignments and percent sequence identity of the regions with the best overlap between the query and search sequences. Another preferred algorithm when comparing a sequence of the present disclosure to a database containing a large number of sequences from different organisms is the computer program BLAST using default parameters, especially BLASTP or TBLASTN. See, for example, Altschul et al. (1990) J. Mol. Biol. 215:403-410 and (1997) Nucleic Acids Res. 25:3389-3402, each of which is incorporated herein by reference.

Residues that are not identical may differ, for example, by conservative amino acid substitutions.

In one aspect, the present disclosure provides a heavy chain variable region polypeptide that binds to PD-1, having an amino acid sequence comprising addition, deletion, or conservative substitution, or any combination thereof of 1 to 10 amino acids compared to the amino acid sequence of SEQ ID NO: 13, 54, 56, or 58.

In one aspect, the present disclosure provides a light chain variable region polypeptide that binds to PD-1, having an amino acid sequence comprising addition, deletion, or conservative substitution, or any combination thereof of 1 to 10 amino acids compared to the amino acid sequence of SEQ ID NO: 15, 55, 57, or 59.

A "conservative amino acid substitution" means that the amino acid residue is replaced by another amino acid residue with a side chain (R group) having similar chemical properties (e.g., charge or hydrophobicity). In general, a conservative amino acid substitution will not substantially change the functional properties of a protein. In cases where two or more amino acid sequences differ from each other by conservative substitutions, the percent similarity or the degree of similarity may be adjusted upwards to correct for the conservative nature of the substitution. Means for making this adjustment are well-known to those skilled in the art. See, e.g., Pearson (1994) Methods Mol. Biol. 24: 307-331, which is incorporated herein by reference. Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartate and glutamate; and 7) sulfur-containing side chains: cysteine and methionine. Preferable conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al. (1992) Science 256:1443-45, which is incorporated herein by reference. A "moderately conservative" replacement is any change having a nonnegative value in the PAM250 log-likelihood matrix.

In one aspect, the present disclosure provides an isolated immunoglobulin heavy chain polypeptide comprising heavy chain complementarity determining region 1 (HCDR1) comprising the amino acid sequence of SEQ ID NO: 1, HCDR2 comprising the amino acid sequence of SEQ ID NO: 3, 63, 64, 65, 66, or 67, and HCDR3 comprising an amino acid sequence selected from the group consisting of SEQ ID NO: 5 and 68 to 82, or an HCDR variant(s) with a conservative amino acid substitution(s) or no more than three amino acid mutations compared to those sequences, and further comprising framework regions and constant regions.

In one aspect, the present disclosure provides an isolated immunoglobulin light chain polypeptide that binds to PD-1, comprising light chain complementarity determining region 1 (LCDR1) comprising the amino acid sequence of SEQ ID NO: 7, 60, 83, or 84, LCDR2 comprising the amino acid sequence of SEQ ID NO: 9, and LCDR3 comprising the amino acid sequence of SEQ ID NO: 11, or an LCDR variant(s) with a conservative amino acid substitution(s) or no more than three amino acid mutations compared to those sequences, and further comprising framework regions and constant regions.

In one aspect, the present disclosure provides an isolated immunoglobulin heavy chain polypeptide comprising a sequence as indicated by SEQ ID NO: 13, 54, 56, or 58, or a variant with 1 to 10 or less amino acid mutations compared to those sequences.

In one aspect, the present disclosure provides an isolated immunoglobulin light chain polypeptide comprising a sequence as indicated by SEQ ID NO: 15, 55, 57, or 59, or a variant with 1 to 10 or less amino acid mutations compared to those sequences.

In one aspect, the present disclosure provides an isolated immunoglobulin heavy chain polypeptide having an amino acid sequence with at least 75%, 80%, 85%, 86%, 87%, 88%, 89%, 90%, 91%, 92%, 93%, 94%, 95%, 96%, 97%, 98%, or 99% sequence identity to SEQ ID NO: 13, 54, 56, or 58.

In one aspect, the present disclosure provides an isolated immunoglobulin light chain polypeptide having an amino acid sequence with at least 75%, 80%, 85%, 86%, 87%, 88%, 89%, 90%, 91%, 92%, 93%, 94%, 95%, 96%, 97%, 98%, or 99% sequence identity to SEQ ID NO: 15, 55, 57, or 59.

In one aspect, the present disclosure provides an immunoglobulin heavy chain polypeptide that binds to PD-1, having an amino acid sequence comprising addition, deletion, or conservative substitution, or any combination thereof of 1 to 10 amino acids compared to the amino acid sequence of SEQ ID NO: 13, 54, 56, or 58.

In one aspect, the present disclosure provides an immunoglobulin light chain polypeptide that binds to PD-1, having an amino acid sequence comprising addition, deletion, or conservative substitution, or any combination thereof of 1 to 10 amino acids compared to the amino acid sequence of SEQ ID NO: 15, 55, 57, or 59.

In some embodiments, the anti-PD-1 antibody or antigen-binding fragment thereof may be a recombinant antibody, preferably a murine antibody, a chimeric antibody, or a humanized antibody.

In some embodiments, the heavy chain constant region of the chimeric or humanized anti-PD-1 antibody may be derived from human IgG1, IgG2, IgG3, IgG4, or a mutant sequence(s) thereof, and the light chain constant region may be derived from human kappa, lambda chain, or a mutant sequence(s) thereof.

In some embodiments of the anti-PD-1 antibody or antigen-binding fragment thereof of the present disclosure, the antibody is a chimeric antibody, and the constant region is derived from a human antibody constant region or a mutant thereof.

In some embodiments of the anti-PD-1 antibody or antigen-binding fragment thereof of the present disclosure, the antibody is a humanized antibody, and the light chain framework region (FR) and heavy chain framework region of the antibody are derived from human germline light chain and heavy chain, respectively, or from a mutant sequence(s) thereof.

In one aspect, the present disclosure provides a PD-1 binding agent comprising an anti-PD-1 antibody or antigen-binding fragment thereof, antibody conjugate, immunoglobulin heavy chain polypeptide and/or immunoglobulin light chain polypeptide, or immunoglobulin heavy chain variable region polypeptide and/or an immunoglobulin light chain variable region polypeptide according to the present disclosure.

The PD-1 binding agent may be, for example, an antibody, an antibody conjugate, or an antigen-binding fragment thereof, but is not limited thereto.

In some embodiments, the present disclosure provides an isolated PD-1 binding agent, anti-PD-1 antibody or antigen-binding fragment thereof, which competes with any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide described above for binding to PD-1 or binds to the same PD-1 epitope as the PD-1 binding agent, anti-PD-1 antibody or antigen-binding fragment thereof.

**Antigen-binding fragment**

In one aspect of the present disclosure, the PD-1 binding agent according to the present disclosure can be, but is not limited to, an antibody or antigen-binding fragment thereof selected from camelized single domain antibody, diabody, F(ab')$_2$, Fab', Fab, Fv, scFv, scFv dimer, BsFv, dsFv, (dsFv)$_2$, dsFv-dsFv', Fv fragment, ds diabody, nanobody, minibody, domain antibody, bivalent domain antibody, dAb, and single chain binding polypeptide.

Unless specifically indicated otherwise, the term "antibody," as used herein, encompasses antibody molecules comprising two immunoglobulin heavy chains and two immunoglobulin light chains (i.e., "full antibody molecules") as well as antigen-binding fragments thereof. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. The term "antigen-binding fragment" or "antibody fragment" of an antibody refers to one or more fragments of an antibody that retain the ability to specifically bind PD-1. An antibody fragment may include a Fab fragment, a F(ab)$_2$ fragment, a Fv fragment, a dAb fragment, a fragment containing a CDR, or an isolated CDR. In certain embodiments, the term "antigen-binding fragment" refers to a polypeptide fragment of a multi-specific antigen-binding molecule. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and (optionally) constant domains. Such DNA is known and/or is readily available from, e.g., commercial sources and DNA libraries (including, e.g., phage-antibody libraries) or can be synthesized. The DNA may be sequenced and manipulated chemically or by using molecular biology techniques, for example, to arrange one or more variable and/or constant domains into a suitable configuration or to introduce codons, create cysteine residues, modify, add or delete amino acids, etc.

Non-limiting examples of antigen-binding fragments include Fab, Fab', F(ab')$_2$, Fv, scFv, BsFv, dsFv, (dsFv)$_2$, dsFv-dsFv', Fd, dAb, and minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g., monovalent nanobodies, bivalent nanobodies, etc.), small modular immunopharmaceuticals (SMIPs), and shark variable IgNAR domains, are also encompassed within the expression "antigen-binding fragment" as used herein.

An antigen-binding fragment of an antibody will typically comprise at least one variable domain. The variable domain may be of any size or amino acid composition and will generally comprise at least one CDR, which is adjacent to or in frame with one or more framework sequences. In antigen-binding fragments having a VH domain associated with a VL domain, the VH and VL domains may be situated relative to one another in any suitable arrangement. For example, the variable region may be dimeric and contain VH-VH, VH-VL, or VL-VL dimers. Alternatively, the antigen-binding fragment of an antibody may contain a monomeric VH or VL domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present disclosure include: (i) VH-CH1; (ii) VH-CH2; (iii) VH-CH3; (iv) VH-CH1-CH2; (v) VH-CH1-CH2-CH3; (vi) VH-CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any configuration of variable and constant domains, including any of the exemplary configurations listed above, the variable and constant domains may be either directly linked to one another or may be linked by a full or partial hinge or linker region. A hinge region may consist of at least 2 (e.g., 5, 10, 15, 20, 40, 60 or more) amino acids, which result in a flexible or semi-flexible linkage between adjacent variable and/or constant domains in a single polypeptide molecule. Moreover, an antigen-binding fragment of an antibody of the present disclosure may comprise a homo-dimer or hetero-dimer (or other multimer) of any of the variable and constant domain configurations listed above in non-covalent association or covalent association (e.g., by a disulfide bond(s)) with one another and/or with one or more monomeric VH or VL domain. As with full antibody molecules, antigen-binding fragments may be monospecific or multi-specific (e.g., bi-specific). A multi-specific antigen-binding fragment of an antibody will typically comprise at least two different variable domains, wherein each variable domain is capable of specifically binding to a separate antigen or to a different epitope on the same antigen. Any multi-specific antibody format, including the exemplary bi-specific antibody formats disclosed herein, may be adapted for use in the context of an antigen-binding fragment of an antibody of the present disclosure using routine techniques available in the art.

**Nucleic acids encoding exemplary anti-PD-1 antibodies and binding agents, etc.**

In one aspect, the present disclosure provides an isolated or purified polynucleotide molecule comprising a polynucleotide sequence that encodes any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or an immunoglobulin light chain polypeptide according to the present disclosure.

In another aspect, the present disclosure provides an isolated or purified polynucleotide molecule comprising the polynucleotide sequence of SEQ ID NO: 14, which encodes a polypeptide of an immunoglobulin heavy chain variable region according to the present disclosure.

The present disclosure provides, in one aspect, an isolated or purified polynucleotide molecule comprising the polynucleotide sequence of SEQ ID NO: 16, encoding a polypeptide of an immunoglobulin light chain variable region according to the present disclosure.

### Preparation of exemplary anti-PD-1 antibodies and binding agents, etc.

In one aspect, the present disclosure provides a method for producing an antibody comprising immunizing PD-1 knockout mice with a PD-1 antigen, isolating B lymphocytes from the spleen removed from the mice, and selecting a hybridoma that produces an antibody that reacts with a human PD-1 antigen from the hybridoma cells obtained by the fusion of myeloma cells with the B lymphocytes.

In one aspect, the present disclosure provides a hybridoma prepared by the above method for producing an antibody.

In one aspect, the present disclosure provides a vector comprising a polynucleotide molecule comprising a polynucleotide sequence encoding any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure.

In one aspect, the present disclosure provides an isolated host cell comprising a vector comprising a polynucleotide molecule comprising a polynucleotide sequence encoding any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure.

In one embodiment, the host cell comprises (1) a vector comprising a polynucleotide encoding an amino acid sequence comprising the VL of an antibody according to the present disclosure and an amino acid sequence comprising the VH of the antibody or (2) a first vector comprising a polynucleotide encoding an amino acid sequence comprising the VL of an antibody according to the present disclosure and a second vector comprising a polynucleotide encoding an amino acid sequence comprising the VH of the antibody (for example, the host cell is transformed with these vectors).

In one embodiment, the present disclosure provides a method of preparing an anti-PD1-antibody comprising culturing a host cell comprising a polynucleotide encoding an antibody that binds to PD-1 under conditions suitable for expression of the antibody and selectively recovering the antibody from the host cell (or host cell culture medium).

Suitable host cells for cloning or expression of antibody-encoding vectors include prokaryotic or eukaryotic cells. For example, antibodies may be produced in bacteria, in particular when glycosylation and Fc effector function are not needed. For expression of antibody fragments and polypeptides in bacteria, see, e.g., U.S. Pat. Nos. 5,648,237, 5,789,199, and 5,840,523. See also Charlton, K.A., In: Methods in Molecular Biology, Vol. 248, Lo, B.K.C. (ed.), Humana Press, Totowa, N.J. (2003), pp. 245-254, describing the expression of antibody fragments in *E. coli.* After expression, the antibody may be isolated from the bacterial cell paste in a soluble fraction and can be further purified. In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, T.U., Nat. Biotech. 22 (2004) 1409-1414 and Li, H. et al., Nat. Biotech. 24 (2006) 210-215.

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts (see, e.g., U.S. Pat. Nos. 5,959,177, 6,040,498, 6,420,548, 7,125,978, and 6,417,429 (describing PLANTIBODIES™ technology for producing antibodies in transgenic plants)).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney cell line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor cells (MMT 060562); TRI cells, as described, e.g., in Mather, J.P. et al., Annals N.Y. Acad. Sci. 383 (1982) 44-68; MRC5 cells; and FS4 cells. Other useful mammalian host cell lines include Chinese hamster ovary (CHO) cells, including DHFR-CHO cells (Urlaub, G. et al., Proc. Natl. Acad. Sci. USA 77 (1980) 4216-4220), and myeloma cell lines such as Y0, NS0, and Sp2/0. For a review of certain mammalian host cell lines suitable for antibody production, see, e.g., Yazaki, P. and Wu, A. M., Methods in Molecular Biology, Vol. 248, Lo, B.K.C. (ed.), Humana Press, Totowa, NJ (2004), pp. 255-268.

In one aspect, the present disclosure provides a transgenic animal engineered to express any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure. The animal may be, for example, a rodent such as a mouse, rat, or the like.

In one aspect, the present disclosure provides a method of expressing any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure, comprising culturing a host cell comprising a vector comprising an isolated polynucleotide encoding said PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide under conditions in which said polynucleotide is expressed.

In one aspect, the present disclosure provides a method of screening a PD-1-like substance comprising reacting any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure with a test substance and measuring the binding activity.

In one aspect, the present disclosure provides a PD-1-like substance screened by a method of screening a PD-1-like substance, comprising reacting any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure with a test substance and measuring the binding activity.

## Multispecific antigen binding molecule, immunoconjugate

In one aspect, the present disclosure provides a multispecific antigen binding molecule, immunoconjugate, chimeric antigen receptor, engineered T cell receptor, or oncolytic virus comprising any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, or immunoglobulin light chain polypeptide according to the present disclosure.

A multispecific (e.g., bispecific) antigen-binding fragment of an antibody will typically comprise at least two different variable domains, wherein each variable domain is capable of specifically binding to a separate antigen or to a different epitope on the same antigen. Any multispecific antibody format, including bispecific antibody formats, may be adapted for use in the context of an antigen-binding fragment of an antibody of the present disclosure using routine techniques available in the art.

In one aspect, the present disclosure includes multispecific antigen-binding molecules or antigen-binding fragments thereof, wherein one specificity of an immunoglobulin is specific for the extracellular domain of PD-1 or a fragment thereof and the other specificity of the immunoglobulin is specific for binding outside the extracellular domain of PD-1, is specific for a second therapeutic target, or is conjugated to a therapeutic moiety. The other specificity of the immunoglobulin may be specific for a second target antigen. The second target antigen may be on the same cell as PD-1 or on a different cell. In one embodiment, the second target cell is on an immune cell other than a T-cell such as a B-cell, antigen-presenting cell, monocyte, macrophage, or dendritic cell. In some embodiments, the second target antigen may be present on a tumor cell or an autoimmune tissue cell or on a virally infected cell.

In another aspect, the present disclosure provides multispecific antigen-binding molecules or antigen-binding fragments thereof comprising a first antigen-binding specificity that binds to PD-1 and a second antigen-binding specificity that binds to a T-cell receptor, a B-cell receptor, or an Fc receptor. In a related aspect, the present disclosure provides multispecific antigen-binding molecules or antigen-binding fragments thereof comprising a first antigen-binding specificity that binds to PD-1 and a second antigen-binding specificity that binds to a different T-cell co-inhibitor such as LAG-3, CTLA-4, BTLA, CD-28, 2B4, LY108, TIGIT, TIM3, LAIR1, ICOS, and CD160.

In another aspect, the present disclosure provides multispecific antigen-binding molecules or antigen-binding fragments thereof comprising a first antigen-binding specificity that binds to PD-1 and a second antigen-binding specificity that binds to an autoimmune tissue-specific antigen. In certain embodiments, the antibodies may be activating or agonist antibodies.

Any of the multispecific antigen-binding molecules of the present disclosure, or variants thereof, may be constructed using standard molecular biological techniques (e.g., recombinant DNA and protein expression technology), as are known to a person of ordinary skill in the art.

In some embodiments, PD-1-specific antibodies are generated in a bispecific format (a "bispecific") in which variable regions binding to distinct domains of PD-1 are linked together to confer dual-domain specificity within a single binding molecule. Appropriately designed bispecifics may enhance overall PD-1 inhibitory efficacy through increasing both specificity and binding avidity. Variable regions with specificity for individual domains (e.g., segments of the N-terminal domain), or that can bind to different regions within one domain, are paired on a structural framework that allows each region to bind simultaneously to the separate epitopes, or to different regions within one domain.

In one example for a bispecific, heavy chain variable regions (VH) from a binder with specificity for one domain are recombined with light chain variable regions (VL) from a series of binders with specificity for a second domain to identify non-cognate VL partners that can be paired with an original VH without disrupting the original specificity for that VH. In this way, a single VL segment (e.g., VL1) can be combined with two different VH domains (e.g., VH1 and VH2) to generate a bispecific comprised of two binding "arms" (VH1-VL1 and VH2-VL1). Use of a single VL segment reduces the complexity of the system and thereby increases efficiency in cloning, expression, and purification processes used to generate the bispecific antibody (See, for example, US13/022759 and US2010/0331527).

Alternatively, antibodies that bind to one or more domains and to a second target, such as, but not limited to, for example, a second different anti-PD-1 antibody, may be prepared in a bispecific format using techniques described herein or other techniques known to those skilled in the art. Antibody variable regions binding to distinct regions may be linked together with variable regions that bind to relevant sites on, for example, the extracellular domain of PD-1, to confer dual-antigen specificity within a single binding molecule. Appropriately designed bispecifics of this nature serve a dual function. Variable regions with specificity for the extracellular domain are combined with a variable region with specificity for outside the extracellular domain and are paired on a structural framework that allows each variable region to bind to the separate antigens.

An exemplary bispecific antibody format that can be used in the context of the present disclosure involves the use of a first immunoglobulin (Ig) CH3 domain and a second Ig CH3 domain, wherein the first and second Ig CH3 domains differ from one another by at least one amino acid, and wherein at least one amino acid difference reduces binding of the bispecific antibody to Protein A as compared to a bispecific antibody lacking the amino acid difference.

In one embodiment, the first Ig CH3 domain binds Protein A and the second Ig CH3 domain contains a mutation that reduces or abolishes Protein A binding such as an H95R modification (by IMGT exon numbering; H435R by EU numbering). The second CH3 may further comprise a Y96F modification (by IMGT; Y436F by EU). Further modifications that may be found within the second CH3 include: D16E, L18M, N44S, K52N, V57M, and V82I (by IMGT; D356E, L358M, N384S, K392N, V397M, and V422I by EU) in the case of IgG1 antibodies; N44S, K52N, and V82I (IMGT; N384S, K392N, and V422I by EU) in the case of IgG2 antibodies; and Q15R, N44S, K52N, V57M, R69K, E79Q, and V82I (by IMGT; Q355R, N384S, K392N, V397M, R409K, E419Q, and V422I by EU) in the case of IgG4 antibodies. Variations on the bispecific antibody format described above are contemplated within the scope of the present disclosure.

Other exemplary bispecific formats that can be used in the context of the present disclosure include, without limitation, e.g., scFv-based or diabody bispecific formats, IgG-scFv fusions, dual variable domain (DVD)-Ig, Quadroma, knobs-into-holes, common light chain (e.g., common light chain with knobs-into-holes, etc.), CrossMab, CrossFab, (SEED) body, leucine zipper, Duobody, IgG1/IgG2, dual acting Fab (DAF)-IgG, and Mab2 bispecific formats (see, e.g., Klein et al. 2012, mAbs 4:6, 1-11, and references cited therein, for a review of the foregoing formats). Bispecific antibodies can also be constructed using peptide/nucleic acid conjugation, e.g., wherein unnatural amino acids with orthogonal chemical reactivity are used to generate site-specific antibody-oligonucleotide conjugates which then self-assemble into multimeric complexes with defined composition, valency, and geometry. (See, e.g., Kazane et al., J. Am. Chem. Soc. [Epub: Dec. 4, 2012]).

The present disclosure encompasses a human anti-PD-1 monoclonal antibody conjugated to a therapeutic moiety ("immunoconjugate"), such as a cytotoxin or a chemotherapeutic agent, to treat cancer. As used herein, the term "immunoconjugate" refers to an antibody which is chemically or biologically linked to a cytotoxin, a radioactive agent, a cytokine, an interferon, a target or reporter moiety, an enzyme, a toxin, a peptide or protein, or a therapeutic agent.

The antibody may be linked to the cytotoxin, radioactive agent, cytokine, interferon, target or reporter moiety, enzyme, toxin, peptide, or therapeutic agent at any location along the molecule so long as it is able to bind its target. Examples of immunoconjugates include antibody drug conjugates and antibody-toxin fusion proteins.

The type of therapeutic moiety that may be conjugated to the anti-PD-1 antibody will take into account the condition to be treated and the desired therapeutic effect to be achieved. Examples of suitable agents for forming immunoconjugates are known in the art; see for example, WO 05/103081.

**Therapeutic administration and formulation**

In one aspect, the present disclosure provides a pharmaceutical composition comprising at least one selected from the group consisting of any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure, and a pharmaceutically acceptable excipient or carrier.

Said pharmaceutical composition may be a pharmaceutical composition for preventing, ameliorating or treating tumor, cancer, metastatic tumor, metastatic cancer, autoimmune disease, neurological disease, neurodegenerative disease, or infectious disease.

In one aspect, the present disclosure provides a pharmaceutical composition further comprising a second therapeutic agent in said pharmaceutical composition.

In one aspect, the present disclosure provides a method for preventing, ameliorating, and/or treating tumor, cancer, metastatic tumor, metastatic cancer, autoimmune disease, neurological disease, neurodegenerative disease, or infectious disease, comprising a step of administering to an individual at least one selected from the group consisting of any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure.

The PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, or a multispecific antigen binding molecule, immunoconjugate, chimeric antigen receptor, engineered T cell receptor, or oncolytic virus comprising the above agent, antibody, fragment, or polypeptide according to the present disclosure is useful, inter alia, for the treatment, prevention, and/or amelioration of any disease, disorder, or pathological condition associated with or mediated by PD-1 expression, signaling, or activity, or treatable by blocking the interaction between PD-1 and a PD-1 ligand (e.g., PD-L1, or PD-L2) or otherwise inhibiting PD-1 activity and/or signaling.

The pharmaceutical composition according to the present disclosure may be for the prevention, treatment, or amelioration of conditions associated with PD-1.

The conditions associated with PD-1 may be, but are not limited to, tumor, cancer, metastatic tumor, metastatic cancer, autoimmune disease, neurological disease, neurodegenerative disease, or infectious disease.

The conditions associated with PD-1 may be, but are not limited to, non-small cell lung cancer, small cell lung cancer, renal cell cancer, kidney cancer, liver cancer, bone cancer, skin cancer, colon cancer, rectal cancer, ovarian cancer, breast cancer, pancreatic cancer, gastric carcinoma, bladder cancer, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycoses fungoids, Merkel cell cancer, and classical Hodgkin's lymphoma (CHL), primary mediastinal large B-cell lymphoma, T-cell/histiocyte-rich B-cell lymphoma, Epstein-Barr virus (EBV)-positive and -negative post-transplant lymphoproliferative disease (PTLD), or EBV-associated diffuse large B-cell lymphoma (DLBCL), plasmablastic lymphoma, external NK/T-cell lymphoma, nasopharyngeal carcinoma, or human herpes virus 8 (HHV8)-associated primary effusion lymphoma, or other hematologic malignancies including Hodgkin's lymphoma, neoplasms in the central nervous system including primary central nervous system (CNS) lymphoma, spinal axis tumor, and brainstem glioma.

The pharmaceutical composition according to the present disclosure may be used to treat early or late-stage symptoms of cancer. In one aspect, an antibody or antigen-binding fragment thereof can be used to treat metastatic cancer. The pharmaceutical composition according to the present disclosure is useful for reducing, inhibiting, or shrinking both solid tumors and blood cancer. In certain embodiments, the treatment of the pharmaceutical composition causes at least 50%, at least 60%, at least 70%, at least 80%, or at least 90% of tumor regression in a subject. In certain embodiments, the pharmaceutical composition may be used to prevent relapse of a tumor. In certain embodiments, the pharmaceutical composition is useful in extending overall survival in a subject with cancer. In some embodiments, the pharmaceutical composition is useful in reducing toxicity due to chemotherapy or radiotherapy while maintaining long-term survival in patients with cancer.

The autoimmune disease associated with the PD-1 may be, but is not limited to, for example, lupus, systemic lupus erythematosus, Sjogren's syndrome, arthritis, rheumatoid arthritis, asthma, COPD, pelvic inflammatory disease, Alzheimer's disease, inflammatory bowel disease, Crohn's disease, ulcerative colitis, Peyronie's disease, coeliac disease, gallbladder disease, Pilonidal disease, peritonitis, psoriasis, psoriatic arthritis, vasculitis, surgical adhesions, stroke, type 1 diabetes, Lyme disease, meningoencephalitis, autoimmune uveitis, multiple sclerosis, Guillain-Barr syndrome, atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune diseases, pancreatitis, trauma (surgery), graft-versus-host disease, transplant rejection, heart disease including ischemic diseases such as myocardial infarction and atherosclerosis, intravascular coagulation, bone resorption, osteoporosis, osteoarthritis, periodontitis and hypochlorhydria, infertility related to lack of fetal-maternal tolerance, vitiligo, myasthenia gravis, or systemic sclerosis.

It has been proposed that an IFNy-dependent systemic immune response is beneficial for the treatment of Alzheimer's disease and other central nervous system pathologies that share neuroinflammatory components, and International Publication No. WO2015/136541 discloses the use of anti-PD-1 antibodies to treat Alzheimer's disease. International Publication No. WO2017/220990 discloses that blocking of the PD-1/PD-L1 inhibitory immune checkpoint pathway increases the secretion of IFNy by IFNy-producing cells, and the increased IFNy activity may enable the brain's choroid plexus to allow selective leukocyte trafficking and infiltration of T-cells and monocytes into the damaged central nervous system for homing of these immune cells to sites of neurodegeneration and neuroinflammation, and may modulate the environment to become less toxic and more permissive for clearance of toxic agents, rescue of neurons, regeneration, and repair.

PD-1 is associated not only with cognitive function, learning, and memory in the central nervous system, but also with other central nervous system disorders such as brain tumor, Alzheimer's disease, stroke, spinal cord injury, multiple sclerosis, glioblastoma, melanoma, and pain (Zunli Zao et al., "Emerging role of PD-1 in the central nervous system and brain diseases," Neurosci. Bull. 2021.04.20, online published). In addition, it has been reported that PD-1 is associated with retinal ganglion cells, which are known to degenerate in neurodegenerative diseases such as Alzheimer's disease, Parkinson's disease, multiple sclerosis, amyotrophic lateral sclerosis (ALS), and the like (Ling Chen et al., Role of the Immune Modulator Programmed Cell Death-1 during Development and Apoptosis of Mouse Retinal Ganglion Cells, Investigative Ophthalmology & Visual Science, 2009, Vol. 50, No. 10, 4941-4948). It is known that anti-PD-1 antibody can be used for neurodegenerative diseases by improving cognitive impairment and pathological characteristics in Alzheimer's disease model mouse 5XFAD and dementia model mouse (Michal Schwartz et al., "Potential immunotherapy for Alzheimer disease and age-related dementia," Dialogues in clinical neuroscience, 21(1), 21, 2019), and the anti-PD-1 antibody nivolumab has been reported to improve learning and memory (Ru-Rong Ji et al., "Anti-PD-1 treatment as a neurotherapy to enhance neuronal excitability, synaptic plasticity and memory," BioRxiv, 2019. 12. 10. Https://doi.org/10.1101.870600).

In one aspect of the present disclosure, the pharmaceutical composition according to the present disclosure can be used to prevent, ameliorate, and treat neurological disease and neurodegenerative disease associated with PD-1 including, but is not limited to, cognitive impairment, brain tumor, Alzheimer's disease, dementia, stroke, spinal cord injury, amyotrophic lateral sclerosis, Parkinson's disease, Huntington's disease, multiple sclerosis, glioblastoma, melanoma, pain, and memory loss.

In one aspect of the present disclosure, a pharmaceutical composition according to the present disclosure is useful for treating a subject suffering from a chronic viral infection. In some embodiments, it is useful for reducing viral titer and/or restoring exhausted T-cells in a host according to the present disclosure.

The infectious disease associated with PD-1 may be chronic viral infection including viral infection of hepatitis B, hepatitis C, herpes virus, Epstein-Barr virus, HIV, cytomegalovirus, herpes simplex virus type 1, herpes simplex virus type 2, human papilloma virus, adenovirus, Kaposi West sarcoma associated with herpes virus epidemics, thin ring virus (Torquetenovirus), lymphocytic choriomeningitis virus (LCMV), JC virus, or BK virus.

In one aspect, a pharmaceutical composition of the present disclosure can be used to treat infection by simian immunodeficiency virus (SIV) in a monkey subject, such as cynomolgus.

In one aspect, a pharmaceutical composition according to the present disclosure may be administered to relieve or prevent or decrease the severity of one or more of the symptoms or conditions of the disease or disorder. It is also contemplated herein to use a pharmaceutical composition of the present disclosure prophylactically to patients at risk for developing a disease or disorder such as cancer, autoimmune disease, and chronic viral infection.

In another embodiment of the present disclosure, a pharmaceutical composition of the present disclosure can be used as adjunct therapy with any other agent or any other therapy known to those skilled in the art useful for treating cancer, autoimmune disease, or viral infection.

A pharmaceutical composition in accordance with the present disclosure can be administered with suitable carriers, excipients, and other agents that are incorporated into formulations to provide improved transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax (see Powell et al. "Compendium of excipients for parenteral formulations" PDA (1998) J Pharm Sci Technol 52:238-311).

The dose of antibody may vary depending upon the age and the size of a subject to be administered, target disease, conditions, route of administration, and the like. When an antibody of the present disclosure is used for treating a disease or disorder in an adult patient, or for preventing such a disease, it is advantageous to administer the antibody of the present disclosure normally at a single dose of about 0.1 to about 60 mg/kg body weight, more preferably about 5 to about 60, about 10 to about 50, or about 20 to about 50 mg/kg body weight. Depending on the severity of the condition, the frequency and the duration of the treatment can be adjusted. In certain embodiments, the antibody or antigen-binding fragment thereof of the present disclosure can be administered as an initial dose of at least about 0.1 mg to about 800 mg, about 1 to about 500 mg, about 5 to about 300 mg, or about 10 to about 200 mg, to about 100 mg, or to about 50 mg. In certain embodiments, the initial dose may be followed by administration of a second or a plurality of subsequent doses of the antibody or antigen-binding fragment thereof in an amount that can be approximately the same or less than that of the initial dose, wherein the subsequent doses are separated by at least 1 day to 3 days; at least one week; at least 2 weeks; at least 3 weeks; at least 4 weeks; at least 5 weeks; at least 6 weeks; at least 7 weeks; at least 8 weeks; at least 9 weeks; at least 10 weeks; at least 12 weeks; or at least 14 weeks.

The pharmaceutical composition of the present disclosure can be administered through various delivery systems, for example, encapsulation in liposomes, microparticles, microcapsules, recombinant cells capable of expressing mutant viruses, and receptor mediated endocytosis (see, e.g., Wu et al. (1987) J. Biol. Chem. 262:4429-4432). Methods of introduction include, but are not limited to, intradermal, transdermal, intramuscular, intraperitoneal, intravenous, subcutaneous, intranasal, epidural, and oral routes. The composition may be administered by any conventional route, for example, by infusion or bolus injection or by absorption through epithelial or mucocutaneous linings (e.g., oral mucosa, rectal and intestinal mucosa, etc.), and may be administered together with other biologically active agents. Administration can be systemic or local.

The pharmaceutical composition can also be delivered in a vesicle, in particular a liposome (see, for example, Langer (1990) Science 249:1527-1533). The use of nanoparticles to deliver the antibodies of the present invention is also contemplated herein. Antibody-conjugated nanoparticles may be used both for therapeutic and diagnostic applications. Antibody-conjugated nanoparticles and methods of preparation and use are described in detail by Arruebo, M., et al. 2009, "Antibody-conjugated nanoparticles for biomedical applications" in J. Nanomat. Volume 2009, Article ID 439389, 24 pages, doi: 10.1155/2009/439389, which is incorporated herein by reference. Nanoparticles may be developed and conjugated to antibodies contained in pharmaceutical compositions to target tumor cells, autoimmune tissue cells, or virally infected cells. Nanoparticles for drug delivery have also been described in, for example, US 8,257,740 or US 8,246,995, each incorporated herein in its entirety.

In certain situations, the pharmaceutical composition can be delivered in a controlled release system. In one embodiment, a pump may be used. In another embodiment, polymeric materials can be used. In yet another embodiment, a controlled release system can be placed in proximity to the composition's target, thus requiring only a fraction of the systemic dose. The injectable preparations may include dosage forms for intravenous, subcutaneous, intracutaneous, intracranial, intraperitoneal, and intramuscular injections, drip infusions, etc. These injectable preparations may be prepared by methods publicly known. For example, the injectable preparations may be prepared by dissolving, suspending, or emulsifying the antibody or its salt described above in a sterile aqueous medium or an oily medium conventionally used for injections. As the aqueous medium for injections, there are, for example, physiological saline, an isotonic solution containing glucose, and other auxiliary agents, which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, sesame oil, soybean oil, etc. can be employed, which may be used in combination with a solubilizing agent such as benzyl benzoate, benzyl alcohol, etc. The injection thus prepared is preferably filled in an appropriate ampoule.

A pharmaceutical composition of the present disclosure can be delivered subcutaneously or intravenously with a standard needle and syringe. In addition, with respect to subcutaneous delivery, a pen delivery device readily has applications in delivering a pharmaceutical composition of the present disclosure. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded. Numerous reusable pen and autoinjector delivery devices have applications in the subcutaneous delivery of a pharmaceutical composition of the present disclosure. Examples include, but certainly are not limited to, AUTOPEN™ (Owen Mumford, Inc., Woodstock, UK), DISETRONIC™ pen (Disetronic Medical Systems, Burghdorf, Switzerland), HUMALOG MIX 75/25™ pen, HUMALOG™ pen, HUMALIN 70/30™ pen (Eli Lilly and Co., Indianapolis, Ind.), NOVOPEN™

I, II, and III (Novo Nordisk, Copenhagen, Denmark), NOVOPEN JUNIOR™ (Novo Nordisk, Copenhagen, Denmark), BD™ pen (Becton Dickinson, Franklin Lakes, N.J.), OPTIPEN™, OPTIPEN PRO™, OPTIPEN STARLET™, and OPTICLIK™ (Sanofi-Aventis, Frankfurt, Germany). Examples of disposable pen delivery devices having applications in subcutaneous delivery of a pharmaceutical composition of the present disclosure include, but certainly are not limited to, the SOLOSTAR™ pen (Sanofi-Aventis), the FLEXPEN™ (Novo Nordisk), the KWIKPEN™ (Eli Lilly), the SURECLICK™ Autoinjector (Amgen, Thousand Oaks, CA), the PENLET™ (Haselmeier, Stuttgart, Germany), the EPIPEN (Dey, L.P.), and the HUMIRA™ Pen (Abbott Labs, Abbott Park, IL).

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc. The amount of the antibody contained is generally about 5 to about 500 mg per dosage form in a unit dose; especially in the form of injection, it is preferred that the antibody is contained in about 5 to about 100 mg and in about 10 to about 250 mg for the other dosage forms.

In one aspect, the present disclosure provides a method for modulating an immune response in an individual comprising administering to the individual at least one selected from the group consisting of any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure.

In one aspect, the present disclosure provides use of a PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, or a multispecific antigen binding molecule, immunoconjugate, chimeric antigen receptor, engineered T cell receptor, or oncolytic virus comprising the above agent, antibody, fragment, or polypeptide according to the present disclosure in the manufacture of a medicament for the prevention, amelioration, or treatment of tumor, cancer, metastatic tumor, metastatic cancer, autoimmune disease, neurological disease, neurodegenerative disease, or infectious disease.

In one aspect, the present disclosure also provides a method of inhibiting the growth of tumor cells in an individual comprising administering to the individual at least one selected from the group consisting of a PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure in a therapeutically effective amount to inhibit the growth of tumor cells.

**Combination administration**

In one aspect of the present disclosure, the pharmaceutical composition according to the present disclosure provides a pharmaceutical composition further comprising a second therapeutic agent.

In various embodiments, the second therapeutic agent to be combined may be an antibody to PD-L1, a second antibody to PD-1 (e.g., nivolumab), a LAG-3 inhibitor, a CTLA-4 inhibitor (e.g., ipilimumab), a TIM3 inhibitor, a BTLA inhibitor, a TIGIT inhibitor, a CD47 inhibitor, an antagonist of another T-cell co-inhibitor or ligand (e.g., an antibody to CD-28, 2B4, LY108, LAIR1, ICOS, CD160 or VISTA), an indoleamine-2,3-dioxygenase (IDO) inhibitor, a vascular endothelial growth factor (VEGF) antagonist [e.g., a "VEGF-Trap" such as aflibercept or other VEGF-inhibiting fusion protein as set forth in US 7,087,411, or an anti-VEGF antibody or antigen binding fragment thereof (e.g., bevacizumab, ranibizumab) or a small molecule kinase inhibitor of VEGF receptor (e.g., sunitinib, sorafenib, or pazopanib)], an Ang2 inhibitor (e.g., nesvacumab), a transforming growth factor beta (TGFP) inhibitor, an epidermal growth factor receptor (EGFR) inhibitor (e.g., erlotinib, cetuximab), an agonist to a co-stimulatory receptor (e.g., an agonist to glucocorticoid-induced TNFR-related protein), an antibody to a tumor-specific antigen (e.g., CA9, CA125, melanoma-associated antigen 3 (MAGE3), carcinoembryonic antigen (CEA), vimentin, tumor-M2-PK, prostate-specific antigen (PSA), mucin-1, MART-1, and CA19-9), a vaccine (e.g., Bacillus Calmette-Guerin, a cancer vaccine), an adjuvant to increase antigen presentation (e.g., granulocyte-macrophage colony-stimulating factor), a bispecific antibody (e.g., CD3×CD20 bispecific antibody, PSMA×CD3 bispecific antibody), a cytotoxin, a chemotherapeutic agent (e.g., dacarbazine, temozolomide, cyclophosphamide, docetaxel, doxorubicin, daunorubicin, cisplatin, carboplatin, gemcitabine, methotrexate, mitoxantrone, oxaliplatin, paclitaxel, and vincristine), cyclophosphamide, radiotherapy, an IL-6R inhibitor (e.g., sarilumab), an IL-4R inhibitor (e.g., dupilumab), an IL-10 inhibitor, a cytokine such as IL-2, IL-7, IL-21, and IL-15, an antibody-drug conjugate (ADC) (e.g., anti-CD19-DM4 ADC, anti-DS6-DM4 ADC), an anti-inflammatory drug (e.g., corticosteroids, non-steroidal anti-inflammatory drugs), a dietary supplement such as anti-oxidants, or any palliative care to treat cancer.

In certain embodiments, the second therapeutic agent may include cancer vaccines including dendritic cell vaccines, oncolytic viruses, tumor cell vaccines, etc. to augment the anti-tumor response. Examples of cancer vaccines may include MAGE3 vaccine for melanoma and bladder cancer, MUC1 vaccine for breast cancer, EGFRv3 (e.g., Rindopepimut) for brain cancer (including glioblastoma multiforme), or ALVAC-CEA (for CEA⁺ cancers).

**Methods and kits for diagnosis and detection**

In one aspect, the present disclosure provides a kit for the treatment, diagnosis, or detection of a disease comprising at least one selected from the group consisting of any PD-1 binding agent, anti-PD-1 antibody or antigen-binding fragment thereof, immunoglobulin heavy chain variable region polypeptide, immunoglobulin light chain variable region polypeptide, immunoglobulin heavy chain polypeptide, immunoglobulin light chain polypeptide, and a multispecific antigen binding molecule, immunoconjugate, chimeric antigen receptor, engineered T cell receptor, or oncolytic virus comprising the above agent, antibody, fragment, or polypeptide according to the present disclosure.

The anti-PD-1 antibodies of the present disclosure may be used to detect and/or measure PD-1 in a sample, e.g., for diagnostic purposes. Some embodiments contemplate the use of one or more antibodies of the present disclosure in assays to detect a disease or disorder such as cancer, autoimmune disease, or chronic viral infection. Exemplary diagnostic assays for PD-1 may comprise, e.g., contacting a sample obtained from a patient with an anti-PD-1 antibody of the present disclosure, wherein the anti-PD-1 antibody is labeled with a detectable label or reporter molecule or used as a capture ligand to selectively isolate PD-1 from patient samples.

Alternatively, an unlabeled anti-PD-1 antibody can be used in diagnostic applications in combination with a secondary antibody which is itself detectably labeled. The detectable label or reporter molecule can be a radioisotope, such as 3H, 14C, 32P, 35S, or 125I; a fluorescent or chemiluminescent moiety, such as fluorescein isothiocyanate or rhodamine; or an enzyme such as alkaline phosphatase, β-galactosidase, horseradish peroxidase, or luciferase.

Specific exemplary assays that can be used to detect or measure PD-1 in a sample include enzyme-linked immunosorbent assay (ELISA), radioimmunoassay (RIA), and fluorescence-activated cell sorting (FACS).

Samples that can be used in PD-1 diagnostic assays according to the present disclosure include any tissue or fluid sample obtainable from a patient which contains detectable quantities of either PD-1 protein or fragments thereof under normal or pathological conditions. Generally, levels of PD-1 in a particular sample obtained from a healthy patient (e.g., a patient not afflicted with cancer or an autoimmune disease) will be measured to initially establish a baseline or standard level of PD-1. This baseline level of PD-1 can then be compared against the levels of PD-1 measured in samples obtained from individuals suspected of having a cancer-related condition or symptoms associated with such condition.

The polypeptide, PD-1 binding agent, antibody, and the like specific for PD-1 according to the present disclosure may contain no additional labels or moieties, or they may contain an N-terminal or C-terminal label or moiety. In one embodiment, the label or moiety is biotin. In a binding assay, the location of a label (if any) may determine the orientation of the peptide relative to the surface upon which the peptide is bound. For example, if a surface is coated with avidin, a peptide containing an N-terminal biotin will be oriented such that the C-terminal portion of the peptide will be distal to the surface.

An aspect of the present disclosure relates to use of the disclosed antibodies as markers for predicting prognosis of cancer or an autoimmune disorder in patients. The polypeptide, PD-1 binding agent, antibody, and the like according to the present disclosure may be used in diagnostic assays to evaluate prognosis of cancer in a patient and to predict survival.

Hereinafter, a method for producing an antibody according to specific embodiments of the present invention will be described in more detail. However, this is presented as an example of the invention, thereby not limiting the scope of the invention. It is apparent to those skilled in the art that various modifications to the embodiments are possible within the scope of the invention.

**Examples**

**Example 1. Preparation of immunogen and establishment of cell lines**

**Protein antigen**

From the pCMV3-C-FLAG vector (Sino) containing the cDNA of human PD-1, the extracellular domain was synthesized by PCR and inserted into the pEM.CMV-SF-IRES-EGFP vector. The constructed vector was transfected into a CHO-S cell line, and cells generating the human PD-1 extracellular domain were selected based on EGFP expression through flow cytometry. Human PD-1 was purified and quantified from cell culture media using FLAG tag affinity chromatography.

**Cell line antigen**

The pCMV3-C-FLAG vector (Sino) containing human PD-1 cDNA was transfected into a CT26 cell line derived from BALB/c mouse colon cancer. Cells expressing human PD-1 were selected by flow cytometry using the APC-cy7 anti-human PD-1 antibody. A single clone was obtained by limiting-dilution in a 96-well plate.

**Acquisition of control groups (Keytruda, Opdivo)**

As a control antibody, Keytruda, a product manufactured by InvivoGen (human IgG4(S228P) isotype, cat.No. hpd1pe-mab14) or a product for clinical use from MSD was purchased and used. Opdivo, a product for clinical use from BMS, was purchased and used.

**Establishment of cell lines**

The pCMV3-C-FLAG vector (Sino) containing cDNA of human PD-1 or mouse PD-1 was transfected into the CHO-S cell line. Cells expressing human PD-1 or mouse PD-1 were selected by flow cytometry using APC-cy7 anti-human PD-1 antibody or APC-cy7 anti-mouse PD-1 antibody. A single clone was obtained by limiting-dilution in a 96-well plate.

**Example 2. Construction of PD-1 knockout mice**

PD-1 knockout mice were constructed using the CRISPR-CAS system in mice (C57BL/6N). After preparing a guide RNA specific for the mouse PD-1 gene sequence and checking whether the mouse PD-1 DNA can be degraded *in vitro,* the guide RNA and Cas9 protein were microinjected into the zygote. Among the guide RNA-injected zygotes, the surviving ones were selected and transplanted into the oviducts of surrogate mothers. After transplantation, the tail was cut from the 2-week-old mice to extract genomic DNA, and the deletion of the mouse PD-1 gene was confirmed by PCR.

Example 3. **Generation of antibody hybridomas**

**Immunization of mice**

8-week-old female PD-1 knockout mice were immunized with human PD-1 antigen to induce antibody production. As antigens for immunization, purified human PD-1 antigen and CT26-derived human PD-1 cell line antigen were used.

For protein antigen immunization, 50 $\mu$g of antigen protein per mouse was mixed with 50 $\mu$g of Titermax gold adjuvant and injected subcutaneously (s.c.) into the left and right back of the mice. For cell line antigen immunization, X-ray irradiation was performed on the cell line antigen 1 day before immunization to suppress cell growth, and $1\times10^6$ cells per mouse were administered intraperitoneally (i.p.). Immunizations were performed at 3-week intervals, and blood was collected by submandibular bleeding from each mouse 10 days after immunization. The titer of the antibody generated in the serum isolated from the blood was measured by ELISA.

**Cell fusion and generation of monoclonal cells (hybridomas)**

B lymphocytes were isolated from the spleen removed from the human PD-1 immunized mice and then fused with cultured myeloma cells (sp2/0). The fused cells were cultured in a medium supplemented with hypoxanthine, aminopterine, and thymidine (HAT medium), and only the hybridomas which are a fusion of myeloma and B lymphocytes were selectively chosen and cultured.

Among the obtained hybridoma cells, a hybridoma producing an antibody that reacts with the human PD-1 antigen was identified by performing a protein-based ELISA analysis. The hybridoma that reacts with human PD-1 was repeatedly cloned using the limiting dilution method, and monoclonal cells (hybridoma) (1G1) that produce an antibody responding to human PD-1 antigen were obtained.

**Example 4. Binding test of hybridoma 1G1 to PD-1**

To confirm the binding between the monoclonal cell (hybridoma) (1G1) prepared in Example 3 and the conformational PD-1 protein expressed on the cell surface, cell-based ELISA and flow cytometry analysis were performed. As an anti-hPD-1 antibody control, Keytruda (Invivogen) was used.

To summarize the ELISA assay, 10,000 CHO-S cells expressing human or mouse PD-1 were coated in collagen-coated 96-well plates (ThermoFisher) overnight at 37°C. The coated cells were fixed with 8% paraformaldehyde at room temperature for 15 minutes. After blocking and washing, Keytruda or hybridoma supernatant was added to the coated plate and incubated for 2 hours at room temperature. After washing, secondary antibody was added and incubated overnight at 4°C. As the secondary antibody, mouse anti-human IgG Fc HRP (GenScript) was used in the wells containing Keytruda, and goat anti-mouse IgG Fc HRP (ThermoFisher) was used in the wells added with the hybridoma supernatant. After washing, TMB substrate (abcam) was added, and the color reaction was stopped with STOP solution (abcam). Absorbance at 450/650 nm was measured using a microplate reader (ThermoFisher).

To summarize the flow cytometry, CHO-S cells expressing human or mouse PD-1 were loaded into a 96-well V-bottom plate (Corning) at a density of $1 \times 10^6$ cells/well, and either Keytruda or hybridoma supernatant was added and incubated at 4°C for 1 hour. After washing with 1XPBS/2%BSA, a secondary antibody was added and incubated with the cells at 4°C for 1 hour. As the secondary antibody, PE anti-human IgG Fc (Biolegend) was used in the wells added with Keytruda, and AF647 goat anti-mouse IgG (H+L) (ThermoFisher) was used in the wells added with the hybridoma supernatant. Thereafter, the cells were washed and resuspended in 1XPBS/2%BSA and analyzed by flow cytometry (BD) and FlowJo software.

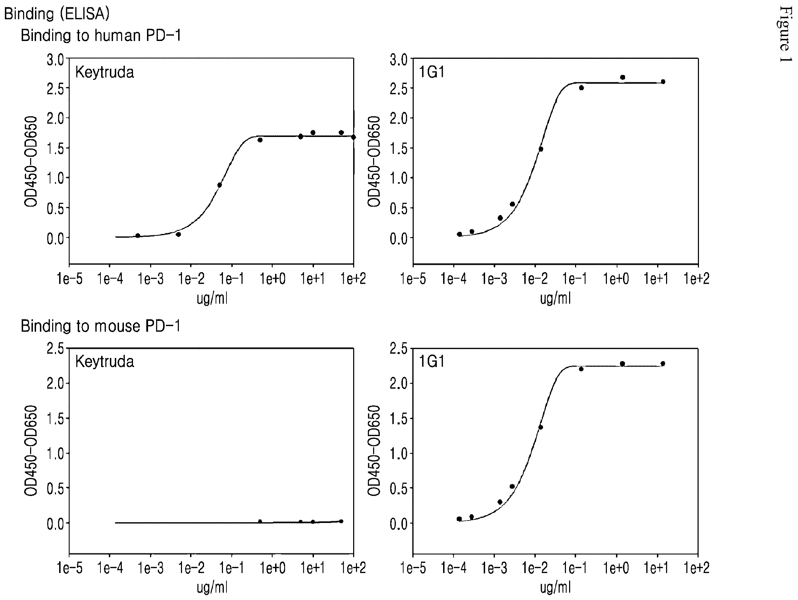

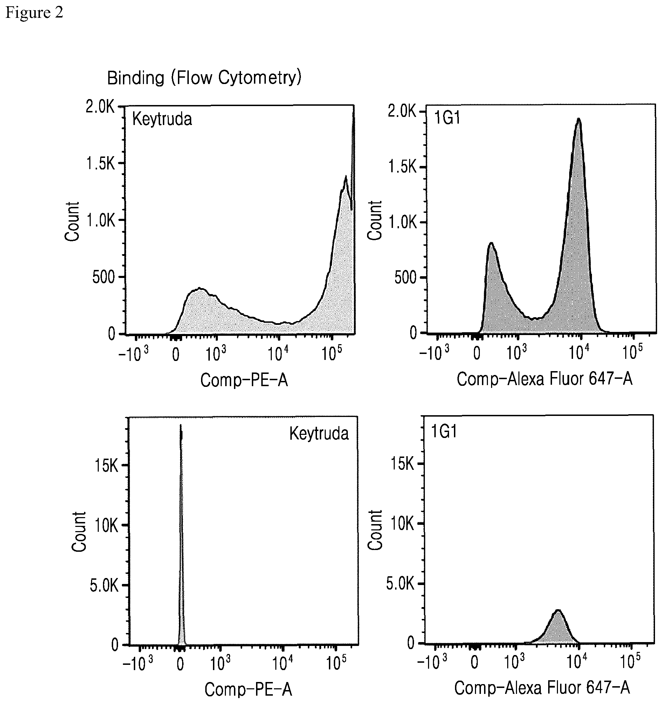

FIG. 1 provides graphs showing the results of a binding test of the hybridoma antibody 1G1 prepared in Example 3 to the cell surface human PD-1 or mouse PD-1 using ELISA. FIG. 2 provides graphs showing the results of the binding test of the hybridoma antibody prepared in Example 3 to the cell surface human PD-1 or mouse PD-1 using flow cytometry. The 1G1 antibody bound to human PD-1 and mouse PD-1 with high affinity.

On the other hand, Keytruda, used as a control, bound only to the cell surface human PD-1, whereas the 1G1 hybridoma antibody prepared in Example 3 bound to both the cell surface human PD-1 and mouse PD-1, showing cross-reactivity.

### Example 5. Ligand blocking test of hybridoma 1G1 antibody

In order to determine whether the monoclonal cell (hybridoma) 1G1 prepared in Example 3 blocks the binding of PD-L1 to cell surface PD-1, cell-based ELISA and flow cytometry analysis were performed. As an anti-hPD-1 antibody control, Keytruda (Invivogen) was used.

To summarize the ELISA assay, 10,000 CHO-S cells expressing human or mouse PD-1 were coated in collagen-coated 96-well plates overnight at 37°C. The coated cells were fixed with 8% paraformaldehyde at room temperature for 15 minutes. After blocking and washing, human PD-L1 llama Fc protein was added to the human PD-1 CHO-S cell-coated wells, and mouse PD-L1 human Fc protein was added to the mouse PD-1 CHO-S cell-coated wells; the plates were then incubated at room temperature for 20 minutes. Keytruda or hybridoma supernatant was added and incubated for 1 hour and 20 minutes at room temperature.

After washing, a secondary antibody was added and incubated overnight at 4°C. As the secondary antibody, mouse anti-llama IgG2/IgG3 HRP (antibody onlines) was used in the wells where human PD-L1 llama Fc protein was added, and mouse anti-human IgG Fc HRP (Genscript) was used in the wells where mouse PD-L1 human Fc protein was added. After washing, TMB substrate (abcam) was added, and the color reaction was stopped with STOP solution (abcam). Absorbance at 450/650 nm was measured using a microplate reader (ThermoFisher).

To summarize the flow cytometry analysis, CHO-S cells expressing human or mouse PD-1 were loaded into a 96-well V-bottom plate (Corning) at a density of $1 \times 10^6$ cells/well, and a mixture of PD-L1 protein and Keytruda or the hybridoma supernatant was added and incubated at 4°C for 1 hour. A mixture of human PD-L1 llama Fc protein and the antibody was added to the wells loaded with the human PD-1 CHO-S cells, and a mixture of the mouse PD-L1 human Fc protein and the antibody was added to the wells loaded with mouse PD-1 CHO-S cells. After washing with 1XPBS/2%BSA, a secondary antibody was added and incubated at 4°C for 1 hour.

As a secondary antibody, FITC goat anti-llama IgG (abcam) was used in the wells containing the mixture of the human PD-L1 llama Fc protein and the antibody, and PE anti-human IgG Fc (Biolegend) was used in the wells containing the mixture of the mouse PD-L1 human Fc protein and the antibody. After washing, the cells were resuspended in 1XPBS/2%BSA and analyzed by flow cytometry (BD) and FlowJo software.

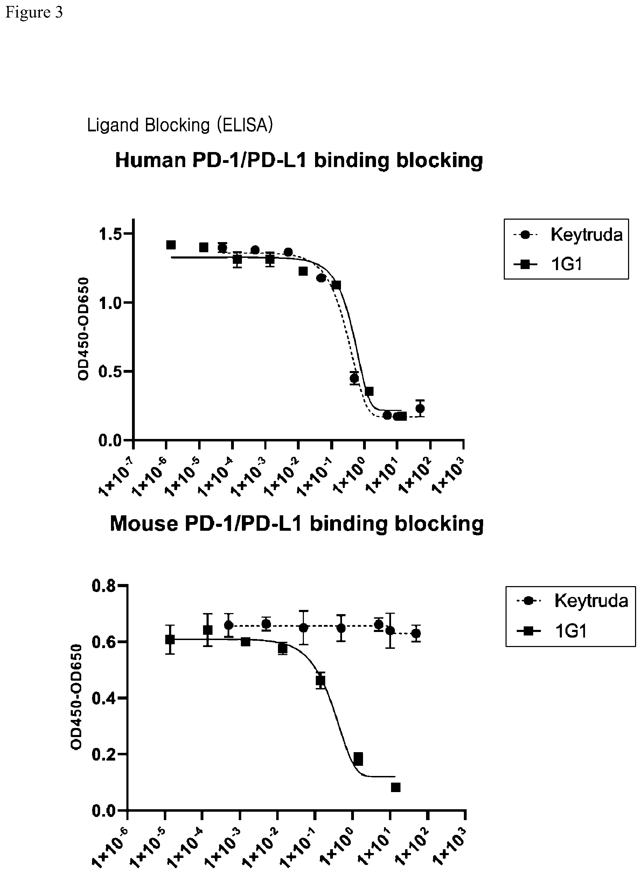

FIG. 3 illustrates graphs showing the test results using ELISA to determine whether the monoclonal cell (hybridoma) 1G1 antibody prepared in Example 3 blocks the binding of human PD-L1 and mouse PD-L1 to the cell surface human PD-1 and mouse PD-1, respectively.

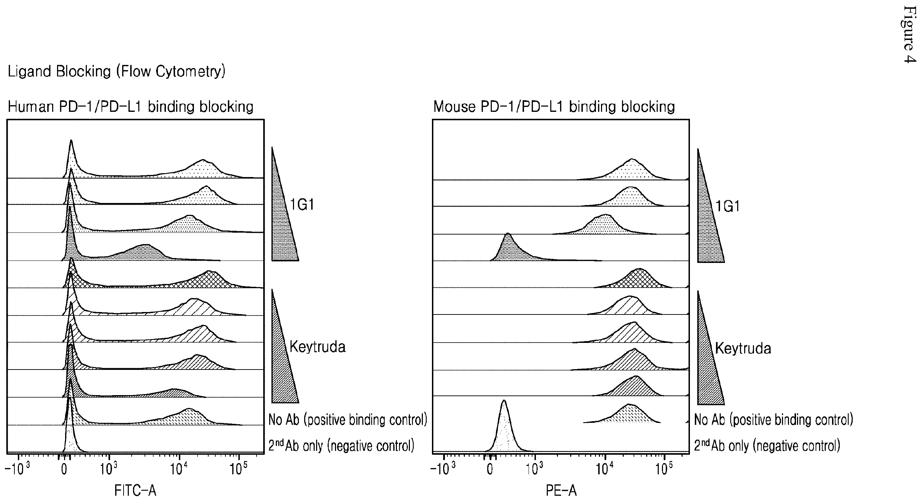

FIG. 4 illustrates graphs showing the test results using flow cytometry to determine whether the monoclonal cell (hybridoma) 1G1 antibody prepared in Example 3 blocks the binding of human PD-L1 and mouse PD-L1 to the cell surface human PD-1 and mouse PD-1, respectively.

The control group, Keytruda, only blocked the binding between the cell surface human PD-1 and PD-L1, whereas the 1G1 hybridoma antibody blocked the binding of both human PD-1/PD-L1 and mouse PD-1/PD-L1.

**Example 6. Binding test of purified hybridoma** 1G1 **antibody to PD-1 (ELISA)**

In order to produce antibodies in the hybridoma, the hybridoma was cultured in DMEM (HyClone, Cytiva) medium containing 3% low-IgG FBS (Gibco) for 1 week, centrifuged, and then filtered with a 0.22 μm filter (Millipore) to recover the cell culture medium.

Antibody proteins were isolated from the hybridoma cell culture medium using affinity chromatography. The hybridoma cell culture medium was loaded on a chromatography column (Bio Rad) containing Protein G beads (Cytiva), and elution was performed with IgG Elution Buffer (Thermo scientific). To minimize protein damage due to the low pH IgG Elution Buffer (pH 2.5-3.0), the antibody was eluted into a tube containing 1 M Tris-HCl solution (pH 8.0), and the pH of the neutralized protein was measured using a pH-indicator strip.

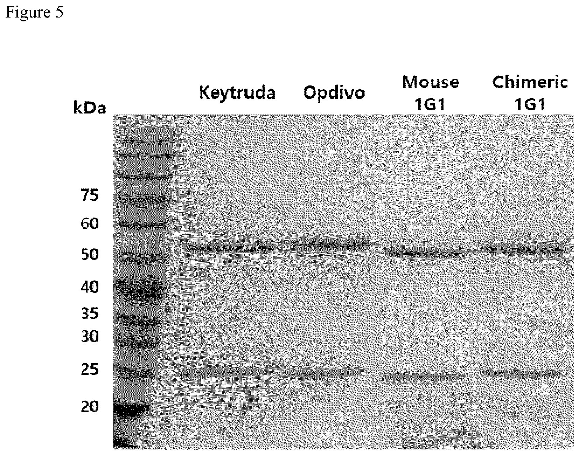

The purified antibody was concentrated using Amicon 100K centrifugal filter (Merck), and protein identification and quantification were performed through Coomassie blue staining and BCA assay (Thermo scientific) (FIG. 5).

In order to measure the binding affinity between the purified antibody 1G1 and PD-1 protein of normal structure expressed on the cell surface, cell-based ELISA was performed. As anti-hPD-1 antibody controls, Keytruda (MSD) and Opdivo (BMS) were used.

To summarize the ELISA analysis, 10,000 CHO-S cells expressing either human PD-1 or mouse PD-1 were coated in a collagen-coated 96-well plate (Thermo scientific) overnight in an incubator at 37°C. The coated cells were fixed with 8% paraformaldehyde at room temperature for 15 minutes. After blocking and washing, Keytruda, Opdivo, and the purified hybridoma anti-PD-1 antibody were added to the coated plate and incubated at room temperature for 2 hours.

After washing, a secondary antibody was added and incubated overnight at 4°C. As the secondary antibody, mouse anti-human IgG Fc HRP (GenScript) was used for the wells added with Keytruda or Opdivo, and goat anti-mouse IgG Fc HRP (ThermoFisher) was used for the wells added with the purified hybridoma anti-PD-1 antibody. After washing, TMB substrate (abcam) was added, and the color reaction was stopped with STOP solution (abcam). Absorbance at 450/650 nm was measured using a microplate reader (ThermoFisher).

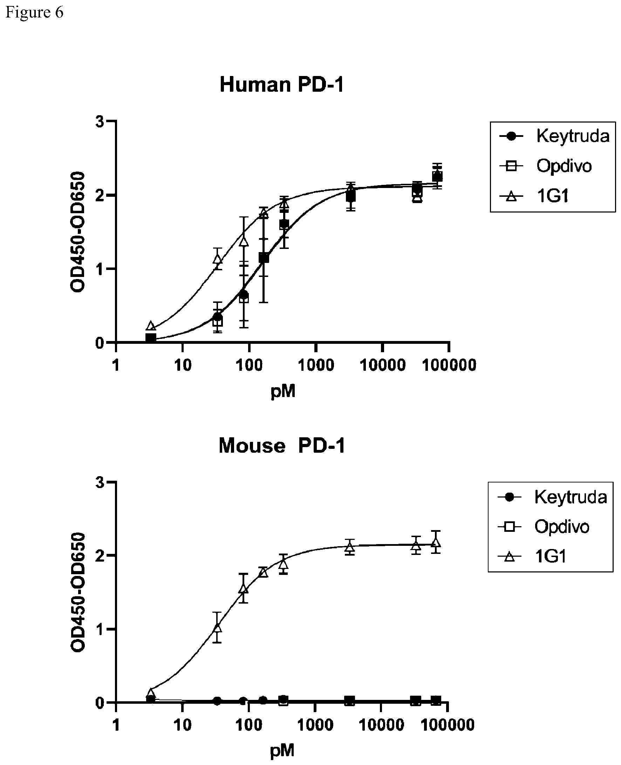

FIG. 6 illustrates graphs showing the binding test results using ELISA to measure the binding activity of the purified hybridoma 1G1 antibody to the cell surface human PD-1 or mouse PD-1. From the test results, the EC50 values of the purified mouse 1G1 antibody and the control group (Keytruda, Opdivo) for the antigen were calculated.

The purified hybridoma anti-PD-1 antibody (1G1) showed about 5-fold improved binding activity (28.13 pM) to the human PD-1 antigen compared to Keytruda (153.52 pM) and Opdivo (157.27 pM). In addition, the purified hybridoma anti-PD-1 antibody (1G1) bound to the mouse PD-1 antigen with a binding activity (31.72 pM) similar to that to the human PD-1 antigen.

|  | Keytruda | Opdivo | Purified hybridoma 1G1 |
|---|---|---|---|
| $EC_{50}$: hPD-1 (pM) | 153.52 | 157.27 | 28.13 |
| $EC_{50}$: mPD-1 (pM) | ND | ND | 31.72 |
| ND: Not Detected | | | |

**Example 7. Cross-reactivity to human immune checkpoints**

In order to determine whether the monoclonal cell (hybridoma) 1G1 prepared in Example 3 specifically binds to PD-1 present on the surface of human T cells, cross-reactivity for the other immune checkpoints were tested using a protein-based ELISA analysis. As an anti-hPD-1 antibody control, Keytruda (Invivogen) was used.

Human PD-1, CD28, CTLA-4, ICOS, and BTLA proteins were coated in 96-well plates (Nunc) overnight at 4°C. After blocking and washing, Keytruda or the hybridoma culture supernatant was added to the coated plates and incubated at 37°C for 1 hour. After washing, a secondary antibody was added and incubated at 37°C for 2 hours. For the secondary antibody, mouse anti-human IgG Fc HRP (Genscript) was used in the wells added with Keytruda, and goat anti-mouse IgG Fc HRP (ThermoFisher) was used in the wells added with the hybridoma supernatant. After washing, TMB substrate (abcam) was added, and the color reaction was stopped with STOP solution (abcam). Absorbance at 450/650 nm was measured using a microplate reader (ThermoFisher).

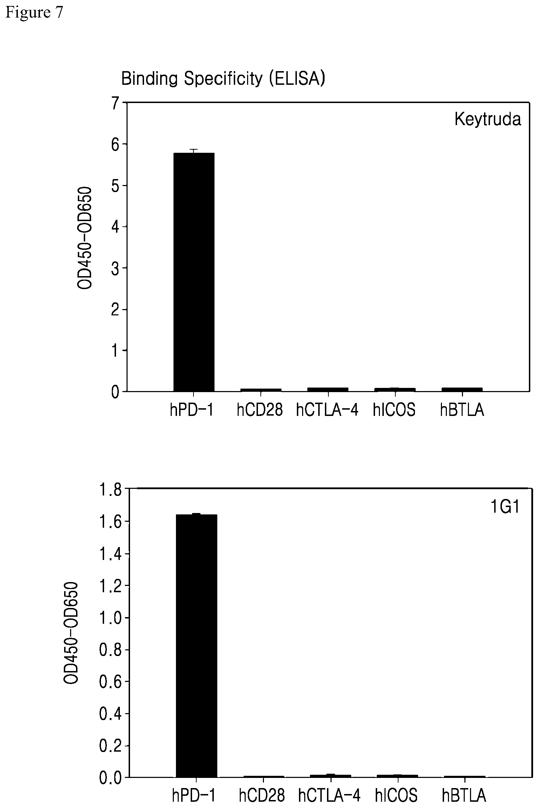

FIG. 7 is a result of testing by ELISA to measure the binding to human T cell surface immune checkpoints. The 1G1 hybridoma antibody specifically bound only to human PD-1 as did the control (Keytruda).

## Example 8. Antibody hybridoma cell sequencing

RNA was extracted from the 1G1 hybridoma cells prepared in Example 3 with Trizol reagent. cDNA was synthesized from RNA using reverse transcriptase, and the VH and VL sequences of the antibody were amplified from the synthesized cDNA as follows:

Primers for the sequence amplification are as follows:

[Table 1]

|  |  | Primer | Sequence (5'-> 3') |
|---|---|---|---|
| VH | Forward | MH1 | AATTTGCTAGCSARGTNMAGCTGSAGSAGTC(SEQ ID NO. 33) |
|  | Forward | MH2 | AATTTGCTAGCSARGTNMAGCTGSAGSAGTCWGG(SEQ ID NO. 34) |
|  | Reverse | IgG1 | AATTTGGATCCATAGACAGATGGGGGTGTCGTTTTGGC(SEQ ID NO. 35) |
| VL | Forward | MK | AATTGGATCCAGGGGCCAGTGGATAGACTGATGG(SEQ ID NO. 36) |
|  | Reverse | CK | AATTTGCGGCCGCGGATACAGTTGGTGCAGCATC (SEQ ID NO.37) |

The PCR reaction was performed as follows:

[Table 2]

| Reaction system (50 μl) |  | Reaction condition |  |  |
|---|---|---|---|---|
| cDNA | 1 μl | 95°C | 5min | 1 cycle |
| 10 x Taq buffer | 5 μl | 95°C | 1min | |
| dNTP (2.5mM) | 4 μl | 45°C | 1min | 30 cycles |
| Forward Primer (100 pmol/μl) | 1 μl | 72°C | 1min | |
| Reverse primer (100 pmol/μl) | 1 μl | 72°C | 5min | 1 cycle |
| Taq (5U/μl) | 0.25 μl | | | |
| TDW | 37.75 μl | | | |

The obtained PCR product (10 μL) was ligated with the pCMV3 vector, and the VH and VL sequences of the hybridoma antibody was identified by sequence analysis using T7 (5'-TAATACGACTCACTATAGGG-3') and pCMV3_F (5'-CGAGGAGGATTTGATATTCAC-3') primers.

The identified VH and VL sequences are as follows, and FR1-4 and CDR1-3 sequences were identified in both VH and VL according to the Kabat system.

[Table 3]

| Heavy (Kabat system) | |
|---|---|
| HFR1 | EVQLQQSGAELVKPGASVKLSCKASGYTFT (SEQ ID NO. 17) |
| CDR-H1 | GYWMH (SEQ ID NO. 1) |
| HFR2 | WVKQRPGQGLEWIG (SEQ ID NO. 19) |

(continued)

| Heavy (Kabat system) | |
|---|---|
| CDR-H2 | MIHPNSDTTTYNEKFKN (SEQ ID NO. 3) |
| HFR3 | RATLTVDKSSGTAYMQLSSLTSEDSAVYYCTG (SEQ ID NO. 21) |
| CDR-H3 | TDQAAWFAF (SEQ ID NO. 5) |
| HFR4 | WGQGTLVTVSA (SEQ ID NO. 23) |
| Kappa (Kabat system) | |
| LFR1 | DIVLTQTPLSLPVSLGDQASISC (SEQ ID NO. 25) |
| CDR-L1 | RSSQNIVHSNGDTYLE (SEQ ID NO. 7) |
| LFR2 | WYLQKPGQSPKLLIY (SEQ ID NO. 27) |
| CDR-L2 | KVSKRFS (SEQ ID NO. 9) |
| LFR3 | GVPDRFSGSGSGTDFTLKISRVEAEDLGVYYC (SEQ ID NO. 29) |
| CDR-L3 | FQGSHVPWT (SEQ ID NO. 11) |
| LFR4 | FGGGTKLEIK (SEQ ID NO. 31) |

## Example 9. Construction, expression and purification of chimeric antibodies

A chimeric antibody was prepared in which the constant region of the mouse anti-PD-1 antibody produced in the hybridoma was replaced with that of a human antibody. Signal peptide and VH or VL sequences of the antibody were linked by overlapping PCR and ligated to pTRIOZ-hIgG4 vector (Invivogen) expressing human IgG4 constant region (S228P) (SEQ ID NOs: 38 to 41).

pTRIOZ-hIgG4 vector containing VH and VL sequences of the antibody was introduced into the ExpiCHO™ expression of system (Thermo scientific) to express the antibody. After transfecting the pTRIOZ-hIgG4 vector into ExpiCHO-S cells, the cells were cultured in the ExpiCHO™ Expression Medium that does not contain FBS, and the cell viability was observed. Seven to ten days after transfection, cell cultures with viability of at least 70% were centrifuged and filtered with a 0.22 $\mu$m filter (Millipore) to recover the cell culture medium. The cell culture medium was loaded on a chromatography column (Bio Rad) containing Protein A beads (Thermo scientific), and elution was performed with IgG Elution Buffer (Thermo scientific). To minimize protein damage due to low pH IgG Elution Buffer (pH 2.5-3.0), the antibody was eluted into a tube containing 1 M Tris-HCl solution (pH 8.0), and the pH of the neutralized protein was measured using a pH-indicator strip. The purified antibody was concentrated using Amicon 50K centrifugal filter (Merck), and protein identification and quantification were performed through Coomassie blue staining and BCA assay (Thermo scientific). FIG. 5 is an SDS-PAGE result identifying the purified mouse 1G1 antibody (1G1 parental) and the chimeric 1G1 antibody (1G1 Chimeric).

## Example 10. Binding test of chimeric 1G1 antibody to PD-1 (SPR)

The binding kinetics of the chimeric 1G1 antibody prepared in Example 9 to human PD-1 was measured by SPR (Surface Plasma Resonance) analysis using Biacore 8K (Cytiva). Anti-human IgG antibody was immobilized on a CM5 chip (Cytiva) via amine coupling. Purified antibodies (Keytruda, Opdivo, chimeric 1G1 antibody) were flowed over the sensor chip and captured by anti-human IgG antibody. Human PD-1 and running buffer at a concentration of 0-100 nM (0, 6.25, 12.5, 25, 50, 100 nM) were flowed onto the sensor chip at a flow rate of 30 $\mu$l/min for an association phase of 120 s, which was followed by a dissociation phase of 900 s. The chip was regenerated with glycine at pH 1.5 after each experiment. Association and dissociation curves were drawn using Cytiva evaluation software, and kinetics and affinity values were determined.

The table below shows the binding affinity of the chimeric 1G1 antibody to human PD-1, tested by SPR assay.

| | Keytruda | Opdivo | Chimeric 1G1 |
|---|---|---|---|
| Kon (1/Ms) | 4.96e+5 | 2.04e+5 | 2.23e+5 |

(continued)

|  | Keytruda | Opdivo | Chimeric 1G1 |
|---|---|---|---|
| Koff (1/s) | 3.14e-3 | 2.14e-3 | 7.49e-4 |
| KD (M) | 6.33e-9 | 1.05e-8 | 3.35e-9 |

The chimeric anti-PD-1 antibody (1G1) had about 2-3 times superior binding affinity (KD, 3.35e-9) for human PD-1 compared to Keytruda (6.33e-9) and Opdivo (1.05e-8). This is due to the improved binding properties of the 1G1 chimeric anti-PD-1 antibody with an association rate (Kon, 2.23e +5) similar to (0.9-2.2 fold) Keytruda (4.96e+5) and Opdivo (2.04e+5) but 2.9-4.2 times slower dissociation (Koff, 7.49e-4) compared to Keytruda (3.14e-3) and Opdivo (2.14e-3).

Example 11. **Epitope mapping of 1G1 antibody**

Epitope mapping (alanine scanning) of the 1G1-chimeric (hIgG4(S228P)) antibody to the human PD-1 antigen (SEQ ID NO: 62) was performed to determine the human PD-1 antigen epitope recognized by the antibody. Selected amino acid residues of PD-1 were mutated to alanine using PCR mutagenesis. The mutated proteins were expressed and analyzed by high-throughput flow cytometry for binding to the 1G1 chimeric antibody.

As a result, three epitopes (P130, L128, and I126) whose mutation resulted in a reduction of the binding activity to 50% or less of the binding activity of the wild-type antigen were identified. All of these amino acids are conserved in mouse PD-1, supporting the cross-reactivity of the 1G1 antibody between human and mouse PD-1.

**Example 12. Humanization of 1G1 antibody**

In order to remove the immunogenicity of the mouse-derived 1G1 antibody and secure stable antibody efficacy in the human body, humanization of the 1G1 antibody was performed using a back mutation library method. The framework (FR) sequences of the 1G1 antibody excluding the complementarity determining region (CDR) sequences were replaced with human antibody sequences to obtain three 1G1 humanized antibodies (humanized antibodies 1G1-h61, 1G1-h68, 1G1-h70), which were purified with protein A affinity chromatography.

The binding affinities of the obtained three 1G1 humanized antibodies (humanized antibodies 1G1-h61, 1G1-h68, 1G1-h70) for the human PD-1 antigen were analyzed by SPR (Surface Plasma Resonance) analysis using Biacore 8K (Cytiva). All three humanized 1G1 antibodies had antigen-binding affinity similar to that of the 1G1-chimeric antibody.

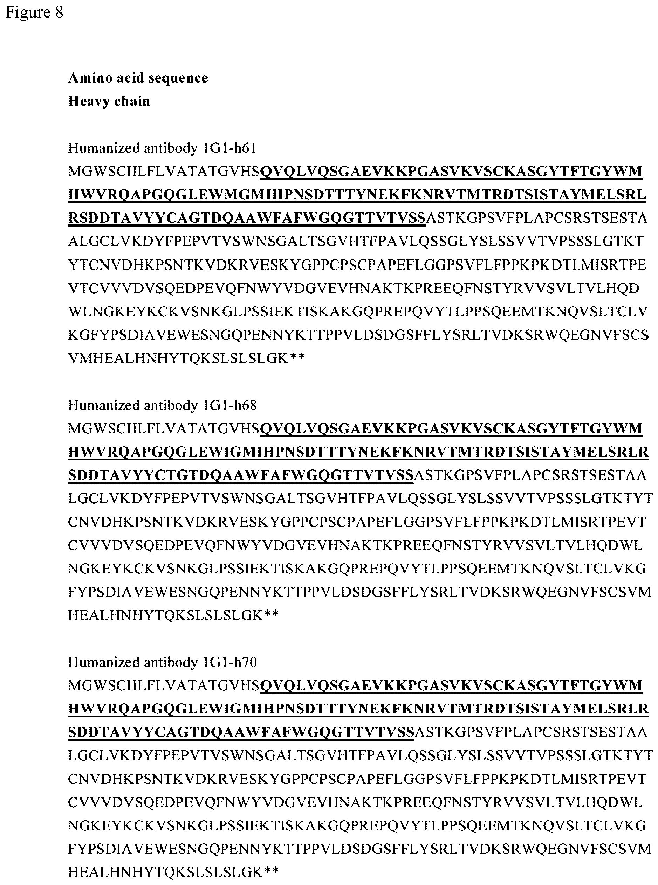

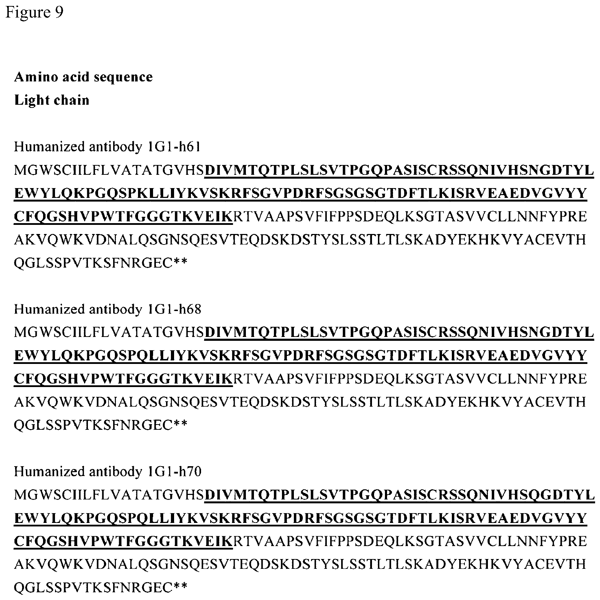

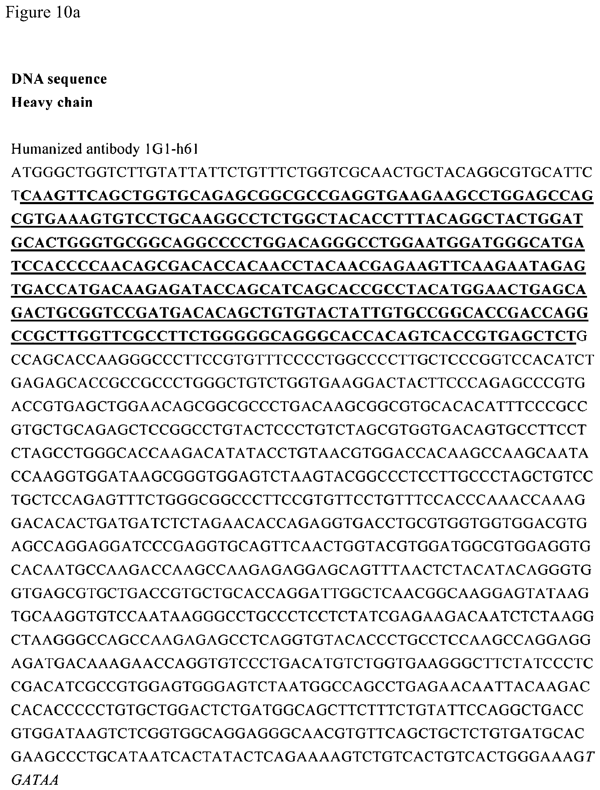

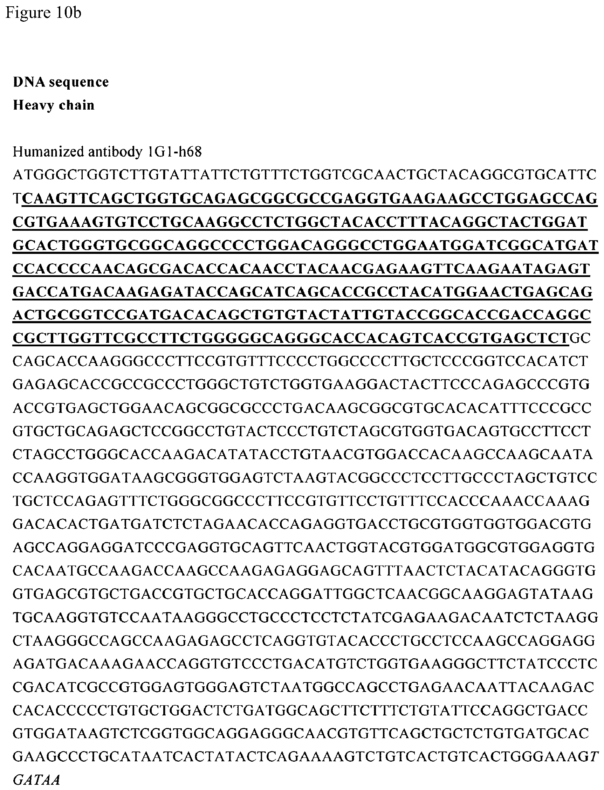

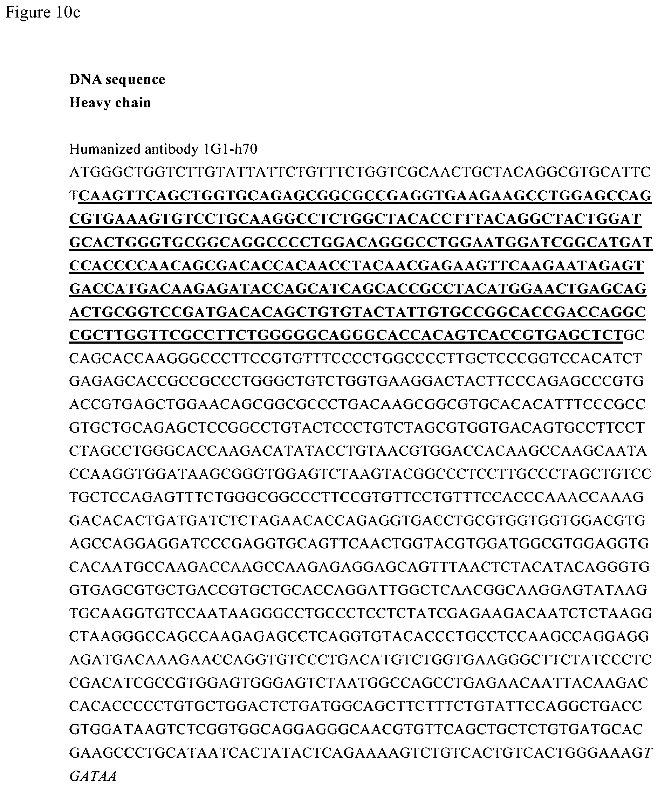

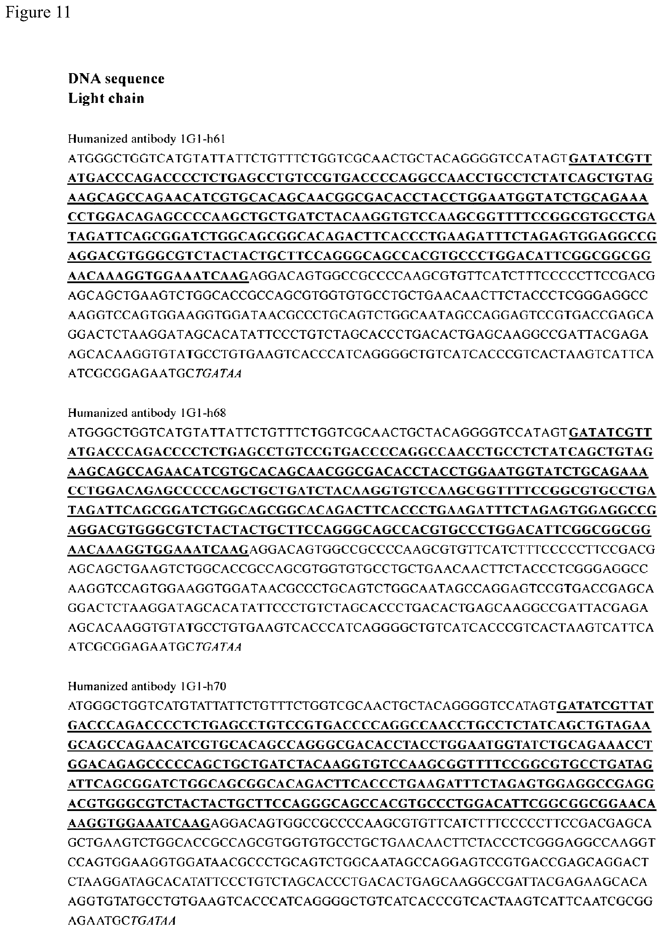

The sequences of each of the obtained three humanized 1G1 antibodies were analyzed and shown in FIGs. 8 to 11. Specifically, the heavy chain variable region (VH) and light chain variable region (VL) of the humanized antibody 1G1-h61 have the amino acid sequences of SEQ ID NOs: 54 and 55, respectively. The heavy chain variable region (VH) and light chain variable region (VL) of the humanized antibody 1G1-h68 have the amino acid sequences of SEQ ID NOs: 56 and 57, respectively. The heavy chain variable region (VH) and light chain variable region (VL) of the humanized antibody 1G1-h70 have the amino acid sequences of SEQ ID NOs: 58 and 59, respectively.

**Example 13. Comparative evaluation of binding kinetics of humanized** 1G1 **antibodies to PD-1 antigen**

The binding kinetics of each antibody to human PD-1 were measured through SPR (Surface Plasma Resonance) analysis using Biacore 8K (Cytiva), and their binding affinities to the antigen were compared.

Anti-human PD-1 antibody controls, Keytruda (MSD (Lot #T020031)) and Opdivo (BMS (Lot #043 FB)), were products for human use purchased from Shinwon Pharmacy Co., Ltd. Control antibodies (Keytruda, Opdivo) and 1G1 antibodies (chimeric antibody 1G1-chimeric, humanized antibodies 1G1-h61, 1G1-h68, and 1G1-h70) were flowed onto a Protein A chip (Cytiva) and captured. Seven concentrations (0-100 M) of human PD-1 were flowed onto the sensor chip at a flow rate of 30 μL/min for an association phase of 120 s, followed by dissociation for 1800 s. The chip was regenerated with glycine at pH 1.5 after each experiment. Association and dissociation curves were drawn using Cytiva evaluation software, and kinetics and affinity values were measured. One-way ANOVA with Tukey test of GraphPad Prism program was performed to determine if there was a significant difference in kinetics and affinity values between the antibodies (****, $P < 0.0001$).

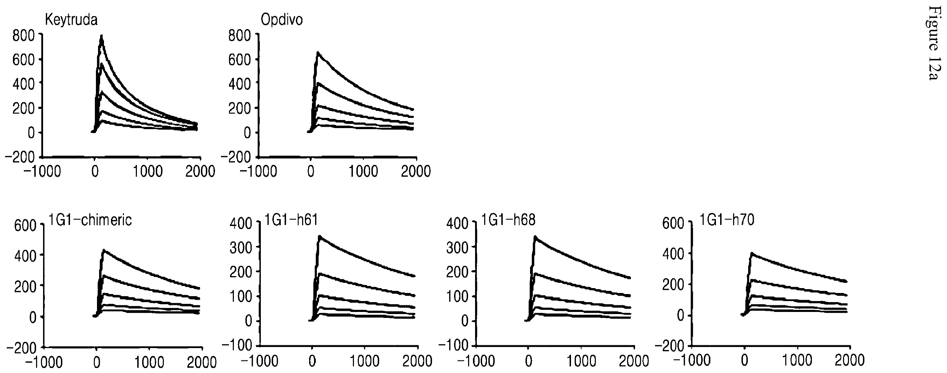

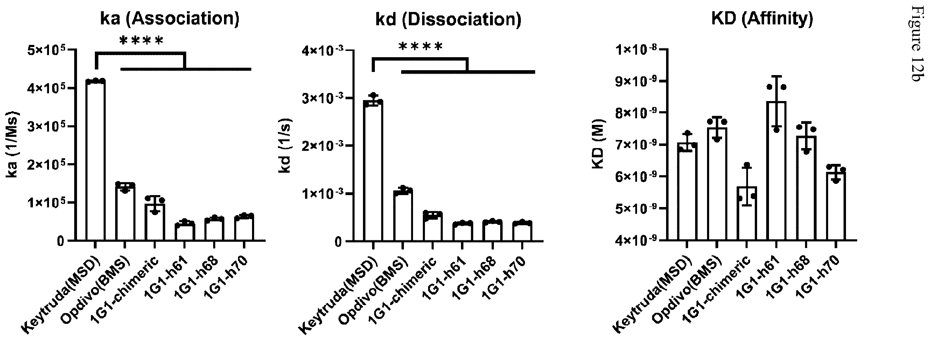

FIGs. 12a and 12b show binding kinetics and affinity values for human PD-1 described by Kon (ka value), Koff (kd value), and KD values. In conclusion, the humanized anti-PD-1 antibodies (1G1), on average, had a binding affinity for human PD-1 (KD, 7.26e-9) similar to that of Keytruda (7.06e-9) and Opdivo (7.54e-9). This indicates that the 1G1 humanized anti-PD-1 antibodies have improved binding properties of dissociating 2.7-7.6 times more slowly (Koff, 3.87e-4) than Keytruda (2.95e-3) and Opdivo (1.06e-3), while in terms of the association rate, they bind 2.6-7.7 times more slowly (Kon, 5.45e+4) than Keytruda (4.18e+5) and Opdivo (1.41e+5). Considering the mechanism of action of anti-PD-1 antibodies, the 1G1 humanized anti-PD-1 antibodies exhibit improved anticancer activity due to their binding property of slow dissociation.

## Example 14. Comparative evaluation of cross-reactivity to PD-1 antigens

To determine the cross-reactivity of the antibody to PD-1 antigens, protein-based ELISA was performed. To summarize the ELISA analysis, a 96-well plate (Thermo scientific) was coated with 10 ng of the extracellular domain (ECD) PD-1 proteins from human, mouse, rabbit, cynomolgus, and rat overnight at 4°C. After blocking and washing, 1 $\mu$g/ml of control antibodies (Keytruda and Opdivo) and 1G1 antibodies (a chimeric antibody and humanized antibodies) were added to the coated plate and incubated at 37°C for 2 hours. After washing, a secondary antibody (anti-human IgG antibody) was added and incubated at 37°C for 2 hours. After washing, TMB substrate (abcam) was added, and the color reaction was stopped with STOP solution (abcam). Absorbance at 450/650 nm was measured using a microplate reader (ThermoFisher).

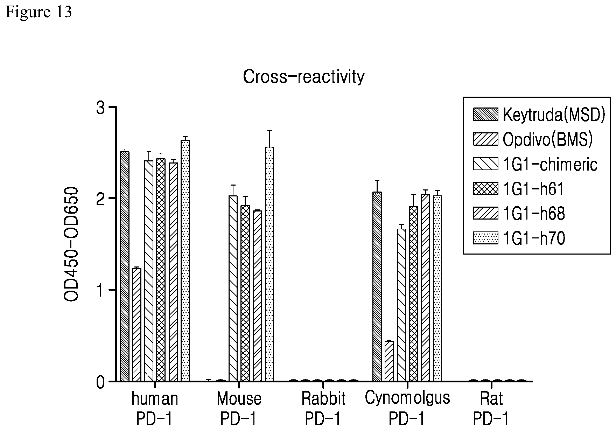

FIG. 13 shows the measurement results of the cross-reactivity of the antibodies to the PD-1 antigens. Keytruda, Opdivo, and 1G1 antibodies all have cross-reactivity to human PD-1 and cynomolgus PD-1 antigens. 1G1 antibodies additionally showed cross-reactivity to mouse PD-1 antigen.

## Example 15. *In vivo* anti-cancer efficacy evaluation

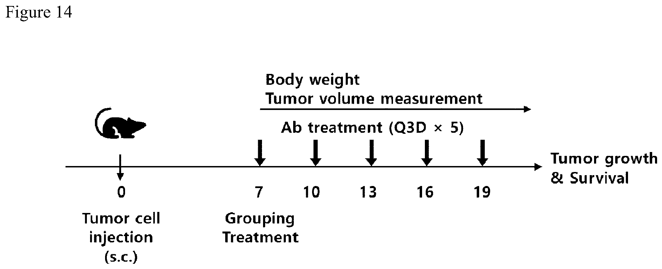

An experiment to evaluate the anticancer efficacy of the 1G1 antibody using a mouse melanoma model was performed as shown in FIG. 14. Mouse melanoma cells (B16F11/OT.EGFP) were cultured and $3\times10^6$ cells were implanted subcutaneously into the right back of 8-week-old C57BL/6 female mice. Six days after the tumor cell transplantation, the size of cancer nodules was measured, and the mice were divided into 5 groups. From the 7th day of transplantation, 1G1-parental antibody was intraperitoneally administered 5 times at 3-day intervals, and the tumor size was measured at 3-day intervals. Tumor size was calculated using the following formula:

$$L\times W^2/2$$

(L: long axis length, W: short axis length)

Survival was recorded as the day when the tumor volume exceeded 1000 mm$^3$, or tumor ulceration or mouse death occurred. Two-way ANOVA with Bonferroni test of the GraphPad Prism program was used to determine the significance of the difference between the tumor growth inhibition rates, and the Log-rank (Mantel-Cox) test was used to determine the significance of the difference between the survival rates (*, P <0.05; ***, P <0.001, survival analysis endpoint: 700 mm$^3$).

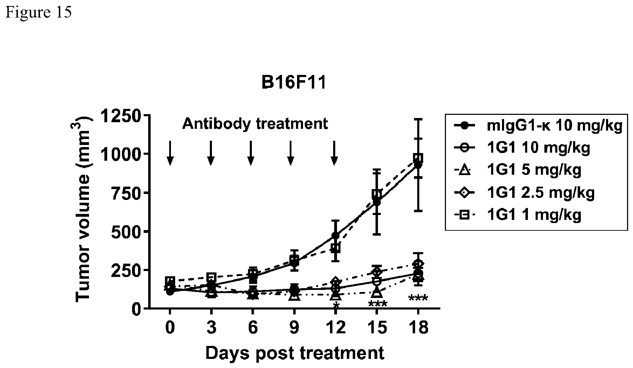

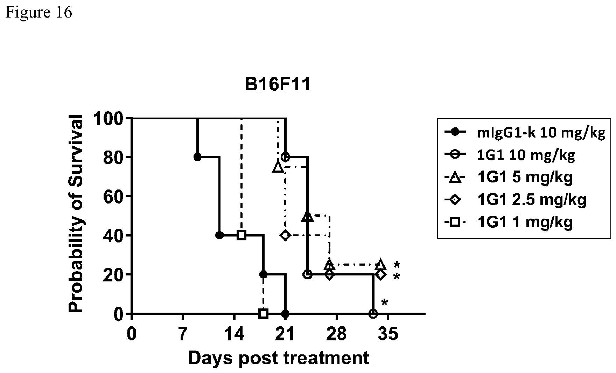

FIG. 15 shows tumor growth over time and tumor growth inhibition according to dose of the 1G1-parental antibody. The 1G1 1 mg/kg group showed tumor growth similar to that of the isotype control group; the 1G1 2.5 to 10 mg/kg groups showed tumor growth inhibition of about 90-100% at day 19, 94-112% at day 22, and 86-94% at day 25 (FIG. 15; Day 22) and an increase in survival rate of about 20% (FIG. 16). These results confirmed that the 1G1-parental antibody exhibited an excellent anticancer effect.

## Example 16. Evaluation *of in vivo* anticancer efficacy in colorectal cancer model

The anticancer efficacy of the 1G1 antibody was evaluated in the MC38 colorectal cancer syngeneic model. 5 $\times$ 10$^5$ MC38 mouse colorectal cancer cells were subcutaneously injected into the left flank of 7-8 week-old C57BL/6 female mice. After injection, when the average tumor size reached 50 to 150 mm$^3$, the mice were divided into 4 groups (n = 13 each) according to tumor volume, and human hIgG4, rat rIgG2a, 1G1-h70 antibody and RMP1-14 (mouse PD-1) antibody were administered at an interval of 3 days at 10 mg/kg each for a total of 5 times. For each group, the animal's body weight and tumor size were measured and evaluated three times a week. Final body weight and final tumor size were measured on the day the study reached the endpoint. The endpoint was defined as when the mean tumor size of the control group reached 1500 mm$^3$. Tumor growth inhibition rate (%TGI) was determined for each treatment group (T) versus the control group (C) using initial (i) and final (f) tumor measurements by the formula below:

$$\%TGI = 1 - (Tf - Ti)/(Cf - Ci)*100$$

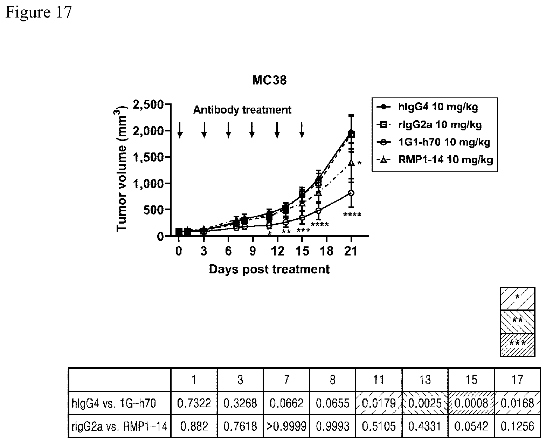

The relative change in tumor size and tumor growth inhibition rate over time for each group are shown in FIG. 17. The 1G1-h70 antibody showed significantly superior tumor growth inhibition compared to the mouse PD-1 antibody, RMP1-14. It is known that the MC38 colorectal cancer syngeneic model does not respond well to anti-PD-1 therapy. Therefore, the excellent tumor growth inhibitory effect of the 1G1-h70 antibody was unexpected. Meanwhile, there was no statistically significant change in the body weight of mice due to treatment in each group (data not shown).

### Example 17. Epitope mapping of 1G1 antibody using crystallography

Epitope mapping (X-ray crystallography method) of the humanized 1G1-h70 antibody to the human PD-1 antigen (SEQ ID NO: 62) was performed to determine the epitope in the human PD-1 antigen recognized by the antibody. The binding complex of 1G1-h70 antibody Fab and PD-1 was crystallized at 20°C using a crystallization solution by the hanging drop vapor diffusion method (drop volume = 0.8 $\mu$l of protein + 0.8 $\mu$l of reservoir, 400 $\mu$l of reservoir volume). An X-ray diffraction experiment was performed on the resulting single crystal to obtain data resolution of 2.30 Å. Table 4 below summarizes information on X-ray diffraction data collection and structure refinement.

[Table 4]
Data collection and refinement statistics

| | |
|---|---|
| Data Collection | |
| X-ray source | PLS 5C |
| Space group | $P22_12_1$ |
| Cell dimensions | |
| *a, b, c* (Å) | 70.580, 78.490,4.220 |
| Resolution (Å) | 2.30 (2.37-2.30)* |
| $R_{sym}$ (%) | 8.2 (65.5) |
| $I/\sigma I$ | 148 (3.6) |
| Completeness (%) | 99.9 (99.9) |
| Redundancy | 6.7 |
| Refinement | |
| Resolution (Å) | 2.30 |
| No. reflections | 28744 |
| $R_{work}/R_{free}$ (%) | 19.4/23.9 |
| No. atoms | |
| Protein | 4069 |
| Water | 95 |
| Average B factor (Å$^2$) | 58.2 |
| R.m.s. deviation | |
| Bond lengths (Å) | 0.008 |
| Bond angles (°) | 0.993 |
| Ramachandran | |
| Favored (%) | 98.64 |
| Allowed (%) | 1.36 |
| Outlier (%) | 0 |
| * Values in parentheses are for the outer resolution shell. | |

Analysis of the interaction between 1G1-h70 Fab and PD-1 showed that 1G1-h70 antibody formed hydrogen bonds with residues N66, Y68, K78, A129, P130, and A132 on the PD-1 antigen (SEQ ID NO: 62). Of these, Y68, K78, and N66 have side chains involved in hydrogen bonding.

Also, the interaction analysis between 1G1-h70 Fab and PD-1 showed that 1G1-h70 antibody formed hydrophobic bonds with residues I126, L128, A129, P130, and A132 on the PD-1 antigen (SEQ ID NO: 62). As also confirmed through the Ala scanning experiment of Example 11, the three amino acid residues (P130, L128, I126) found to play the most important role in binding to the 1G1 antibody are located in the FG loop region of PD-1, and it appears that the hydrophobic interactions formed by the residues exert synergy, contributing significantly to the binding affinity.

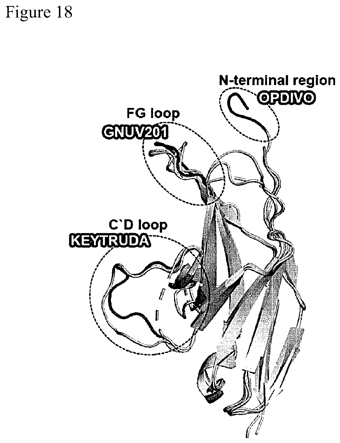

It is known that the loop regions in the PD-1 molecule are very flexible and adopt an appropriate conformation for binding depending on the binding partner. FIG. 18 shows the structural differences of the FG loop, which plays an important role in binding to the 1G1 antibody; the C'D loop, which is important for binding to Keytruda; and the N-terminal region, which is important for binding to Opdivo, in PD-1. The difference in these structures will cause a difference in the binding pattern of PD-1 interactome and in the anticancer immunity patterns of each antibody.

**Example 18. pH-dependent binding of 1G1 antibody**

It is known that there is a difference in pH between blood (pH 7.4) and the tumor microenvironment (pH 5.0 to 7.0). Therefore, the therapeutic efficacy of an immuno-oncology antibody may be affected by whether or not the antibody has a pH-dependent binding activity. In general, histidine is the most sensitive residue for pH-dependent binding activity. Keytruda and Opdivo, which are conventional PD-1 antibodies, do not have histidine among the residues involved in binding. In contrast, since the 1G1 antibody has a histidine residue such as the H52 residue of the heavy chain CDR2 in the CDR region (Kabat system) involved in hydrogen bonding or hydrophobic bonding with the PD-1 antigen, H52 is expected to contribute to the binding with PD-1 in the low pH tumor microenvironment. Accordingly, the pH-dependency of binding of 1G1-h70 to PD-1 was investigated compared to Keytruda and Opdivo.

The binding kinetics of each antibody to human PD-1 were measured through SPR (Surface Plasma Resonance) analysis using Biacore 8K (Cytiva), and the antibodies' binding affinities to the antigen were compared to one another. Anti-human PD-1 antibody controls, Keytruda (MSD (Lot #T020031)) and Opdivo (BMS (Lot #043 FB)), were products for human use purchased from Shinwon Pharmacy Co., Ltd. In addition, a 2E5 antibody was prepared according to the 2E5 clone described in WO 2018/053709 of CStone Pharmaceuticals. It was disclosed that the 2E5 antibody was able to bind to both human PD-1 and mouse PD-1, and its epitopes were located on the FG loop of PD-1.

Control antibodies (Keytruda, Opdivo), 2E5 antibody, and 1G1 antibody (1G1-h70) were flowed over a Protein A chip (Cytiva) and captured. Seven concentrations (0-100 M) of human PD-1 were flowed onto the sensor chip at a flow rate of 30 μL/min for an association phase of 120 s, followed by dissociation for 1800 s. Capture, association, and dissociation were performed in HBS-EP+ buffer at pH 6.0. The chip was regenerated with glycine at pH 1.5 after each experiment. Association and dissociation curves were drawn using Cytiva evaluation software, and kinetics and affinity values were determined. Significance of the difference between the antibodies' kinetics and affinity values was determined using one-way ANOVA with Tukey test of GraphPad Prism program (****, P <0.0001).

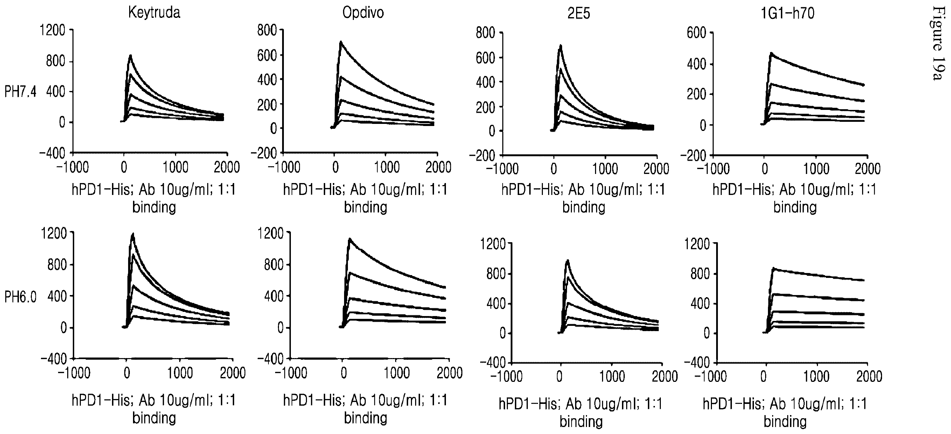

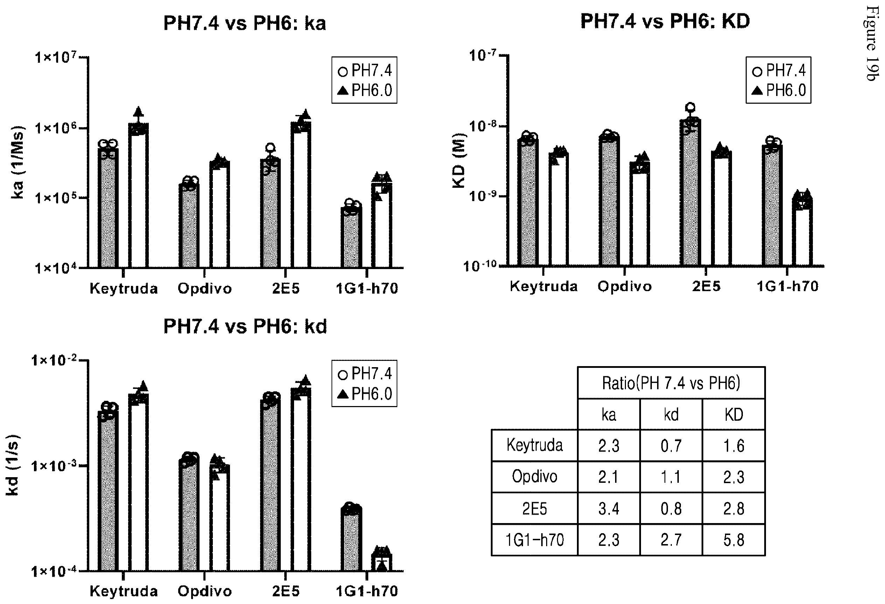

FIGs. 19a and 19b show the binding kinetics and affinity for human PD-1 described by Kon (ka value), Koff (kd value), and KD values. At pH 7.4 (blood), the 1G1-h70 antibody (KD = 5.4 nM) had a binding affinity for human PD-1 at a similar level comparable to those of Keytruda (KD = 6.6 nM), Opdivo (KD = 7.1 nM), and the 2E5 antibody (KD = 12.6 nM). However, at pH 6.0 (tumor microenvironment), 1G1-h70 antibody (KD = 0.9 nM) showed a much stronger binding affinity for human PD-1 than Keytruda (KD = 4.2 nM), Opdivo (KD = 3.1 nM), and 2E5 antibody (KD = 4.5 nM). This is because the 1G1-h70 antibody has an improved binding property in terms of dissociation (Koff), dissociating slowly compared to Keytruda, Opdivo, and 2E5 antibodies at pH 6.0. As the anticancer efficacy of anticancer drugs including immune-oncology antibodies is affected by the low pH of the tumor microenvironment, the 1G1-h70 antibody's strong binding affinity to human PD-1 even at the low pH of the tumor microenvironment provides a basis for conferring excellent anticancer activity in the tumor microenvironment *in vivo.*

**Example 19. Affinity Maturation**

Affinity maturation was performed to increase the affinity of the 1G1-h70 antibody for human PD-1. Each amino acid residue in the CDR region of the 1G1-h70 antibody was mutated into other 19 amino acids using optimal codons *for E. coli.* DNA oligonucleotide libraries were synthesized on microarrays, and clones were selected for expression in *E. coli.*

The crude protein secreted in medium was analyzed by ELISA against BSA and human PD-1 for the assessment of expression and binding affinity. Clones with improved values were selected for sequencing. The "beneficial mutants" were confirmed by affinity ranking by SPR. Off-rate screening was performed on a Biacore T200. The running buffer was HBS-EP (10 mM HEPES, 500 mM NaCl, 3 mM EDTA, 0.05% Tween 20, pH 7.4). Fab-SASA of the selected clones secreted into the culture medium was captured onto SASA capture biosensors. After equilibration, the antigen was injected for 120 seconds (association phase) followed by the injection of running buffer for 420 seconds (dissociation phase). The surface was regenerated before the injection of other selected clones. The process was repeated until all samples were analyzed. Dissociation rates of Fab-SASA clones were obtained by fitting the experimental data locally to 1:1 interaction model using the Biacore T200 evaluation software. The selected mutants were ranked by their dissociation rate constants (off-rates, kd).

Once the "beneficial mutants" were identified, a combinatorial library was constructed with random combinations of these mutations by PCR. The combinatorial clones were analyzed by ELISA and subjected to DNA sequencing and affinity ranking. The top combinations of "beneficial mutants" that led to the highest affinity increase without compromising expression were finally selected for antibody affinity measurement.

After affinity maturation, a total of 13 humanized PD-1 antibodies (AHF16556, AHF16557, AHF16558, AHF16559, AHF16560, AHF16561, AHF16563, AHF16564, AHF16565, AHF16566, AHF16568, AHF16569, andAHF16570) were obtained. These antibodies had amino acid substitutions in the three CDR regions (VH CDR2, VH CDR3, VL CDR1) compared to the 1G1-h70 parent antibody (WT) as shown in Table 5 below.

**[Table 5]**

| NO. | Sequence ID | VH | | | | | VL |
|---|---|---|---|---|---|---|---|
| | | 59 | 62 | 102 | 105 | 106 | 31. |
| | WT | T | E | A | F | A | H |
| 1 | AHF16556 | V | W | E | A | . | R |
| 2 | AHF16557 | V | W | . | A | G | . |
| 3 | AHF16558 | . | . | E | H | . | R |
| 4 | AHF16559 | V | . | . | H | . | . |
| 5 | AHF16560 | I | . | E | H | G | R |
| 6 | AHF16561 | I | . | . | A | G | R |
| 7 | AHF16563 | V | W | E | M | G | . |
| 8 | AHF16564 | . | | E | A | G | R |
| 9 | AHF16565 | V | W | E | H | G | R |
| 10 | AHF16566 | V | W | . | A | . | . |
| 11 | AHF16568 | I | W | E | M | G | R |
| 12 | AHF16569 | I | W | E | A | . | R |
| 13 | AHF16570 | I | W | . | A | G | R |

The binding kinetics data of the antibodies to human PD-1 are shown in Table 6 below.

**[Table 6]**

| NO. | Ligand | ka (1/Ms) | kd (1/s) | KD (M) | Ratio(kd) | Ratio(kD) |
|---|---|---|---|---|---|---|
| 1 | AHF16556 | 1.48E+05 | 6.31E-05 | 4.27E-10 | 6.45 | 5.27 |
| 2 | AHF16557 | 2.04E+05 | 8.68E-05 | 4.25E-10 | 4.69 | 5.29 |
| 3 | AHF16558 | 2.48E+05 | 7.69E-05 | 3.10E-10 | 5.29 | 7.26 |
| 4 | AHF16559 | 2.06E+05 | 7.62E-05 | 3.71E-10 | 5.34 | 6.06 |
| 5 | AHF16560 | 2.04E+05 | 6.74E-05 | 3.31E-10 | 6.04 | 6.80 |
| 6 | AHF16561 | 1.83E+05 | 5.53E-05 | 3.02E-10 | 7.36 | 7.45 |
| 7 | AHF16563 | 2.18E+05 | 3.60E-05 | 1.65E-10 | 11.31 | 13.64 |
| 8 | AHF16564 | 2.30E+05 | 3.68E-05 | 1.60E-10 | 11.06 | 14.06 |
| 9 | AHF16565 | 1.30E+05 | 1.18E-04 | 9.13E-10 | 3.45 | 2.46 |
| 10 | AHF16566 | 2.15E+05 | 7.53E-05 | 3.49E-10 | 5.41 | 6.45 |
| 11 | AHF16568 | 1.46E+05 | 3.52E-05 | 2.41E-10 | 11.56 | 9.34 |
| 12 | AHF16569 | 1.99E+05 | 6.74E-05 | 3.39E-10 | 6.04 | 6.64 |
| 13 | AHF16570 | 1.64E+05 | 3.29E-05 | 2.00E-10 | 12.37 | 11.25 |
| | U299-WT1 | 1.74E+05 | 4.02E-04 | 2.32E-09 | | |
| | U299-WT2 | 1.90E+05 | 4.12E-04 | 2.17E-09 | | |

Sequence Listing

[Table 7]

| Seq ID No. | | Sequences |
|---|---|---|
| 1 | HCDR1 amino acids | GYWMH |
| 2 | HCDR1 nucleotides | GGCTACTGGATGCAC |
| 3 | HCDR2 amino acids | MIHPNSDTTTYNEKFKN |
| 4 | HCDR2 nucleotides | ATGATTCATCCTAACAGTGATACTACTACCTACAAT GAGAAGTTCAAAAAC |
| 5 | HCDR3 amino acids | TDQAAWFAF |
| 6 | HCDR3 nucleotides | ACAGATCAGGCCGCCTGGTTTGCTTTC |
| 7 | LCDR1 amino acids | RSSQNIVHSNGDTYLE |
| 8 | LCDR1 nucleotides | agatctagtcagaacattgtacatagtaatggagacacctatttagaa |
| 9 | LCDR2 amino acids | KVSKRFS |
| 10 | LCDR2 nucleotides | aaagtttccaagcgattttct |
| 11 | LCDR3 amino acids | FQGSHVPWT |
| 12 | LCDR3 nucleotides | tttcaaggttcacatgttccgtggacg |
| 13 | HCVR amino acids | EVQLQQSGAELVKPGASVKLSCKASGYTFTGYWMH WVKQRPGQGLEWIGMIHPNSDTTTYNEKFKNRATL TVDKSSGTAYMQLSSLTSEDSAVYYCTGTDQAAWFA FWGQGTLVTVSA |
| 14 | HCVR nucleotides | GAGGTCCAGCTGCAGCAGTCTGGGGCTGAGCTGG TTAAGCCTGGGGCTTCAGTGAAGCTGTCCTGCAAG GCTTCTGGCTACACTTTCACCGGCTACTGGATGCA CTGGGTGAAGCAGAGGCCTGGACAAGGCCTTGAG TGGATTGGAATGATTCATCCTAACAGTGATACTACT ACCTACAATGAGAAGTTCAAAAACAGGGCCACAC TGACTGTAGACAAATCCTCCGGCACAGCCTACATG CAACTCAGCAGCCTGACATCTGAGGACTCTGCGGT CTATTACTGTACAGGGACAGATCAGGCCGCCTGGT TTGCTTTC TGGGGCCAAGGGACTCTGGTCACTGTCTCTGCA |
| 15 | LCVR amino acids | DIVLTQTPLSLPVSLGDQASISCRSSQNIVHSNGDTYL EWYLQKPGQSPKLLIYKVSKRFSGVPDRFSGSGSGT DFTLKISRVEAEDLGVYYCFQGSHVPWTFGGGTKLE IK |

(continued)

| Seq ID No. | | Sequences |
|---|---|---|
| 16 | LCVR nucleotides | GATATTGTGCTGACACAAACTCCActctccctgcctgtcagtc ttggagatcaagcctccatctcttgcagatctagtcagaacattgtacatagtaatgg agacacctatttagaatggtacctgcagaaaccaggccagtctccaaagctcctga tctacaaagtttccaagcgattttctggggtcccagacaggttcagtggcagtggat cagggacagatttcacactcaagatcagcagagtggaggctgaggatctgggagt ttattactgctttcaaggttcacatgttccgtggacgttcggtggaggcaccaagctg gaaatcaaa |
| 17 | HFR1 amino acids | EVQLQQSGAELVKPGASVKLSCKASGYTFT |
| 18 | HFR1 nucleotides | GAGGTCCAGCTGCAGCAGTCTGGGGCTGAGCTGG TTAAGCCTGGGGCTTCAGTGAAGCTGTCCTGCAAG GCTTCTGGCTACACTTTCACC |
| 19 | HFR2 amino acids | WVKQRPGQGLEWIG |
| 20 | HFR2 nucleotides | TGGGTGAAGCAGAGGCCTGGACAAGGCCTTGAGT GGATTGGA |
| 21 | HFR3 amino acids | RATLTVDKSSGTAYMQLSSLTSEDSAVYYCTG |
| 22 | HFR3 nucleotides | AGGGCCACACTGACTGTAGACAAATCCTCCGGCA CAGCCTACATGCAACTCAGCAGCCTGACATCTGAG GACTCTGCGGTCTATTACTGTACAGGG |
| 23 | HFR4 amino acids | WGQGTLVTVSA |
| 24 | HFR4 nucleotides | TGGGGCCAAGGGACTCTGGTCACTGTCTCTGCA |
| 25 | LFR1 amino acids | DIVLTQTPLSLPVSLGDQASISC |
| 26 | LFR1 nucleotides | GATATTGTGCTGACACAAACTCCActctccctgcctgtcagtc ttggagatcaagcctccatctcttgc |
| 27 | LFR2 amino acids | WYLQKPGQSPKLLIY |
| 28 | LFR2 nucleotides | tggtacctgcagaaaccaggccagtctccaaagctcctgatctac |
| 29 | LFR3 amino acids | GVPDRFSGSGSGTDFTLKISRVEAEDLGVYYC |
| 30 | LFR3 nucleotides | ggggtcccagacaggttcagtggcagtggatcagggacagatttcacactcaaga tcagcagagtggaggctgaggatctgggagtttattactgc |
| 31 | LFR4 amino acids | FGGGTKLEIK |
| 32 | LFR4 nucleotides | ttcggtggaggcaccaagctggaaatcaaa |
| 33 | MH1 primer | AATTTGCTAGCSARGTNMAGCTGSAGSAGTC |
| 34 | MH2 primer | AATTTGCTAGCSARGTNMAGCTGSAGSAGTCWGG |
| 35 | IgG1 primer | AATTTGGATCCATAGACAGATGGGGGTGTCGTTTT GGC |
| 36 | MK primer | AATTGGATCCAGGGGCCAGTGGATAGACTGATGG |
| 37 | CK primer | AATTTGCGGCCGCGGATACAGTTGGTGCAGCATC |

(continued)

| Seq ID No. | | Sequences |
|---|---|---|
| 38 | Human IgG4(S228P) constant region, heavy chain nucleotides | GCTAGCACCAAGGGCCCATCGGTCTTCCCCCTGGC GCCCTGCTCCAGGAGCACCTCCGAGAGCACAGCC GCCCTGGGCTGCCTGGTCAAGGACTACTTCCCCGA ACCCGTGACGGTGTCGTGGAACTCAGGCGCCCTG ACCAGCGGCGTGCACACCTTCCCGGCTGTCCTACA GTCCTCAGGACTCTACTCCCTCAGCAGCGTGGTGA CCGTGCCCTCCAGCAGCTTGGGCACGAAGACCTA CACCTGCAACGTAGATCACAAGCCCAGCAACACC AAGGTGGACAAGAGAGTTGAGTCCAAATATGGTC CCCCATGCCCACCATGCCCAGCACCTGAGTTCCTG GGGGGACCATCAGTCTTCCTGTTCCCCCCAAAACC CAAGGACACTCTCATGATCTCCCGGACCCCTGAGG TCACGTGCGTGGTGGTGGACGTGAGCCAGGAAGA CCCCGAGGTCCAGTTCAACTGGTACGTGGATGGCG TGGAGGTGCATAATGCCAAGACAAAGCCGCGGGA GGAGCAGTTCAACAGCACGTACCGTGTGGTCAGC GTCCTCACCGTCCTGCACCAGGACTGGCTGAACG GCAAGGAGTACAAGTGCAAGGTCTCCAACAAAGG CCTCCCGTCCTCCATCGAGAAAACCATCTCCAAAG CCAAAGGGCAGCCCCGAGAGCCACAGGTGTACAC CCTGCCCCCATCCCAGGAGGAGATGACCAAGAAC CAGGTCAGCCTGACCTGCCTGGTCAAAGGCTTCTA CCCCAGCGACATCGCCGTGGAGTGGGAGAGCAAT GGGCAGCCGGAGAACAACTACAAGACCACGCCTC CCGTGCTGGACTCCGACGGCTCCTTCTTCCTCTAC AGCAGGCTAACCGTGGACAAGAGCAGGTGGCAGG AGGGGAATGTCTTCTCATGCTCCGTGATGCATGAG GCTCTGCACAACCACTACACACAGAAGAGCCTCT CCCTGTCTCCGGGTAAA |

(continued)

| Seq ID No. | | Sequences |
|---|---|---|
| 39 | Human IgG4(S228P) constant region, heavy chain amino acids | ASTKGPSVFPLAPCSRSTSESTAALGCLVKDYFPEPV TVSWNSGALTSGVHTFPAVLQSSGLYSLSSVVTVPSS SLGTKTYTCNVDHKPSNTKVDKRVESKYGPPCPPCP APEFLGGPSVFLFPPKPKDTLMISRTPEVTCVVVDVS QEDPEVQFNWYVDGVEVHNAKTKPREEQFNSTYRV VSVLTVLHQDWLNGKEYKCKVSNKGLPSSIEKTISK AKGQPREPQVYTLPPSQEEMTKNQVSLTCLVKGFYP SDIAVEWESNGQPENNYKTTPPVLDSDGSFFLYSRLT VDKSRWQEGNVFSCSVMHEALHNHYTQKSLSLSPG K |
| 40 | Human IgG4(S228P) constant region, kappa chain nucleotides | CGTACGGTGGCTGCACCATCTGTCTTCATCTTCCCG CCATCTGATGAGCAGTTGAAATCTGGAACTGCCTC TGTTGTGTGCCTGCTGAATAACTTCTATCCCAGAG AGGCCAAAGTACAGTGGAAGGTGGATAACGCCCT CCAATCGGGTAACTCCCAGGAGAGTGTCACAGAG CAGGACAGCAAGGACAGCACCTACAGCCTCAGCA GCACCCTGACGCTGAGCAAAGCAGACTACGAGAA ACACAAAGTCTACGCCTGCGAAGTCACCCATCAG GGCCTGAGCTCGCCCGTCACAAAGAGCTTCAACA GGGGAGAGTGT |
| 41 | Human IgG4(S228P) constant region, kappa chain amino acids | RTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC |

(continued)

| Seq ID No. | | Sequences |
|---|---|---|
| 42 | Humanized antibody, 1G1-h61 heavy chain nucleotides | CAAGTTCAGCTGGTGCAGAGCGGCGCCGAGGTGA AGAAGCCTGGAGCCAGCGTGAAAGTGTCCTGCAA GGCCTCTGGCTACACCTTTACAGGCTACTGGATGC ACTGGGTGCGGCAGGCCCCTGGACAGGGCCTGGA ATGGATGGGCATGATCCACCCCAACAGCGACACCA CAACCTACAACGAGAAGTTCAAGAATAGAGTGAC CATGACAAGAGATACCAGCATCAGCACCGCCTACA TGGAACTGAGCAGACTGCGGTCCGATGACACAGC TGTGTACTATTGTGCCGGCACCGACCAGGCCGCTT GGTTCGCCTTCTGGGGGCAGGGCACCACAGTCAC CGTGAGCTCTGCCAGCACCAAGGGCCCTTCCGTGT TTCCCCTGGCCCCTTGCTCCCGGTCCACATCTGAG AGCACCGCCGCCCTGGGCTGTCTGGTGAAGGACT ACTTCCCAGAGCCCGTGACCGTGAGCTGGAACAG CGGCGCCCTGACAAGCGGCGTGCACACATTTCCC GCCGTGCTGCAGAGCTCCGGCCTGTACTCCCTGTC TAGCGTGGTGACAGTGCCTTCCTCTAGCCTGGGCA CCAAGACATATCCTGTAACGTGGACCACAAGCCA |

(continued)

| Seq ID No. | | Sequences |
|---|---|---|
| | | AGCAATACCAAGGTGGATAAGCGGGTGGAGTCTA AGTACGGCCCTCCTTGCCCTAGCTGTCCTGCTCCA GAGTTTCTGGGCGGCCCTTCCGTGTTCCTGTTTCC ACCCAAACCAAAGGACACACTGATGATCTCTAGA ACACCAGAGGTGACCTGCGTGGTGGTGGACGTGA GCCAGGAGGATCCCGAGGTGCAGTTCAACTGGTA CGTGGATGGCGTGGAGGTGCACAATGCCAAGACC AAGCCAAGAGAGGAGCAGTTTAACTCTACATACA GGGTGGTGAGCGTGCTGACCGTGCTGCACCAGGA TTGGCTCAACGGCAAGGAGTATAAGTGCAAGGTG TCCAATAAGGGCCTGCCCTCCTCTATCGAGAAGAC AATCTCTAAGGCTAAGGGCCAGCCAAGAGAGCCT CAGGTGTACACCCTGCCTCCAAGCCAGGAGGAGA TGACAAAGAACCAGGTGTCCCTGACATGTCTGGTG AAGGGCTTCTATCCCTCCGACATCGCCGTGGAGTG GGAGTCTAATGGCCAGCCTGAGAACAATTACAAG ACCACACCCCCTGTGCTGGACTCTGATGGCAGCTT CTTTCTGTATTCCAGGCTGACCGTGGATAAGTCTCG GTGGCAGGAGGGCAACGTGTTCAGCTGCTCTGTG ATGCACGAAGCCCTGCATAATCACTATACTCAGAA AAGTCTGTCACTGTCACTGGGAAAGTGATAA |
| 43 | Humanized antibody, 1G1-h61 heavy chain amino acids | QVQLVQSGAEVKKPGASVKVSCKASGYTFTGYWM HWVRQAPGQGLEWMGMIHPNSDTTTYNEKFKNRV TMTRDTSISTAYMELSRLRSDDTAVYYCAGTDQAAW FAFWGQGTTVTVSSASTKGPSVFPLAPCSRSTSESTA ALGCLVKDYFPEPVTVSWNSGALTSGVHTFPAVLQS SGLYSLSSVVTVPSSSLGTKTYTCNVDHKPSNTKVD KRVESKYGPPCPSCPAPEFLGGPSVFLFPPKPKDTLMI SRTPEVTCVVVDVSQEDPEVQFNWYVDGVEVHNAK TKPREEQFNSTYRVVSVLTVLHQDWLNGKEYKCKV SNKGLPSSIEKTISKAKGQPREPQVYTLPPSQEEMTK NQVSLTCLVKGFYPSDIAVEWESNGQPENNYKTTPP VLDSDGSFFLYSRLTVDKSRWQEGNVFSCSVMHEAL HNHYTQKSLSLSLGK |

(continued)

| Seq ID No. | | Sequences |
|---|---|---|
| 44 | Humanized antibody, 1G1-h68 heavy chain | CAAGTTCAGCTGGTGCAGAGCGGCGCCGAGGTGA AGAAGCCTGGAGCCAGCGTGAAAGTGTCCTGCAA GGCCTCTGGCTACACCTTTACAGGCTACTGGATGC |

(continued)

| Seq ID No. | | Sequences |
|---|---|---|
| | nucleotides | ACTGGGTGCGGCAGGCCCCTGGACAGGGCCTGGA ATGGATCGGCATGATCCACCCCAACAGCGACACCA CAACCTACAACGAGAAGTTCAAGAATAGAGTGAC CATGACAAGAGATACCAGCATCAGCACCGCCTACA TGGAACTGAGCAGACTGCGGTCCGATGACACAGC TGTGTACTATTGTACCGGCACCGACCAGGCCGCTT GGTTCGCCTTCTGGGGGCAGGGCACCACAGTCAC CGTGAGCTCTGCCAGCACCAAGGGCCCTTCCGTGT TTCCCCTGGCCCCTTGCTCCCGGTCCACATCTGAG AGCACCGCCGCCCTGGGCTGTCTGGTGAAGGACT ACTTCCCAGAGCCCGTGACCGTGAGCTGGAACAG CGGCGCCCTGACAAGCGGCGTGCACACATTTCCC GCCGTGCTGCAGAGCTCCGGCCTGTACTCCCTGTC TAGCGTGGTGACAGTGCCTTCCTCTAGCCTGGGCA CCAAGACATATACCTGTAACGTGGACCACAAGCCA AGCAATACCAAGGTGGATAAGCGGGTGGAGTCTA AGTACGGCCCTCCTTGCCCTAGCTGTCCTGCTCCA GAGTTTCTGGGCGGCCCTTCCGTGTTCCTGTTTCC ACCCAAACCAAAGGACACACTGATGATCTCTAGA ACACCAGAGGTGACCTGCGTGGTGGTGGACGTGA GCCAGGAGGATCCCGAGGTGCAGTTCAACTGGTA CGTGGATGGCGTGGAGGTGCACAATGCCAAGACC AAGCCAAGAGAGGAGCAGTTTAACTCTACATACA GGGTGGTGAGCGTGCTGACCGTGCTGCACCAGGA TTGGCTCAACGGCAAGGAGTATAAGTGCAAGGTG TCCAATAAGGGCCTGCCCTCCTCTATCGAGAAGAC AATCTCTAAGGCTAAGGGCCAGCCAAGAGAGCCT CAGGTGTACACCCTGCCTCCAAGCCAGGAGGAGA TGACAAAGAACCAGGTGTCCCTGACATGTCTGGTG AAGGGCTTCTATCCCTCCGACATCGCCGTGGAGTG GGAGTCTAATGGCCAGCCTGAGAACAATTACAAG ACCACACCCCTGTGCTGGACTCTGATGGCAGCTT CTTTCTGTATTCCAGGCTGACCGTGGATAAGTCTCG GTGGCAGGAGGGCAACGTGTTCAGCTGCTCTGTG ATGCACGAAGCCCTGCATAATCACTATACTCAGAA AAGTCTGTCACTGTCACTGGGAAAGTGATAA |

(continued)

| Seq ID No. | | Sequences |
|---|---|---|
| 45 | Humanized antibody, 1G1-h68 heavy chain amino acids | QVQLVQSGAEVKKPGASVKVSCKASGYTFTGYWM HWVRQAPGQGLEWIGMIHPNSDTTTYNEKFKNRVT MTRDTSISTAYMELSRLRSDDTAVYYCTGTDQAAWF AFWGQGTTVTVSSASTKGPSVFPLAPCSRSTSESTAA LGCLVKDYFPEPVTVSWNSGALTSGVHTFPAVLQSS GLYSLSSVVTVPSSSLGTKTYTCNVDHKPSNTKVDK RVESKYGPPCPSCPAPEFLGGPSVFLFPPKPKDTLMIS RTPEVTCVVVDVSQEDPEVQFNWYVDGVEVHNAK TKPREEQFNSTYRVVSVLTVLHQDWLNGKEYKCKV SNKGLPSSIEKTISKAKGQPREPQVYTLPPSQEEMTK NQVSLTCLVKGFYPSDIAVEWESNGQPENNYKTTPP VLDSDGSFFLYSRLTVDKSRWQEGNVFSCSVMHEAL HNHYTQKSLSLSLGK |

(continued)

| Seq ID No. | | Sequences |
|---|---|---|
| 46 | Humanized antibody, 1G1-h70 heavy chain nucleotides | CAAGTTCAGCTGGTGCAGAGCGGCGCCGAGGTGA AGAAGCCTGGAGCCAGCGTGAAAGTGTCCTGCAA GGCCTCTGGCTACACCTTTACAGGCTACTGGATGC ACTGGGTGCGGCAGGCCCCTGGACAGGGCCTGGA ATGGATCGGCATGATCCACCCCAACAGCGACACCA CAACCTACAACGAGAAGTTCAAGAATAGAGTGAC CATGACAAGAGATACCAGCATCAGCACCGCCTACA TGGAACTGAGCAGACTGCGGTCCGATGACACAGC TGTGTACTATTGTGCCGGCACCGACCAGGCCGCTT GGTTCGCCTTCTGGGGGCAGGGCACCACAGTCAC CGTGAGCTCTGCCAGCACCAAGGGCCCTTCCGTGT TTCCCCTGGCCCCTTGCTCCCGGTCCACATCTGAG AGCACCGCCGCCCTGGGCTGTCTGGTGAAGGACT ACTTCCCAGAGCCCGTGACCGTGAGCTGGAACAG CGGCGCCCTGACAAGCGGCGTGCACACATTTCCC GCCGTGCTGCAGAGCTCCGGCCTGTACTCCCTGTC TAGCGTGGTGACAGTGCCTTCCTCTAGCCTGGGCA CCAAGACATATACCTGTAACGTGGACCACAAGCCA AGCAATACCAAGGTGGATAAGCGGGTGGAGTCTA AGTACGGCCCTCCTTGCCCTAGCTGTCCTGCTCCA GAGTTTCTGGGCGGCCCTTCCGTGTTCCTGTTTCC ACCCAAACCAAAGGACACACTGATGATCTCTAGA ACACCAGAGGTGACCTGCGTGGTGGTGGACGTGA GCCAGGAGGATCCCGAGGTGCAGTTCAACTGGTA CGTGGATGGCGTGGAGGTGCACAATGCCAAGACC |

(continued)

| Seq ID No. | | Sequences |
|---|---|---|
| | | AAGCCAAGAGAGGAGCAGTTTAACTCTACATACA GGGTGGTGAGCGTGCTGACCGTGCTGCACCAGGA TTGGCTCAACGGCAAGGAGTATAAGTGCAAGGTG TCCAATAAGGGCCTGCCCTCCTCTATCGAGAAGAC AATCTCTAAGGCTAAGGGCCAGCCAAGAGAGCCT CAGGTGTACACCCTGCCTCCAAGCCAGGAGGAGA TGACAAAGAACCAGGTGTCCCTGACATGTCTGGTG AAGGGCTTCTATCCCTCCGACATCGCCGTGGAGTG GGAGTCTAATGGCCAGCCTGAGAACAATTACAAG ACCACACCCCCTGTGCTGGACTCTGATGGCAGCTT CTTTCTGTATTCCAGGCTGACCGTGGATAAGTCTCG GTGGCAGGAGGGCAACGTGTTCAGCTGCTCTGTG ATGCACGAAGCCCTGCATAATCACTATACTCAGAA AAGTCTGTCACTGTCACTGGGAAAGTGATAA |
| 47 | Humanized antibody, 1G1-h70 heavy chain amino acids | QVQLVQSGAEVKKPGASVKVSCKASGYTFTGYWM HWVRQAPGQGLEWIGMIHPNSDTTTYNEKFKNRVT MTRDTSISTAYMELSRLRSDDTAVYYCAGTDQAAWF AFWGQGTTVTVSSASTKGPSVFPLAPCSRSTSESTAA LGCLVKDYFPEPVTVSWNSGALTSGVHTFPAVLQSS GLYSLSSVVTVPSSSLGTKTYTCNVDHKPSNTKVDK RVESKYGPPCPSCPAPEFLGGPSVFLFPPKPKDTLMIS RTPEVTCVVVDVSQEDPEVQFNWYVDGVEVHNAK TKPREEQFNSTYRVVSVLTVLHQDWLNGKEYKCKV SNKGLPSSIEKTISKAKGQPREPQVYTLPPSQEEMTK NQVSLTCLVKGFYPSDIAVEWESNGQPENNYKTTPP VLDSDGSFFLYSRLTVDKSRWQEGNVFSCSVMHEAL HNHYTQKSLSLSLGK |

(continued)

| Seq ID No. | | Sequences |
|---|---|---|
| 48 | Humanized antibody, 1G1-h61 light chain nucleotides | GATATCGTTATGACCCAGACCCCTCTGAGCCTGTCC GTGACCCCAGGCCAACCTGCCTCTATCAGCTGTAG AAGCAGCCAGAACATCGTGCACAGCAACGGCGAC ACCTACCTGGAATGGTATCTGCAGAAACCTGGACA GAGCCCCAAGCTGCTGATCTACAAGGTGTCCAAGC GGTTTTCCGGCGTGCCTGATAGATTCAGCGGATCT GGCAGCGGCACAGACTTCACCCTGAAGATTTCTAG AGTGGAGGCCGAGGACGTGGGCGTCTACTACTGC TTCCAGGGCAGCCACGTGCCCTGGACATTCGGCG GCGGAACAAAGGTGGAAATCAAGAGGACAGTGGC CGCCCCAAGCGTGTTCATCTTTCCCCCTTCCGACG AGCAGCTGAAGTCTGGCACCGCCAGCGTGGTGTG CCTGCTGAACAACTTCTACCCTCGGGAGGCCAAG GTCCAGTGGAAGGTGGATAACGCCCTGCAGTCTG GCAATAGCCAGGAGTCCGTGACCGAGCAGGACTC TAAGGATAGCACATATTCCCTGTCTAGCACCCTGAC ACTGAGCAAGGCCGATTACGAGAAGCACAAGGTG TATGCCTGTGAAGTCACCCATCAGGGGCTGTCATC ACCCGTCACTAAGTCATTCAATCGCGGAGAATGCT GATAA |
| 49 | Humanized antibody, 1G1-h61 light chain amino acids | DIVMTQTPLSLSVTPGQPASISCRSSQNIVHSNGDTYL EWYLQKPGQSPKLLIYKVSKRFSGVPDRFSGSGSGT DFTLKISRVEAEDVGVYYCFQGSHVPWTFGGGTKV EIKRTVAAPSVFIFPPSDEQLKSGTASVVCLLNNFYPR EAKVQWKVDNALQSGNSQESVTEQDSKDSTYSLSS TLTLSKADYEKHKVYACEVTHQGLSSPVTKSFNRGE C |

(continued)

| Seq ID No. | | Sequences |
|---|---|---|
| 50 | Humanized antibody, 1G1-h68 light chain nucleotides | GATATCGTTATGACCCAGACCCCTCTGAGCCTGTCC GTGACCCCAGGCCAACCTGCCTCTATCAGCTGTAG AAGCAGCCAGAACATCGTGCACAGCAACGGCGAC ACCTACCTGGAATGGTATCTGCAGAAACCTGGACA GAGCCCCCAGCTGCTGATCTACAAGGTGTCCAAGC GGTTTTCCGGCGTGCCTGATAGATTCAGCGGATCT GGCAGCGGCACAGACTTCACCCTGAAGATTCTAG AGTGGAGGCCGAGGACGTGGGCGTCTACTACTGC TTCCAGGGCAGCCACGTGCCCTGGACATTCGGCG GCGGAACAAAGGTGGAAATCAAGAGGACAGTGGC CGCCCCAAGCGTGTTCATCTTTCCCCCTTCCGACG AGCAGCTGAAGTCTGGCACCGCCAGCGTGGTGTG CCTGCTGAACAACTTCTACCCTCGGGAGGCCAAG GTCCAGTGGAAGGTGGATAACGCCCTGCAGTCTG GCAATAGCCAGGAGTCCGTGACCGAGCAGGACTC TAAGGATAGCACATATTCCCTGTCTAGCACCCTGAC ACTGAGCAAGGCCGATTACGAGAAGCACAAGGTG TATGCCTGTGAAGTCACCCATCAGGGGCTGTCATC ACCCGTCACTAAGTCATTCAATCGCGGAGAATGCT GATAA |
| 51 | Humanized antibody, 1G1-h68 light chain amino acids | DIVMTQTPLSLSVTPGQPASISCRSSQNIVHSNGDTYL EWYLQKPGQSPQLLIYKVSKRFSGVPDRFSGSGSGT DFTLKISRVEAEDVGVYYCFQGSHVPWTFGGGTKV EIKRTVAAPSVFIFPPSDEQLKSGTASVVCLLNNFYPR EAKVQWKVDNALQSGNSQESVTEQDSKDSTYSLSS TLTLSKADYEKHKVYACEVTHQGLSSPVTKSFNRGE C |

(continued)

| Seq ID No. | | Sequences |
|---|---|---|
| 52 | Humanized antibody, 1G1-h70 light chain nucleotides | GATATCGTTATGACCCAGACCCCTCTGAGCCTGTCCGTGACCCCAGGCCAACCTGCCTCTATCAGCTGTAGAAGCAGCCAGAACATCGTGCACAGCCAGGGCGACACCTACCTGGAATGGTATCTGCAGAAACCTGGACAGAGCCCCCAGCTGCTGATCTACAAGGTGTCCAAGCGGTTTTCCGGCGTGCCTGATAGATTCAGCGGATCTGGCAGCGGCACAGACTTCACCCTGAAGATTTCTAGAGTGGAGGCCGAGGACGTGGGCGTCTACTACTGCTTCCAGGGCAGCCACGTGCCCTGGACATTCGGCGGCGGAACAAAGGTGGAAATCAAGAGGACAGTGGCCGCCCCAAGCGTGTTCATCTTTCCCCCTTCCGACGAGCAGCTGAAGTCTGGCACCGCCAGCGTGGTGTGCCTGCTGAACAACTTCTACCCTCGGGAGGCCAAGGTCCAGTGGAAGGTGGATAACGCCCTGCAGTCTGGCAATAGCCAGGAGTCCGTGACCGAGCAGGACTCTAAGGATAGCACATATTCCCTGTCTAGCACCCTGACACTGAGCAAGGCCGATTACGAGAAGCACAAGGTGTATGCCTGTGAAGTCACCCATCAGGGGCTGTCATCACCCGTCACTAAGTCATTCAATCGCGGAGAATGCTGATAA |
| 53 | Humanized antibody, 1G1-h70 light chain amino acids | DIVMTQTPLSLSVTPGQPASISCRSSQNIVHSQGDTYLEWYLQKPGQSPQLLIYKVSKRFSGVPDRFSGSGSGTDFTLKISRVEAEDVGVYYCFQGSHVPWTFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 54 | Humanized antibody, 1G1-h61 VH amino acids | QVQLVQSGAEVKKPGASVKVSCKASGYTFTGYWMHWVRQAPGQGLEWMGMIHPNSDTTTYNEKFKNRVTMTRDTSISTAYMELSRLRSDDTAVYYCAGTDQAAWFAFWGQGTTVTVSS |
| 55 | Humanized antibody, 1G1-h61 VL amino acids | DIVMTQTPLSLSVTPGQPASISCRSSQNIVHSNGDTYLEWYLQKPGQSPKLLIYKVSKRFSGVPDRFSGSGSGTDFTLKISRVEAEDVGVYYCFQGSHVPWTFGGGTKVEIK |

(continued)

| Seq ID No. | | Sequences |
|---|---|---|
| 56 | Humanized antibody, 1G1-h68 VH amino acids | QVQLVQSGAEVKKPGASVKVSCKASGYTFTGYWM HWVRQAPGQGLEWIGMIHPNSDTTTYNEKFKNRVT MTRDTSISTAYMELSRLRSDDTAVYYCTGTDQAAWF AFWGQGTTVTVSS |
| 57 | Humanized antibody, 1G1-h68 VL amino acids | DIVMTQTPLSLSVTPGQPASISCRSSQNIVHSNGDTYL EWYLQKPGQSPQLLIYKVSKRFSGVPDRFSGSGSGT DFTLKISRVEAEDVGVYYCFQGSHVPWTFGGGTKV EIK |
| 58 | Humanized antibody, 1G1-h70 VH amino acids | QVQLVQSGAEVKKPGASVKVSCKASGYTFTGYWM HWVRQAPGQGLEWIGMIHPNSDTTTYNEKFKNRVT MTRDTSISTAYMELSRLRSDDTAVYYCAGTDQAAWF AFWGQGTTVTVSS |
| 59 | Humanized antibody, 1G1-h70 VL amino acids | DIVMTQTPLSLSVTPGQPASISCRSSQNIVHSQGDTYL EWYLQKPGQSPQLLIYKVSKRFSGVPDRFSGSGSGT DFTLKISRVEAEDVGVYYCFQGSHVPWTFGGGTKV EIK |
| 60 | LCDR1 amino acids | RSSQNIVHSQGDTYLE |
| 61 | LCDR1 nucleotides | agaagcagccagaacatcgtgcacagccagggcgacacctacctggaa |
| 62 | Human PD-1 protein, full amino acids | *MQIPQAPWPVVWAVLQLGWRPGW*<u>FLDSPDRPWNPPT FSPALLVVTEGDNATFTCSFSNTSESFVLNWYRMSPS NQTDKLAAFPEDRSQPGQDCRFRVTQLPNGRDFHM SVVRARRNDSGTYLCGAISLAPKAQIKESLRAELRVT ERRAEVPTAHPSPSPRPAGQFQTLVVGVVGGLLGSLV LLVWVLAVICSRAARGTIGARRTGQPLKEDPSAVPVF SVDYGELDFQWREKTPEPPVPCVPEQTEYATIVFPSG MGTSSPARRGSADGPRSAQPLRPEDGHCSWPL</u> The signal sequence is indicated in italics. The ECD (extracellular domain) is underlined. |
| 63 | HCDR2 amino acids | MIHPNSDTTVYNEKFKN |
| 64 | HCDR2 amino acids | MIHPNSDTTIYNEKFKN |
| 65 | HCDR2 amino acids | MIHPNSDTTTYNWKFKN |
| 66 | HCDR2 amino acids | MIHPNSDTTVYNWKFKN |
| 67 | HCDR2 amino acids | MIHPNSDTTIYNWKFKN |
| 68 | HCDR3 amino acids | TDQEAWFAF |
| 69 | HCDR3 amino acids | TDQAAWAAF |
| 70 | HCDR3 amino acids | TDQAAWMAF |
| 71 | HCDR3 amino acids | TDQAAWHAF |
| 72 | HCDR3 amino acids | TDQAAWFGF |

(continued)

| Seq ID No. | | Sequences |
|---|---|---|
| 73 | HCDR3 amino acids | TDQEAWAAF |
| 74 | HCDR3 amino acids | TDQEAWMAF |
| 75 | HCDR3 amino acids | TDQEAWHAF |
| 76 | HCDR3 amino acids | TDQEAWFGF |
| 77 | HCDR3 amino acids | TDQAAWAGF |
| 78 | HCDR3 amino acids | TDQAAWMGF |
| 79 | HCDR3 amino acids | TDQAAWHGF |
| 80 | HCDR3 amino acids | TDQEAWAGF |
| 81 | HCDR3 amino acids | TDQEAWMGF |
| 82 | HCDR3 amino acids | TDQEAWHGF |
| 83 | LCDR1 amino acids | RSSQNIVRSNGDTYLE |
| 84 | LCDR1 amino acids | RSSQNIVRSQGDTYLE |

Although the present disclosure has been described above, the present disclosure is not limited by the disclosed examples and the accompanying drawings and may be variously modified by those skilled in the art within the scope not departing from the spirit of the present disclosure. In addition, the technical ideas described in the examples of the present disclosure may be implemented independently, or two or more may be implemented in combination with each other.

**Claims**

1.  An antibody or antigen-binding fragment thereof that binds to an epitope of a programmed cell death-1 (PD-1) protein comprising amino acids P130, L128, and 1126 of SEQ ID NO: 62.

2.  The antibody or antigen-binding fragment thereof according to claim 1, wherein the antibody or antigen-binding fragment thereof binds to an additional epitope comprising at least one selected from the group consisting of N66, Y68, K78, A129, and A132 of SEQ ID NO: 62.

3.  The antibody or antigen-binding fragment thereof according to claim 1 or 2, wherein the antibody or antigen-binding fragment thereof has an equivalent level of binding affinity for human PD-1 and mouse PD-1.

4.  The antibody or antigen-binding fragment thereof according to claim 1 or 2, wherein the antibody or antigen-binding fragment thereof binds to human PD-1 with a KD of 9E-10 M or less at pH 6.0.

5.  An antibody or antigen-binding fragment thereof that binds to PD-1, wherein the antibody or antigen-binding fragment comprises

    heavy chain complementarity determining region 1 (HCDR1) comprising the amino acid sequence of SEQ ID NO: 1,
    HCDR2 comprising the amino acid sequence of SEQ ID NO: 3, 63, 64, 65, 66, or 67,
    HCDR3 comprising an amino acid sequence selected from the group consisting of SEQ ID NOs: 5 and 68 to 82,
    light chain complementarity determining region 1 (LCDR1) comprising the amino acid sequence of SEQ ID NO: 7, 60, 83, or 84,
    LCDR2 comprising the amino acid sequence of SEQ ID NO: 9, and
    LCDR3 comprising the amino acid sequence of SEQ ID NO: 11.

6.  The antibody or antigen-binding fragment thereof according to claim 5, wherein the antibody is a murine antibody, a chimeric antibody, or a humanized antibody.

**7.** An antibody or antigen-binding fragment thereof that binds to PD-1, wherein the antibody or antigen-binding fragment comprises

a heavy chain variable region comprising the amino acid sequence of SEQ ID NO: 13, 54, 56, or 58 and
a light chain variable region comprising the amino acid sequence of SEQ ID NO: 15, 55, 57, or 59.

**8.** An antibody or antigen-binding fragment thereof that binds to PD-1, wherein the antibody or antigen-binding fragment comprises

a heavy chain variable region comprising an amino acid sequence that has at least 75%, 80%, 85%, 90%, 95%, 96%, 97%, 98%, or 99% sequence identity to the amino acid sequence of SEQ ID NO: 13, 54, 56, or 58 and
a light chain variable region comprising an amino acid sequence that has at least 75%, 80%, 85%, 90%, 95%, 96%, 97%, 98%, or 99% sequence identity to the amino acid sequence of SEQ ID NO: 15, 55, 57, or 59.

**9.** A nucleic acid molecule encoding the antibody or antigen-binding fragment thereof of any one of claims 1, 2, and 5 to 8.

**10.** A cloning or expression vector comprising the nucleic acid molecule of claim 9.

**11.** A host cell comprising the cloning or expression vector of claim 10.

**12.** A method for producing the antibody or antigen-binding fragment thereof of any one of claims 1, 2, and 5 to 8 comprising culturing a host cell comprising a cloning or expression vector comprising a nucleic acid molecule encoding the antibody or antigen-binding fragment thereof of any one of claims 1, 2, and 5 to 8.

**13.** The antibody or antigen-binding fragment thereof according to any one of claims 1, 2, and 5 to 8, wherein the antibody or antigen-binding fragment is selected from a camelized single domain antibody, diabody, F(ab')$_2$, Fab', Fab, Fv, scFv, scFv dimer, BsFv, dsFv, (dsFv)$_2$, dsFv-dsFv', Fv fragment, ds diabody, nanobody, minibody, domain antibody, bivalent domain antibody, dAb, and single chain binding polypeptide.

**14.** A transgenic animal engineered to express the antibody or antigen-binding fragment thereof of any one of claims 1, 2, and 5 to 8.

**15.** The transgenic animal according to claim 14, wherein the animal is a rodent.

**16.** A multispecific antigen binding molecule, immunoconjugate, chimeric antigen receptor, engineered T cell receptor, or oncolytic virus comprising the antibody or antigen-binding fragment thereof of any one of claims 1, 2, and 5 to 8.

**17.** A pharmaceutical composition for preventing or treating a condition associated with PD-1 comprising the antibody or antigen-binding fragment thereof of any one of claims 1, 2, and 5 to 8, or a multispecific antigen-binding molecule, immunoconjugate, chimeric antigen receptor, engineered T cell receptor, or oncolytic virus comprising the antibody or antigen-binding fragment thereof.

**18.** The pharmaceutical composition according to claim 17, wherein the condition associated with PD-1 is a tumor, cancer, autoimmune disease, neurological disease, neurodegenerative disease, or infectious disease.

**19.** The pharmaceutical composition according to claim 18, wherein the tumor or cancer associated with PD-1 is selected from non-small cell lung cancer, small cell lung cancer, renal cell cancer, kidney cancer, liver cancer, bone cancer, skin cancer, colon cancer, rectal cancer, ovarian cancer, breast cancer, pancreatic cancer, gastric carcinoma, bladder cancer, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycoses fungoids, Merkel cell cancer, and classical Hodgkin's lymphoma (CHL), primary mediastinal large B-cell lymphoma, T-cell/histiocyte-rich B-cell lymphoma, Epstein-Barr virus (EBV)-positive and -negative post-transplant lymphoproliferative disease (PTLD), and EBV-associated diffuse large B-cell lymphoma (DLBCL), plasmablastic lymphoma, external NK/T-cell lymphoma, nasopharyngeal carcinoma, and human herpes virus 8 (HHV8)-associated primary effusion lymphoma, other hematologic malignancies including Hodgkin's lymphoma, neoplasms in the central nervous system including primary central nervous system (CNS) lymphoma, spinal axis tumor, and brainstem glioma.

**20.** The pharmaceutical composition according to claim 18, wherein the autoimmune disease associated with PD-1 is selected from lupus, systemic lupus erythematosus, Sjogren's Syndrome, arthritis, rheumatoid arthritis, asthma, COPD, pelvic inflammatory disease, Alzheimer's disease, inflammatory bowel disease, Crohn's disease, ulcerative colitis, Peyronie's Disease, coeliac disease, gallbladder disease, Pilonidal disease, peritonitis, psoriasis, psoriatic arthritis, vasculitis, surgical adhesions, stroke, type 1 diabetes, Lyme disease, meningoencephalitis, autoimmune uveitis, multiple sclerosis, Guillain-Barr syndrome, atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune diseases, pancreatitis, trauma (surgery), graft-versus-host disease, transplant rejection, heart disease including ischemic diseases such as myocardial infarction and atherosclerosis, intravascular coagulation, bone resorption, osteoporosis, osteoarthritis, periodontitis and hypochlorhydria, infertility related to lack of fetal-maternal tolerance, vitiligo, myasthenia gravis, and systemic sclerosis.

21. The pharmaceutical composition according to claim 18, wherein the neurological disease or neurodegenerative disease associated with PD-1 is selected from cognitive impairment, brain tumor, Alzheimer's disease, dementia, stroke, spinal cord injury, amyotrophic lateral sclerosis, Parkinson's disease, Huntington's disease, multiple sclerosis, glioblastoma, melanoma, pain, and memory loss.

22. The pharmaceutical composition according to claim 18, wherein the infectious disease associated with PD-1 is selected from chronic viral infections including viral infection of hepatitis B, hepatitis C, herpes virus, Epstein-Barr virus, HIV, cytomegalovirus, herpes simplex virus type 1, herpes simplex virus type 2, human papilloma virus, adenovirus, Kaposi West sarcoma associated with herpes virus epidemics, thin ring virus (Torquetenovirus), JC virus, and BK virus.

23. A method for preventing or treating tumors, cancer, autoimmune diseases, neurological diseases, neurodegenerative diseases, or infectious diseases, comprising administering to an individual at least one selected from the group consisting of the antibody or antigen-binding fragment thereof of any one of claims 1, 2, and 5 to 8, and a multispecific antigen-binding molecule, immunoconjugate, chimeric antigen receptor, engineered T cell receptor, and oncolytic virus comprising the antibody or antigen-binding fragment thereof.

24. A pharmaceutical composition for increasing an immune response in a subject having cancer comprising the antibody or antigen-binding fragment thereof of any one of claims 1, 2, and 5 to 8, or a multispecific antigen-binding molecule, immunoconjugate, chimeric antigen receptor, engineered T cell receptor, or oncolytic virus comprising the antibody or antigen-binding fragment thereof.

25. A method for producing the antibody or antigen-binding fragment of any one of claims 1, 2, and 5 to 8 comprising immunizing PD-1 knockout mice with a PD-1 antigen, isolating B lymphocytes from the spleen removed from the mice, and selecting a hybridoma that produces an antibody that reacts with a human PD-1 antigen from the hybridoma cells obtained by the fusion of myeloma cells with the B lymphocytes.

26. A hybridoma produced by the method according to claim 25.

Figure 1

Figure 2

## Binding (Flow Cytometry)

Figure 3

Ligand Blocking (ELISA)

## Human PD-1/PD-L1 binding blocking

## Mouse PD-1/PD-L1 binding blocking

Figure 4

Figure 5

Figure 6

## Human PD-1

## Mouse  PD-1

Figure 7

Binding Specificity (ELISA)

Figure 8

**Amino acid sequence**

**Heavy chain**

Humanized antibody 1G1-h61

MGWSCIILFLVATATGVHS<u>QVQLVQSGAEVKKPGASVKVSCKASGYTFTGYWM
HWVRQAPGQGLEWMGMIHPNSDTTTYNEKFKNRVTMTRDTSISTAYMELSRL
RSDDTAVYYCAGTDQAAWFAFWGQGTTVTVSS</u>ASTKGPSVFPLAPCSRSTSESTA
ALGCLVKDYFPEPVTVSWNSGALTSGVHTFPAVLQSSGLYSLSSVVTVPSSSLGTKT
YTCNVDHKPSNTKVDKRVESKYGPPCPSCPAPEFLGGPSVFLFPPKPKDTLMISRTPE
VTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVFSCS
VMHEALHNHYTQKSLSLSLGK**

Humanized antibody 1G1-h68

MGWSCIILFLVATATGVHS<u>QVQLVQSGAEVKKPGASVKVSCKASGYTFTGYWM
HWVRQAPGQGLEWIGMIHPNSDTTTYNEKFKNRVTMTRDTSISTAYMELSRLR
SDDTAVYYCTGTDQAAWFAFWGQGTTVTVSS</u>ASTKGPSVFPLAPCSRSTSESTAA
LGCLVKDYFPEPVTVSWNSGALTSGVHTFPAVLQSSGLYSLSSVVTVPSSSLGTKTYT
CNVDHKPSNTKVDKRVESKYGPPCPSCPAPEFLGGPSVFLFPPKPKDTLMISRTPEVT
CVVVDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGNVFSCSVM
HEALHNHYTQKSLSLSLGK**

Humanized antibody 1G1-h70

MGWSCIILFLVATATGVHS<u>QVQLVQSGAEVKKPGASVKVSCKASGYTFTGYWM
HWVRQAPGQGLEWIGMIHPNSDTTTYNEKFKNRVTMTRDTSISTAYMELSRLR
SDDTAVYYCAGTDQAAWFAFWGQGTTVTVSS</u>ASTKGPSVFPLAPCSRSTSESTAA
LGCLVKDYFPEPVTVSWNSGALTSGVHTFPAVLQSSGLYSLSSVVTVPSSSLGTKTYT
CNVDHKPSNTKVDKRVESKYGPPCPSCPAPEFLGGPSVFLFPPKPKDTLMISRTPEVT
CVVVDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTCLVKG
FYPSDIAVEWESNGQPENNYKTTPPVLDSDGSFFLYSRLTVDKSRWQEGNVFSCSVM
HEALHNHYTQKSLSLSLGK**

Figure 9

**Amino acid sequence**
**Light chain**

Humanized antibody 1G1-h61
MGWSCIILFLVATATGVHS**DIVMTQTPLSLSVTPGQPASISCRSSQNIVHSNGDTYL**
**EWYLQKPGQSPKLLIYKVSKRFSGVPDRFSGSGSGTDFTLKISRVEAEDVGVYY**
**CFQGSHVPWTFGGGTKVEIK**RTVAAPSVFIFPPSDEQLKSGTASVVCLLNNFYPRE
AKVQWKVDNALQSGNSQESVTEQDSKDSTYSLSSTLTLSKADYEKHKVYACEVTH
QGLSSPVTKSFNRGEC**

Humanized antibody 1G1-h68
MGWSCIILFLVATATGVHS**DIVMTQTPLSLSVTPGQPASISCRSSQNIVHSNGDTYL**
**EWYLQKPGQSPQLLIYKVSKRFSGVPDRFSGSGSGTDFTLKISRVEAEDVGVYY**
**CFQGSHVPWTFGGGTKVEIK**RTVAAPSVFIFPPSDEQLKSGTASVVCLLNNFYPRE
AKVQWKVDNALQSGNSQESVTEQDSKDSTYSLSSTLTLSKADYEKHKVYACEVTH
QGLSSPVTKSFNRGEC**

Humanized antibody 1G1-h70
MGWSCIILFLVATATGVHS**DIVMTQTPLSLSVTPGQPASISCRSSQNIVHSQGDTYL**
**EWYLQKPGQSPQLLIYKVSKRFSGVPDRFSGSGSGTDFTLKISRVEAEDVGVYY**
**CFQGSHVPWTFGGGTKVEIK**RTVAAPSVFIFPPSDEQLKSGTASVVCLLNNFYPRE
AKVQWKVDNALQSGNSQESVTEQDSKDSTYSLSSTLTLSKADYEKHKVYACEVTH
QGLSSPVTKSFNRGEC**

Figure 10a


**DNA sequence**
**Heavy chain**


Humanized antibody 1G1-h61

ATGGGCTGGTCTTGTATTATTCTGTTTCTGGTCGCAACTGCTACAGGCGTGCATTC

T<u>CAAGTTCAGCTGGTGCAGAGCGGCGCCGAGGTGAAGAAGCCTGGAGCCAG</u>

<u>CGTGAAAGTGTCCTGCAAGGCCTCTGGCTACACCTTTACAGGCTACTGGAT</u>

<u>GCACTGGGTGCGGCAGGCCCCTGGACAGGGCCTGGAATGGATGGGCATGA</u>

<u>TCCACCCCAACAGCGACACCACAACCTACAACGAGAAGTTCAAGAATAGAG</u>

<u>TGACCATGACAAGAGATACCAGCATCAGCACCGCCTACATGGAACTGAGCA</u>

<u>GACTGCGGTCCGATGACACAGCTGTGTACTATTGTGCCGGCACCGACCAGG</u>

<u>CCGCTTGGTTCGCCTTCTGGGGGCAGGGCACCACAGTCACCGTGAGCTCTG</u>

CCAGCACCAAGGGCCCTTCCGTGTTTCCCCTGGCCCCTTGCTCCCGGTCCACATCT

GAGAGCACCGCCGCCCTGGGCTGTCTGGTGAAGGACTACTTCCCAGAGCCCGTG

ACCGTGAGCTGGAACAGCGGCGCCCTGACAAGCGGCGTGCACACATTTCCCGCC

GTGCTGCAGAGCTCCGGCCTGTACTCCCTGTCTAGCGTGGTGACAGTGCCTTCCT

CTAGCCTGGGCACCAAGACATATACCTGTAACGTGGACCACAAGCCAAGCAATA

CCAAGGTGGATAAGCGGGTGGAGTCTAAGTACGGCCCTCCTTGCCCTAGCTGTCC

TGCTCCAGAGTTTCTGGGCGGCCCTTCCGTGTTCCTGTTTCCACCCAAACCAAAG

GACACACTGATGATCTCTAGAACACCAGAGGTGACCTGCGTGGTGGTGGACGTG

AGCCAGGAGGATCCCGAGGTGCAGTTCAACTGGTACGTGGATGGCGTGGAGGTG

CACAATGCCAAGACCAAGCCAAGAGAGGAGCAGTTTAACTCTACATACAGGGTG

GTGAGCGTGCTGACCGTGCTGCACCAGGATTGGCTCAACGGCAAGGAGTATAAG

TGCAAGGTGTCCAATAAGGGCCTGCCCTCCTCTATCGAGAAGACAATCTCTAAGG

CTAAGGGCCAGCCAAGAGAGCCTCAGGTGTACACCCTGCCTCCAAGCCAGGAGG

AGATGACAAAGAACCAGGTGTCCCTGACATGTCTGGTGAAGGGCTTCTATCCCTC

CGACATCGCCGTGGAGTGGGAGTCTAATGGCCAGCCTGAGAACAATTACAAGAC

CACACCCCCTGTGCTGGACTCTGATGGCAGCTTCTTTCTGTATTCCAGGCTGACC

GTGGATAAGTCTCGGTGGCAGGAGGGCAACGTGTTCAGCTGCTCTGTGATGCAC

GAAGCCCTGCATAATCACTATACTCAGAAAAGTCTGTCACTGTCACTGGGAAAG*T*

*GATAA*

Figure 10b

**DNA sequence**
**Heavy chain**

Humanized antibody 1G1-h68
ATGGGCTGGTCTTGTATTATTCTGTTTCTGGTCGCAACTGCTACAGGCGTGCATTC
T<u>CAAGTTCAGCTGGTGCAGAGCGGCGCCGAGGTGAAGAAGCCTGGAGCCAG</u>
<u>CGTGAAAGTGTCCTGCAAGGCCTCTGGCTACACCTTTACAGGCTACTGGAT</u>
<u>GCACTGGGTGCGGCAGGCCCCTGGACAGGGCCTGGAATGGATCGGCATGAT</u>
<u>CCACCCCAACAGCGACACCACAACCTACAACGAGAAGTTCAAGAATAGAGT</u>
<u>GACCATGACAAGAGATACCAGCATCAGCACCGCCTACATGGAACTGAGCAG</u>
<u>ACTGCGGTCCGATGACACAGCTGTGTACTATTGTACCGGCACCGACCAGGC</u>
<u>CGCTTGGTTCGCCTTCTGGGGGCAGGGCACCACAGTCACCGTGAGCTCT</u>GC
CAGCACCAAGGGCCCTTCCGTGTTTCCCCTGGCCCCTTGCTCCCGGTCCACATCT
GAGAGCACCGCCGCCCTGGGCTGTCTGGTGAAGGACTACTTCCCAGAGCCCGTG
ACCGTGAGCTGGAACAGCGGCGCCCTGACAAGCGGCGTGCACACATTTCCCGCC
GTGCTGCAGAGCTCCGGCCTGTACTCCCTGTCTAGCGTGGTGACAGTGCCTTCCT
CTAGCCTGGGCACCAAGACATATACCTGTAACGTGGACCACAAGCCAAGCAATA
CCAAGGTGGATAAGCGGGTGGAGTCTAAGTACGGCCCTCCTTGCCCTAGCTGTCC
TGCTCCAGAGTTTCTGGGCGGCCCTTCCGTGTTCCTGTTTCCACCCAAACCAAAG
GACACACTGATGATCTCTAGAACACCAGAGGTGACCTGCGTGGTGGTGGACGTG
AGCCAGGAGGATCCCGAGGTGCAGTTCAACTGGTACGTGGATGGCGTGGAGGTG
CACAATGCCAAGACCAAGCCAAGAGAGGAGCAGTTTAACTCTACATACAGGGTG
GTGAGCGTGCTGACCGTGCTGCACCAGGATTGGCTCAACGGCAAGGAGTATAAG
TGCAAGGTGTCCAATAAGGGCCTGCCCTCCTCTATCGAGAAGACAATCTCTAAGG
CTAAGGGCCAGCCAAGAGAGCCTCAGGTGTACACCCTGCCTCCAAGCCAGGAGG
AGATGACAAAGAACCAGGTGTCCCTGACATGTCTGGTGAAGGGCTTCTATCCCTC
CGACATCGCCGTGGAGTGGGAGTCTAATGGCCAGCCTGAGAACAATTACAAGAC
CACACCCCCTGTGCTGGACTCTGATGGCAGCTTCTTTCTGTATTCCAGGCTGACC
GTGGATAAGTCTCGGTGGCAGGAGGGCAACGTGTTCAGCTGCTCTGTGATGCAC
GAAGCCCTGCATAATCACTATACTCAGAAAAGTCTGTCACTGTCACTGGGAAAG*T*
*GATAA*

Figure 10c

**DNA sequence**
**Heavy chain**

Humanized antibody 1G1-h70
ATGGGCTGGTCTTGTATTATTCTGTTTCTGGTCGCAACTGCTACAGGCGTGCATTC
T<u>CAAGTTCAGCTGGTGCAGAGCGGCGCCGAGGTGAAGAAGCCTGGAGCCAG</u>
<u>CGTGAAAGTGTCCTGCAAGGCCTCTGGCTACACCTTTACAGGCTACTGGAT</u>
<u>GCACTGGGTGCGGCAGGCCCCTGGACAGGGCCTGGAATGGATCGGCATGAT</u>
<u>CCACCCCAACAGCGACACCACAACCTACAACGAGAAGTTCAAGAATAGAGT</u>
<u>GACCATGACAAGAGATACCAGCATCAGCACCGCCTACATGGAACTGAGCAG</u>
<u>ACTGCGGTCCGATGACACAGCTGTGTACTATTGTGCCGGCACCGACCAGGC</u>
<u>CGCTTGGTTCGCCTTCTGGGGGCAGGGCACCACAGTCACCGTGAGCTCT</u>GC
CAGCACCAAGGGCCCTTCCGTGTTTCCCCTGGCCCCTTGCTCCCGGTCCACATCT
GAGAGCACCGCCGCCCTGGGCTGTCTGGTGAAGGACTACTTCCCAGAGCCCGTG
ACCGTGAGCTGGAACAGCGGCGCCCTGACAAGCGGCGTGCACACATTTCCCGCC
GTGCTGCAGAGCTCCGGCCTGTACTCCCTGTCTAGCGTGGTGACAGTGCCTTCCT
CTAGCCTGGGCACCAAGACATATACCTGTAACGTGGACCACAAGCCAAGCAATA
CCAAGGTGGATAAGCGGGTGGAGTCTAAGTACGGCCCTCCTTGCCCTAGCTGTCC
TGCTCCAGAGTTTCTGGGCGGCCCTTCCGTGTTCCTGTTTCCACCCAAACCAAAG
GACACACTGATGATCTCTAGAACACCAGAGGTGACCTGCGTGGTGGTGGACGTG
AGCCAGGAGGATCCCGAGGTGCAGTTCAACTGGTACGTGGATGGCGTGGAGGTG
CACAATGCCAAGACCAAGCCAAGAGAGGAGCAGTTTAACTCTACATACAGGGTG
GTGAGCGTGCTGACCGTGCTGCACCAGGATTGGCTCAACGGCAAGGAGTATAAG
TGCAAGGTGTCCAATAAGGGCCTGCCCTCCTCTATCGAGAAGACAATCTCTAAGG
CTAAGGGCCAGCCAAGAGAGCCTCAGGTGTACACCCTGCCTCCAAGCCAGGAGG
AGATGACAAAGAACCAGGTGTCCCTGACATGTCTGGTGAAGGGCTTCTATCCCTC
CGACATCGCCGTGGAGTGGGAGTCTAATGGCCAGCCTGAGAACAATTACAAGAC
CACACCCCCTGTGCTGGACTCTGATGGCAGCTTCTTTCTGTATTCCAGGCTGACC
GTGGATAAGTCTCGGTGGCAGGAGGGCAACGTGTTCAGCTGCTCTGTGATGCAC
GAAGCCCTGCATAATCACTATACTCAGAAAAGTCTGTCACTGTCACTGGGAAAG*T*
*GATAA*

Figure 11

**DNA sequence**
**Light chain**

Humanized antibody 1G1-h61
ATGGGCTGGTCATGTATTATTCTGTTTCTGGTCGCAACTGCTACAGGGGTCCATAGT**GATATCGTT**
**ATGACCCAGACCCCTCTGAGCCTGTCCGTGACCCCAGGCCAACCTGCCTCTATCAGCTGTAG**
**AAGCAGCCAGAACATCGTGCACAGCAACGGCGACACCTACCTGGAATGGTATCTGCAGAAA**
**CCTGGACAGAGCCCCAAGCTGCTGATCTACAAGGTGTCCAAGCGGTTTTCCGGCGTGCCTGA**
**TAGATTCAGCGGATCTGGCAGCGGCACAGACTTCACCCTGAAGATTTCTAGAGTGGAGGCCG**
**AGGACGTGGGCGTCTACTACTGCTTCCAGGGCAGCCACGTGCCCTGGACATTCGGCGGCGG**
**AACAAAGGTGGAAATCAAG**AGGACAGTGGCCGCCCCAAGCGTGTTCATCTTTCCCCCTTCCGACG
AGCAGCTGAAGTCTGGCACCGCCAGCGTGGTGTGCCTGCTGAACAACTTCTACCCTCGGGAGGCC
AAGGTCCAGTGGAAGGTGGATAACGCCCTGCAGTCTGGCAATAGCCAGGAGTCCGTGACCGAGCA
GGACTCTAAGGATAGCACATATTCCCTGTCTAGCACCCTGACACTGAGCAAGGCCGATTACGAGA
AGCACAAGGTGTATGCCTGTGAAGTCACCCATCAGGGGCTGTCATCACCCGTCACTAAGTCATTCA
ATCGCGGAGAATGC*TGATAA*

Humanized antibody 1G1-h68
ATGGGCTGGTCATGTATTATTCTGTTTCTGGTCGCAACTGCTACAGGGGTCCATAGT**GATATCGTT**
**ATGACCCAGACCCCTCTGAGCCTGTCCGTGACCCCAGGCCAACCTGCCTCTATCAGCTGTAG**
**AAGCAGCCAGAACATCGTGCACAGCAACGGCGACACCTACCTGGAATGGTATCTGCAGAAA**
**CCTGGACAGAGCCCCCAGCTGCTGATCTACAAGGTGTCCAAGCGGTTTTCCGGCGTGCCTGA**
**TAGATTCAGCGGATCTGGCAGCGGCACAGACTTCACCCTGAAGATTTCTAGAGTGGAGGCCG**
**AGGACGTGGGCGTCTACTACTGCTTCCAGGGCAGCCACGTGCCCTGGACATTCGGCGGCGG**
**AACAAAGGTGGAAATCAAG**AGGACAGTGGCCGCCCCAAGCGTGTTCATCTTTCCCCCTTCCGACG
AGCAGCTGAAGTCTGGCACCGCCAGCGTGGTGTGCCTGCTGAACAACTTCTACCCTCGGGAGGCC
AAGGTCCAGTGGAAGGTGGATAACGCCCTGCAGTCTGGCAATAGCCAGGAGTCCGTGACCGAGCA
GGACTCTAAGGATAGCACATATTCCCTGTCTAGCACCCTGACACTGAGCAAGGCCGATTACGAGA
AGCACAAGGTGTATGCCTGTGAAGTCACCCATCAGGGGCTGTCATCACCCGTCACTAAGTCATTCA
ATCGCGGAGAATGC*TGATAA*

Humanized antibody 1G1-h70
ATGGGCTGGTCATGTATTATTCTGTTTCTGGTCGCAACTGCTACAGGGGTCCATAGT**GATATCGTTAT**
**GACCCAGACCCCTCTGAGCCTGTCCGTGACCCCAGGCCAACCTGCCTCTATCAGCTGTAGAA**
**GCAGCCAGAACATCGTGCACAGCCAGGGCGACACCTACCTGGAATGGTATCTGCAGAAACCT**
**GGACAGAGCCCCCAGCTGCTGATCTACAAGGTGTCCAAGCGGTTTTCCGGCGTGCCTGATAG**
**ATTCAGCGGATCTGGCAGCGGCACAGACTTCACCCTGAAGATTTCTAGAGTGGAGGCCGAGG**
**ACGTGGGCGTCTACTACTGCTTCCAGGGCAGCCACGTGCCCTGGACATTCGGCGGCGGAACA**
**AAGGTGGAAATCAAG**AGGACAGTGGCCGCCCCAAGCGTGTTCATCTTTCCCCCTTCCGACGAGCA
GCTGAAGTCTGGCACCGCCAGCGTGGTGTGCCTGCTGAACAACTTCTACCCTCGGGAGGCCAAGGT
CCAGTGGAAGGTGGATAACGCCCTGCAGTCTGGCAATAGCCAGGAGTCCGTGACCGAGCAGGACT
CTAAGGATAGCACATATTCCCTGTCTAGCACCCTGACACTGAGCAAGGCCGATTACGAGAAGCACA
AGGTGTATGCCTGTGAAGTCACCCATCAGGGGCTGTCATCACCCGTCACTAAGTCATTCAATCGCGG
AGAATGC*TGATAA*

Figure 12a

Figure 12b

Figure 13

Cross-reactivity

Figure 14

**Body weight**
**Tumor volume measurement**

**Ab treatment (Q3D × 5)**

| | | | | |
|---|---|---|---|---|

0          7    10   13   16   19

**Tumor growth & Survival**

**Tumor cell injection (s.c.)**

**Grouping Treatment**

Figure 15

Figure 16

B16F11

Legend:
- mIgG1-k 10 mg/kg
- 1G1 10 mg/kg
- 1G1 5 mg/kg
- 1G1 2.5 mg/kg
- 1G1 1 mg/kg

Figure 17

MC38

| | 1 | 3 | 7 | 8 | 11 | 13 | 15 | 17 |
|---|---|---|---|---|---|---|---|---|
| hIgG4 vs. 1G-h70 | 0.7322 | 0.3268 | 0.0662 | 0.0655 | 0.0179 | 0.0025 | 0.0008 | 0.0168 |
| rIgG2a vs. RMP1-14 | 0.882 | 0.7618 | >0.9999 | 0.9993 | 0.5105 | 0.4331 | 0.0542 | 0.1256 |

Figure 18

Figure 19a

Figure 19b

PH7.4 vs PH6: ka

PH7.4 vs PH6: KD

PH7.4 vs PH6: kd

| | Ratio(PH 7.4 vs PH6) | | |
|---|---|---|---|
| | ka | kd | KD |
| Keytruda | 2.3 | 0.7 | 1.6 |
| Opdivo | 2.1 | 1.1 | 2.3 |
| 2E5 | 3.4 | 0.8 | 2.8 |
| 1G1–h70 | 2.3 | 2.7 | 5.8 |

EP 4 289 866 A2

72

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015136541 A
- WO 2017220990 A
- US 5648237 A
- US 5789199 A
- US 5840523 A
- US 5959177 A
- US 6040498 A
- US 6420548 B
- US 7125978 B
- US 6417429 B
- US 13022759 B
- US 20100331527 A
- WO 05103081 A
- US 8257740 B
- US 8246995 B
- US 7087411 B
- WO 2018053709 A

### Non-patent literature cited in the description

- **SYN et al.** *Lancet Oncol,* 2017, vol. 18 (12), e731-e741
- **PATSOUKIS et al.** *Sci. Adv.,* 2020, vol. 6, eabd2712
- **GOLDEN-MASON et al.** *J Immunol,* 2008, vol. 180, 3637-3641
- **OKAZAKI et al.** *Int Immunol,* 2007, vol. 19, 813-824
- **GREBINOSKI ; VIGNALI.** *Curr Opin Immunol,* 2020, vol. 67, 1-9
- **ALTSCHUL et al.** *J. Mol. Biol.,* 1990, vol. 215, 403-410
- *Nucleic Acids Res.,* 1997, vol. 25, 3389-3402
- **PEARSON.** *Methods Mol. Biol.,* 1994, vol. 24, 307-331
- **GONNET et al.** *Science,* 1992, vol. 256, 1443-45
- **CHARLTON, K.A.** Methods in Molecular Biology. Humana Press, 2003, vol. 248, 245-254
- **GERNGROSS, T.U.** *Nat. Biotech.,* 2004, vol. 22, 1409-1414
- **LI, H. et al.** *Nat. Biotech.,* 2006, vol. 24, 210-215
- **GRAHAM, F.L. et al.** *J. Gen Virol.,* 1977, vol. 36, 59-74
- **MATHER, J.P.** *Biol. Reprod.,* 1980, vol. 23, 243-252
- **MATHER, J.P et al.** *Annals N.Y. Acad. Sci.,* 1982, vol. 383, 44-68
- **URLAUB, G. et al.** *Proc. Natl. Acad. Sci. USA,* 1980, vol. 77, 4216-4220
- **YAZAKI, P. ; WU, A. M.** Methods in Molecular Biology. Humana Press, 2004, vol. 248, 255-268
- **KLEIN et al.** *mAbs,* 2012, vol. 4 (6), 1-11
- **KAZANE et al.** *J. Am. Chem. Soc.,* 04 December 2012
- **ZUNLI ZAO et al.** Emerging role of PD-1 in the central nervous system and brain diseases. *Neurosci. Bull.,* 20 April 2021
- **LING CHEN et al.** Role of the Immune Modulator Programmed Cell Death-1 during Development and Apoptosis of Mouse Retinal Ganglion Cells. *Investigative Ophthalmology & Visual Science,* 2009, vol. 50 (10), 4941-4948
- **MICHAL SCHWARTZ et al.** Potential immunotherapy for Alzheimer disease and age-related dementia. *Dialogues in clinical neuroscience,* 2019, vol. 21 (1), 21
- **RU-RONG JI et al.** Anti-PD-1 treatment as a neurotherapy to enhance neuronal excitability, synaptic plasticity and memory. *BioRxiv,* 10 December 2019
- **POWELL et al.** Compendium of excipients for parenteral formulations. *J Pharm Sci Technol,* 1998, vol. 52, 238-311
- **WU et al.** *J. Biol. Chem.,* 1987, vol. 262, 4429-4432
- **LANGER.** *Science,* 1990, vol. 249, 1527-1533
- **ARRUEBO, M. et al.** Antibody-conjugated nanoparticles for biomedical applications. *J. Nanomat.,* 2009, vol. 2009, 24